# EUROPEAN PATENT SPECIFICATION

(11) **EP 3 837 690 B1**
(45) Date of publication and mention of the grant of the patent: **26.06.2024**
(21) Application number: 19753337.5
(22) Date of filing: 12.08.2019
(51) Int. Cl.: G16B 20/20, G16B 40/20, G16B 30/10

(54) **SYSTEMS AND METHODS FOR USING NEURAL NETWORKS FOR GERMLINE AND SOMATIC VARIANT CALLING**
SYSTEME UND VERFAHREN ZUR VERWENDUNG NEURONALER NETZE FÜR KEIMBAHN- UND SOMATISCHES VARIANT-CALLING
SYSTÈMES ET PROCÉDÉS D'UTILISATION DE RÉSEAUX NEURONAUX POUR L'APPEL DE VARIANTS DE LIGNÉE GERMINALE ET SOMATIQUES

(30) Priority: 13.08.2018 US 201862718338 P; 31.05.2019 US 201962855541 P
(43) Date of publication of application: 23.06.2021
(73) Proprietor: F. Hoffmann-La Roche AG, 4070 Basel (CH); Roche Diagnostics GmbH, 68305 Mannheim (DE)
(72) Inventor: LAM, Hugo Y. K., Pleasanton, California 94588 (US); MOHIYUDDIN, Marghoob, Pleasanton, California 94588 (US); SAHRAEIAN, Mohammad, Pleasanton, California 94588 (US)
(74) Representative: Merkel, Patrick
(86) International application number: PCT/EP2019/071580
(87) International publication number: WO 2020/035446

(56) References cited:
- Ruibang Luo ET AL: "Clairvoyante: a multi-task convolutional deep neural network for variant calling in Single Molecule Sequencing", bioRxiv, 26 April 2018 (2018-04-26), XP055608907, DOI: 10.1101/310458 Retrieved from the Internet: URL:https://www.biorxiv.org/content/biorxi v/early/2018/04/28/310458.full-text.pdf [retrieved on 2019-07-25] cited in the application
- Ryan Poplin ET AL: "Creating a universal SNP and small indel variant caller with deep neural networks", bioRxiv, 20 March 2018 (2018-03-20), XP055585250, DOI: 10.1101/092890 Retrieved from the Internet: URL:https://www.biorxiv.org/content/biorxi v/early/2018/03/20/092890.full.pdf [retrieved on 2019-10-29] cited in the application
- RYAN POPLIN ET AL: "A universal SNP and small-indel variant caller using deep neural networks", NATURE BIOTECHNOLOGY, 24 September 2018 (2018-09-24), XP055572781, New York ISSN: 1087-0156, DOI: 10.1038/nbt.4235
- Jason Chin: "0/29/2019 Simple Convolutional Neural Network for Genomic Variant Calling with TensorFlowsimple-convolution-neural-networ k-for-genomic-variant-calling-with-tensorf low Simple Convolutional Neural Network for Genomic Variant Calling with TensorFlow", , 17 July 2017 (2017-07-17), XP055637183, Retrieved from the Internet: URL:https://towardsdatascience.com/simple- convolution-neural-network-for-genomic-var iant-calling-with-tensorflow-c085dbc2026f [retrieved on 2019-10-29] cited in the application
- Remi Torracinta ET AL: "Adaptive Somatic Mutations Calls with Deep Learning and Semi-Simulated Data", bioRxiv, 4 October 2016 (2016-10-04), XP055540696, DOI: 10.1101/079087 Retrieved from the Internet: URL:https://www.biorxiv.org/content/biorxi v/early/2016/10/04/079087.full-text.pdf [retrieved on 2019-10-29] cited in the application
- LI TAI FANG ET AL: "An ensemble approach to accurately detect somatic mutations using SomaticSeq", GENOME BIOLOGY, vol. 16, no. 1, 17 September 2015 (2015-09-17), XP055531382, DOI: 10.1186/s13059-015-0758-2 cited in the application
- J. DING ET AL: "Feature-based classifiers for somatic mutation detection in tumour-normal paired sequencing data", BIOINFORMATICS., vol. 28, no. 2, 13 November 2011 (2011-11-13), pages 167-175, XP055257193, GB ISSN: 1367-4803, DOI: 10.1093/bioinformatics/btr629
- CHANG XU: "A review of somatic single nucleotide variant calling algorithms for next-generation sequencing data", COMPUTATIONAL AND STRUCTURAL BIOTECHNOLOGY JOURNAL, vol. 16, 6 February 2018 (2018-02-06), pages 15-24, XP055636960, Sweden ISSN: 2001-0370, DOI: 10.1016/j.csbj.2018.01.003
- TRAVERS CHING ET AL: "Opportunities and obstacles for deep learning in biology and medicine", JOURNAL OF THE ROYAL SOCIETY. INTERFACE, THE ROYAL SOCIETY, LONDON, GB, vol. 15, no. 20170387, 4 April 2018 (2018-04-04), pages 1-47, XP002789257, ISSN: 1742-5689, DOI: 10.1098/RSIF.2017.0387

## Description

### FIELD

Embodiments of the invention relate generally to systems and methods for variant calling, and more particularly to using machine learning algorithms for variant calling.

### BACKGROUND

Identifying genetic mutations using sequencing data is a crucial task in bioinformatics that has implications in diagnosis, prognosis, and treatment of multiple diseases including cancer. The task is a non-trivial one, especially when the sequencing technology used to generate the sequencing data has a high-error rate (which is common to single-molecule sequencing technologies) or when the mutations occur at low frequency (which is particularly true for cancer mutations) or in complex genomic regions. The low frequency of cancer mutations in a sequencing sample could be due to sample contamination (since tumor samples may contain some DNA from normal cells) or due to tumor heterogeneity.

Traditional approaches narrow down the scope of the problem by limiting to specific technologies and the type of mutations to detect. Therefore, different sets of tools and frameworks exist to identify germline and cancer mutations which are further stratified by whether the mutations are small or large. Additionally, the approaches used for low-error sequencing technologies (e.g., short-read sequencing) differ from the ones used for high-error sequencing technologies (e.g., single-molecule sequencing). The differences manifest primarily in data signatures as well as the statistical models used to differentiate errors from true mutations. The use of explicit data signatures and statistical models limits the generalizability of traditional approaches to multiple technologies. Furthermore, even the traditional approaches require parameter tuning to capture the specific characteristics of samples, which can depend on a multitude of factors including the technology used, sequencing protocol, sequencing sample, sample purity and so on.

Deep learning is a machine learning technique that uses multiple data layers to implicitly capture the data signatures as well as the statistical model, as long as the right training data is available. This makes deep learning a potentially appealing solution in terms of generalizability for the mutation detection problem. Several deep learning solutions for classifying germline mutations are known.

Poplin, R. et al. ("Creating a Universal SNP and Small Indel Variant Caller With Deep Neural Networks", bioRxiv, 092890 (2016)), proposes a method where candidate variant locations are first identified by the genome scan, and then a pileup image of reads aligned around each identified candidate are provided as inputs into a convolutional neural network (CNN), which then predicts the genotype of the candidate variants. Such approach is not very practical because it requires a complex neural network architecture and vast amounts of memory.

Luo, R. et al. ("Clairvoyante: a Multi-task Convolutional Deep Neural Network For Variant Calling In Single Molecule Sequencing. bioRxiv, 310458 (2018)) uses a data summarization approach, which includes scanning the genome to identify the candidate variant sites, and preparing for each candidate position multiple input matrices, one matrix for each type of variant. A similar technique is discussed in Chin, J. ("Simple Convolutional Neural Network for Genomic Variant Calling with TensorFlow," https://towardsdatascience.com/simple-convolution-neural-network-for-genomic-variant-calling-with-tensorflow-c085dbc2026f, published Jul 16, 2017). These approaches are also not optimal, because they cannot clearly represent all the inserted sequences across the window. For example, the Clairvoyante system devotes a separate input matrix for insertion events that only captures insertions at the variant location, while ignoring real/erroneous insertions at other locations within the window. Full-picture information about precise locations of inserted sequences across the window can be critical in distinguishing real insertion variants from background noise.

Torracinta, R. et al. ("Adaptive somatic mutations calls with deep learning and semi-simulated data. bioRxiv, 79087 (2016)), describes applying a six-layer fully-connected neural network to a set of manually extracted features. But a system having fully-connected layers only cannot leverage the power provided by convolutional neural networks, which is to learn feature representations directly from the raw sequence data using patterns seen in local genomic context of candidate variants. Additionally, since fully-connected networks are more complex, that approach allows for less generalizability and scalability than that enabled by the use of CNNs.

### SUMMARY OF THE INVENTION

The invention is defined by the appended claims.

### SUMMARY OF THE DISCLOSURE

The present invention relates generally systems and methods for variant calling, and more particularly to using machine learning algorithms for variant calling.

In some embodiments, a method for germline variant calling is provided. The method can include obtaining a reference sequence, a plurality of sequence reads, and a position of a candidate variant within the sequence reads; obtaining augmented sequence reads by inserting one or more spaces in one or more sequence reads; obtaining an augmented reference sequence by inserting one or more spaces in the reference sequence; converting a segment of the augmented sequence reads around the candidate variant into a sample matrix; converting a segment of the augmented reference sequence around the candidate variant into a reference matrix; providing the sample matrix and the reference matrix to a trained neural network; and obtaining, at the output of the trained neural network, prediction data related to a variant within the plurality of sequence reads.

In some embodiments, the method further includes detecting one or more inserted bases within the plurality of sequence reads, wherein augmenting the sequence reads and the reference sequence includes: for each inserted base detected in any of the sequence reads, inserting a space in the reference sample at the position of the inserted base.

In some embodiments, the method further includes: for each inserted base detected in any of the sequence reads, inserting a space at the position of the inserted base in every sequence read for which no insertions were detected at the position of the inserted base.

In some embodiments, the sample matrix includes at least four lines representing four types of nucleotide bases, each line representing the number of bases of the respective nucleotide base type at different positions within the segment of the augmented sequence reads; and at least one line representing the number of inserted spaces at different positions within the segment of the augmented sequence reads.

In some embodiments, the reference matrix has the same dimensions as the sample matrix and wherein the reference matrix provides a complete representation of the locations of different nucleotide bases and spaces within the augmented reference sequence.

In some embodiments, the trained neural network comprises a trained convolutional neural network.

In some embodiments, the method further includes providing to the trained neural network at least one of: a variant position matrix representing the candidate variant's position within the segment of the augmented sequence reads; a coverage matrix representing coverage or depth of the segment of the augmented sequence reads; an alignment feature matrix representing an alignment feature of the augmented sequence reads; a knowledgeable base matrix representing information about publicly known information about one or more variants.

In some embodiments, the prediction data related to the variant comprises at least one of: a predicted type of the variant; a predicted position of the variant; a predicted length of the variant; and a predicted genotype of the variant.

In some embodiments, the prediction data related to the variant includes a predicted type of the variant, and wherein the neural network is configured to produce one of a plurality of values for predicted type of the variant, the plurality of values including: a first value indicating a probability that the variant is a false positive; a second value indicating a probability that the variant is a single-nucleotide- polymorphism variant; a third value indicating a probability that the variant is a deletion variant; and a fourth value indicating a probability that the variant is an insertion variant.

In some embodiments, a method for somatic variant calling is provided. The method can include obtaining a plurality of normal sequence reads and a plurality of tumor sequence reads; converting a segment of the normal sequence reads and a segment of the tumor sequence reads into a normal sample matrix and a tumor sample matrix, respectively; feeding the normal sample matrix and the tumor sample matrix into a trained convolutional neural network; and obtaining, at the output of the trained convolutional neural network, a predicted type of a somatic variant within the plurality of tumor sequence reads.

In some embodiments, the plurality of tumor sequence reads represent genetic information of a patient's tumor sample, and the plurality of normal sequence reads represent genetic information of the patient's normal sample.

In some embodiments, converting the segment of the normal sequence reads into the normal sample matrix includes augmenting the segment of the normal sequence reads by inserting one or more spaces in one or more normal sequence reads; and converting the segment of the tumor sequence reads into the tumor sample matrix comprises augmenting the segment of the tumor sequence reads by inserting one or more spaces in one or more tumor sequence reads.

In some embodiments, the tumor sample matrix includes at least one line for each nucleotide base type, each line representing the number of occurrences of the respective nucleotide base type at each position within the segment of the tumor sequence reads; and at least one line representing the number of inserted spaces at each position within the segment of the tumor sequence reads.

In some embodiments, the method further includes providing to the trained convolutional neural network one or more matrices representing one or more features obtained from one or more other variant callers that have analyzed the plurality of tumor sequence reads and/or the plurality of normal sequence reads.

In some embodiments, the method further includes obtaining a reference sequence; converting the reference sequence into a reference matrix; and feeding the reference matrix into the trained convolutional matrix along with the normal sample matrix and the tumor sample matrix.

In some embodiments, a non-transitory computer-readable medium including instructions which, when executed by one or more processors of a computing system, causes the computing system to perform operations including: obtaining a plurality of normal sequence reads and a plurality of tumor sequence reads; converting a segment of the normal sequence reads and a segment of the tumor sequence reads into a normal sample matrix and a tumor sample matrix, respectively; feeding the normal sample matrix and the tumor sample matrix into a trained convolutional neural network; and obtaining, at the output of the trained convolutional neural network, a predicted type of a somatic variant within the plurality of normal sequence reads.

In some embodiments, a computing system including one or more processors and coupled to one or more non- transitory computer-readable memories storing instructions which, when executed by the computing system, cause the computing system to perform operations including: obtaining a plurality of tumor sequence reads; obtaining augmented tumor sequence reads by inserting one or more spaces in one or more tumor sequence reads; converting a segment of the tumor sequence reads into a tumor sample matrix; feeding the normal sample matrix and the tumor sample matrix into a trained neural network; and obtaining, at the output of the trained neural network, a predicted type of a somatic variant within the plurality of tumor sequence reads.

In some embodiments, a method for variant calling is provided. The method can include: obtaining a reference sequence and a plurality of sequence reads; optionally performing a first alignment of the plurality of sequence reads with the reference sequence, unless the obtained plurality of sequence reads and reference sequence are obtained in an already aligned configuration; identifying a candidate variant position from the aligned sequence reads and reference sequence; augmenting the sequence reads and/or the reference sequence around the candidate variant position to achieve a second alignment of the plurality of sequence reads with the reference sequence; generating a reference matrix for the candidate variant position from the augmented reference sequence and a sample matrix for the candidate variant position from the plurality of augmented sequence reads; inputting the reference matrix and the sample matrix into a neural network; and determining with the neural network whether a variant type exists at the candidate variant position.

In some embodiments, the step of augmenting the sequence reads and/or the reference sequence includes introducing one or more spaces to the sequence reads and/or the reference sequence to account for insertions and/or deletions in the sequence reads.

In some embodiments, the method further includes generating a plurality of training matrices from a training dataset, wherein the training matrices have a structure that corresponds to the sample matrix and the reference matrix, wherein the training dataset includes sequence data that comprises a plurality of mutations, the mutations including single nucleotide variants, insertions, and deletions; and training the neural network with the plurality of training matrices.

In some embodiments, the training dataset includes a plurality of subsets, wherein each subset includes a tumor purity level ranging from 0% to 100%, wherein at least two of the subsets each has a different tumor purity level.

In some embodiments, at least three of the subsets each has a different tumor purity level.

In some embodiments, the plurality of subsets includes a first subset with a tumor purity level less than about 30%, a second subset with a tumor purity level between about 30% and 70%, and a third subset with a third tumor purity level of at least about 70%.

In some embodiments, the plurality of subsets includes a first subset with a tumor purity level less than about 40%, a second subset with a tumor purity level between about 40% and 60%, and a third subset with a tumor purity level of at least about 60%.

In some embodiments, the plurality of subsets includes a subset with a tumor purity level of less than about 10%.

In some embodiments, the plurality of subsets includes a subset with a tumor purity level of less than about 5%.

In some embodiments, the training dataset includes synthetic data.

In some embodiments, the synthetic data includes artificially generated mutations, wherein the artificially generated mutations comprise single nucleotide variants, insertions, and deletions.

In some embodiments, the training dataset includes real data, wherein the real data includes real mutations, wherein the real mutations include single nucleotide variants, insertions, and deletions.

In some embodiments, the training dataset includes a plurality of subsets, wherein each subset includes a variant allele frequency ranging from 0% to 100%, wherein at least two of the subsets each has a different variant allele frequency level.

In some embodiments, at least three of the subsets each has a different variant allele frequency level.

In some embodiments, at least one of the subsets has a variant allele frequency of at least 2.5%.

In some embodiments, at least one of the subsets has a variant allele frequency of at least 5%.

In some embodiments, at least one of the subsets has a variant allele frequency of at least 10%.

In some embodiments, the method further includes inputting at least one prediction from at least one mutation calling algorithm into the neural network.

In some embodiments, the at least one prediction includes at least three predictions from at least three separate mutation calling algorithms.

In some embodiments, the at least one prediction includes at least five predictions from at least five separate mutation calling algorithms.

In some embodiments, the training dataset includes a mixture of synthetic data and real data.

In some embodiments, the training dataset includes at least 5% synthetic data.

In some embodiments, the training dataset includes at least 10% synthetic data.

In some embodiments, the training dataset includes whole genome sequencing data.

In some embodiments, the training dataset includes whole exome sequencing data.

In some embodiments, the training dataset includes targeted sequencing data.

In some embodiments, the training dataset includes data obtained from a formalin-fixed paraffin-embedded sample.

In some embodiments, the training dataset includes at least two of whole genome sequencing data, whole exome sequencing data, targeted sequencing data, and data obtained from a formalin-fixed paraffin-embedded sample.

In some embodiments, the training dataset includes at least three of whole genome sequencing data, whole exome sequencing data, targeted sequencing data, and data obtained from a formalin-fixed paraffin-embedded sample.

In some embodiments, the training dataset includes whole genome sequencing data, whole exome sequencing data, targeted sequencing data, and data obtained from a formalin-fixed paraffin-embedded sample.

In some embodiments, a method for variant calling is provided. The method can include obtaining a reference sequence, a plurality of tumor sequence reads, and a plurality of normal sequence reads; optionally performing a first alignment of the plurality of tumor sequence reads and the plurality of normal sequence reads with the reference sequence, unless the obtained plurality of tumor sequence reads and the plurality of normal sequence reads and the reference sequence are obtained in an already aligned configuration; identifying a candidate variant position from the aligned tumor sequence reads, normal sequence reads, and reference sequence; augmenting the tumor sequence reads and/or the normal sequence reads, and/or the reference sequence around the candidate variant position to achieve a second alignment of the plurality of tumor sequence reads and the plurality of normal sequence reads with the reference sequence; generating a reference matrix for the candidate variant position from the augmented reference sequence and a tumor matrix for the candidate variant position from the plurality of augmented tumor sequence reads and a normal matrix for the candidate variant position from the plurality of augmented normal sequence reads; inputting the reference matrix, the tumor matrix, and the normal matrix into a neural network; and determining with the neural network whether a variant type exists at the candidate variant position.

In some embodiments, the method further includes generating a plurality of training matrices from a training dataset, wherein the training matrices have a structure that corresponds to the tumor matrix, normal matrix and the reference matrix, wherein the training dataset includes tumor sequence data and normal sequence data; and training the neural network with the plurality of training matrices.

In some embodiments, both the tumor sequence data and the normal sequence data include a plurality of mutations, the mutations including single nucleotide variants, insertions, and deletions.

In some embodiments, the normal sequence data includes up to 5% tumor sequence data.

In some embodiments, the normal sequence data includes up to 10% tumor sequence data.

In some embodiments, the tumor sequence data includes a tumor purity level between about 10% to 100%.

In some embodiments, the training dataset includes a plurality of tumor sequence data subsets, wherein each tumor sequence data subset comprises a tumor purity level ranging from 10% to 100%, wherein at least two of the tumor sequence data subsets each has a different tumor purity level.

In some embodiments, at least three of the tumor sequence data subsets each has a different tumor purity level.

In some embodiments, the plurality of tumor sequence data subsets includes a first tumor sequence data subset with a tumor purity level less than about 30%, a second tumor sequence data subset with a tumor purity level between about 30% and 70%, and a third tumor sequence data subset with a tumor purity level of at least about 70%.

In some embodiments, the plurality of tumor sequence data subsets includes a first tumor sequence data subset with a tumor purity level less than about 40%, a second tumor sequence data subset with a tumor purity level between about 40% and 60%, and a third tumor sequence data subset with a tumor purity level of at least about 60%.

In some embodiments, the training dataset includes synthetic data.

In some embodiments, the synthetic data includes artificially generated mutations, wherein the artificially generated mutations comprise single nucleotide variants, insertions, and deletions.

In some embodiments, the training dataset includes real data, wherein the real data includes real mutations, wherein the real mutations include single nucleotide variants, insertions, and deletions.

In some embodiments, the training dataset includes whole genome sequencing data.

In some embodiments, the training dataset includes whole exome sequencing data.

In some embodiments, the training dataset includes targeted sequencing data.

In some embodiments, the training dataset includes data obtained from a formalin-fixed paraffin-embedded sample.

In some embodiments, a system is provided.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
FIG. 1 illustrates an exemplary system comprising a sequencing device and a computing system, in accordance with some embodiments;
FIGS. 2a and 2b illustrate exemplary methods for germline variant calling, in accordance with some embodiments;
FIG. 3 illustrates an augmentation step of the exemplary method of FIG. 2, in accordance with some embodiments;
FIG. 4 illustrates exemplary data that can be provided to and obtained from a neural network, in accordance with some embodiments;
FIG. 5 illustrates an exemplary architecture of a neural network, in accordance with some embodiments;
FIG. 6 illustrates an exemplary method for somatic variant calling, in accordance with some embodiments;
FIG. 7 illustrates an augmentation step that can be performed as part of the exemplary method of FIG. 6, in accordance with some embodiments;
FIG. 8 illustrates another example of data that can be provided to and obtained from a neural network, in accordance with some embodiments;
FIG. 9 illustrates an exemplary architecture of a neural network, in accordance with some embodiments;
FIG. 10a illustrates the generation of an input matrix for a given candidate somatic SNV; FIG. 10b illustrates the input matrix and the network architecture; FIG. 10c illustrates the generation of an input matrix for a given candidate somatic deletion; FIG. 10d illustrates the generation of an input matrix for a given candidate somatic insertion;
FIG. 11a-11c illustrate the performance of different detection methods on the Platinum two sample mixture dataset;
FIGS. 12a-12c illustrate the performance of different detection methods on the Dream Stage 3 dataset;
FIGS. 13a-13c illustrate the performance of different detection methods on the Dream Stage 4 dataset;
FIGS. 14a-14c illustrate the performance of two different detection methods on the PacBio dataset;
FIGS. 15a-15b illustrate the impact of sequence coverage on the performance of different detection methods on the whole-exome sample mixture dataset;
FIGS. 16a-16d illustrate the performance of different detection methods on the Platinum tumor spike dataset;
FIGS. 17a and 17c illustrate the performance of different somatic mutation detection methods on a whole exome dataset; FIGS. 17b and 17d illustrate the performance of somatic mutation detection methods on a targeted panel dataset;
FIG. 18a illustrates the performance analysis of using models trained on whole-genome and whole-exome to test on exome mixture dataset; FIG. 18b illustrates the performance analysis of using models trained on whole-genome and whole-exome to test on targeted panel mixture dataset;
FIG. 19 illustrates the size distribution of ground truth INDELs in Dream Stage 3, Dream Stage 4, Platinum two sample mixture, Platinum tumor spike, PacBio, and exome datasets;
FIG. 20a and 20b FIG. 20a illustrate the performance analysis of various detection methods on INDELs based on position and type of the predicted somatic mutations for various datasets;
FIG. 21 illustrates the performance analysis of cross sample training for Dream challenge Stage 3 dataset;
FIG. 22a shows the performance of different somatic mutation detection methods on real dataset COLO-829; FIG. 22b shows the performance of different somatic mutation detection methods on real dataset CLL1; FIG. 22c shows the performance of different somatic mutation detection methods on real dataset TCGA-AZ-6601;
FIG. 23 is a list of 261 TCGA cancer samples used for Microsoft Azure experiment;
FIGS. 24a-24m illustrate the different network architectures tested;
FIG. 25 shows the performance analysis of different network architectures shown in FIGS. 24a-24m;
FIGS. 26a and 26b illustrate a run-time comparison of different somatic mutation detection algorithms;
FIG. 27 illustrates VAF distribution of the ground truth SNV and INDEL somatic mutations in the super set of calls for HCC1395 classified by SEQC-II consortium to four confidence levels (High, Med, Low, and Unclassified);
FIGS. 28a-28e show NeuSomatic's overall performance on 123 replicates in SEQC-II dataset;
FIGS. 29a-29d show a performance comparison of various models on 21 WGS replicates dataset. FIG. 29a shows the F1-score (%) and FIG. 29b shows the Precision-Recall comparison across different somatic mutation callers. Here, the SEQC-WGS-GT50-SpikeWGS 10 trained models were used for NeuSomatic and NeuSomatic-S. FIG. 29c shows the F1-score (%) comparison for various NeuSomatic trained models. FIG. 29d shows F1-score (%) performance of different somatic mutation detection methods and NeuSomatic network trained models on the WGS dataset;

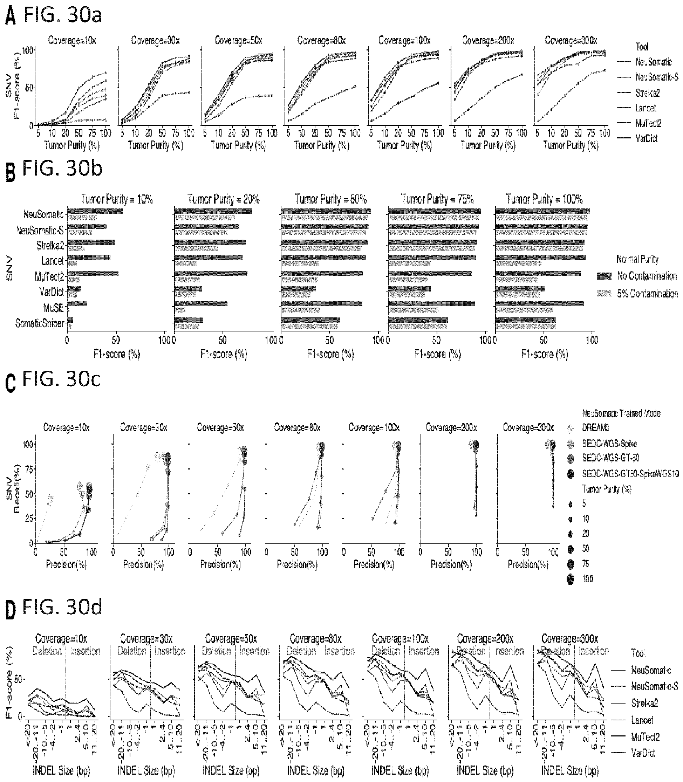

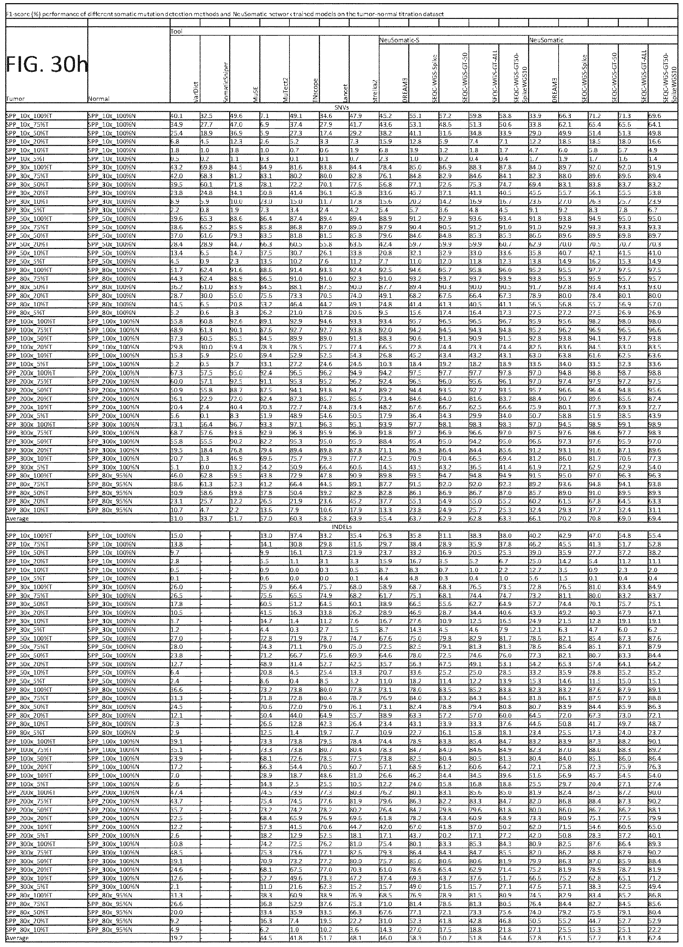

FIGS. 30a-30d shows a performance comparison on Tumor-purity dataset. FIG. 30a shows SNV and INDEL F1-score (%) comparison for different somatic mutation callers across different coverages (10×-300×) and tumor purities (5%-100%). FIG. 30b shows robustness to tumor contamination in the matched normal: F1-score (%) change when matched normal was contaminated with 5% of tumor is shown versus the F1-score (%) at pure normal for 80× coverage and 5-100% tumor purities. For FIG. 30a and FIG. 30b, the SEQC-WGS-GT50-SpikeWGS10 trained models were used for NeuSomatic andNeuSomatic-S. FIG. 30c shows F1-score (%) comparison for various NeuSomatic trained models. FIG. 30d shows performance analysis for different INDEL sizes. Negative INDEL sizes reflect deletion;
FIGS. 30e and 30f show Precision-Recall analysis on Tumor-purity dataset. FIG. 30e shows SNV and FIG. 30f shows INDEL accuracies are compared for different somatic mutation callers across different coverages (10x-300x) and tumor purities (5%-100%);
FIG. 30g shows Precision-Recall comparison for various NeuSomatic-S trained models on the tumor normal titration dataset;
FIG. 30h shows F1-score (%) performance of different somatic mutation detection methods and NeuSomatic network trained models on the tumor-normal titration dataset;

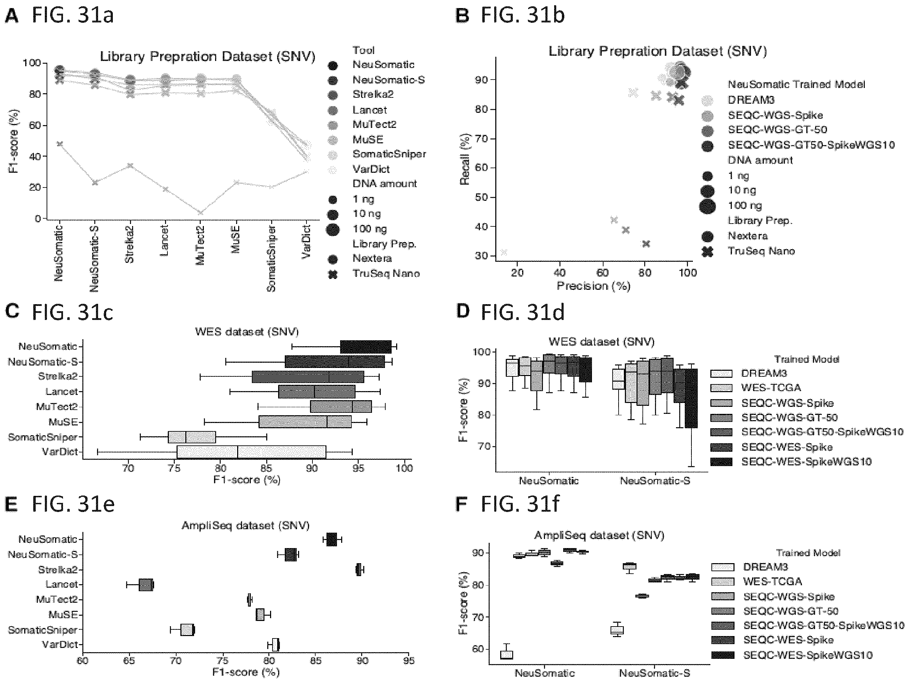

FIGS. 31a-31f show a performance comparison of library prep, WES, and AmpliSeq datasets. FIG. 31a shows SNV and INDEL F1-score (%) comparison for different somatic mutation callers across different library-kits and DNA amounts. FIG. 31b shows Precision-Recall comparison for various NeuSomatic trained models for different library-kits and DNA amounts. FIGS. 31c and 31e show SNV F1-score (%) comparison for different somatic mutation callers. FIGS. 31d and 31f show F1-score (%) comparison for various NeuSomatic trained models. For FIG. 31a, FIG. 31c, and FIG. 31e, the SEQC-WGS-GT50-SpikeWGS10 trained models were used for NeuSomatic and NeuSomatic-S;
FIG. 31g shows Precision-Recall comparison for various NeuSomatic-S trained models on the Library preparation dataset;
FIG. 31h shows F1-score (%) performance of different somatic mutation detection methods and NeuSomatic network trained models on the library-preparation dataset;
FIG. 31i shows F1-score (%) performance of different somatic mutation detection methods and NeuSomatic network trained models on the WES dataset;
FIG. 31j shows F1-score (%) performance of different somatic mutation detection methods and NeuSomatic network trained models on the AmpliSeq dataset;

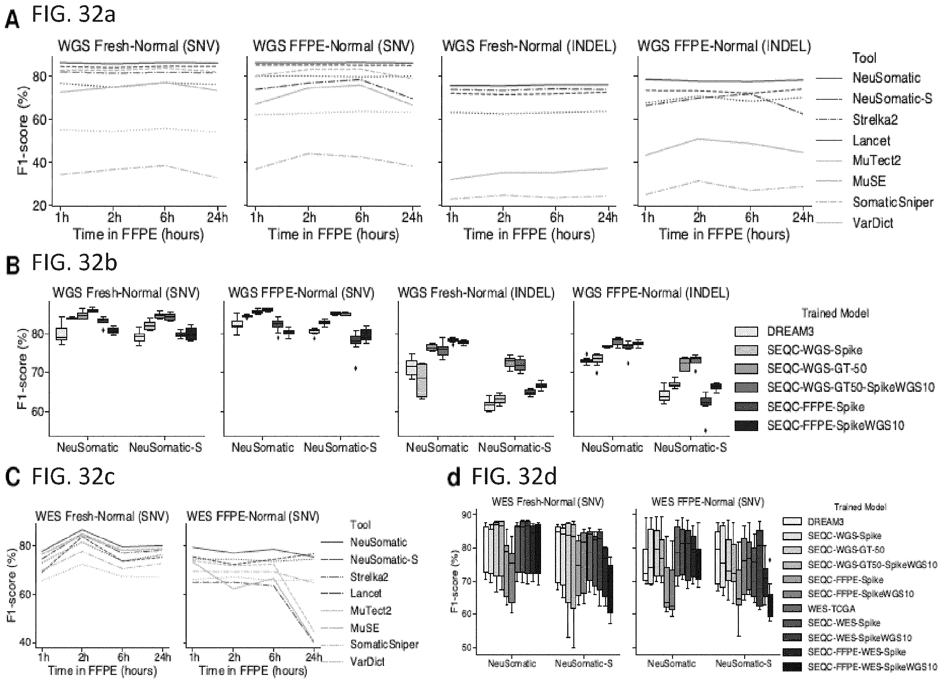

FIGS. 32a-32d show Performance comparison of FFPE dataset. FIGS. 32a and 32b, show performance on 16 FFPE WGS replicates. FIGS. 32c and 32d show performance on 14 FFPE WES replicates. FIGS. 32a and 32c show F1-score (%) comparison for different somatic mutation callers across FFPE and fresh normal samples. FIGS. 32b and 32d show F1-score (%) comparison for various NeuSomatic trained models. FIG. 32b shows Precision-Recall plot of 16 FFPE WGS replicates for different somatic mutation callers with FFPE and fresh normal. For FIGS. 32a and 32c, the SEQC-WGS-GT50-SpikeWGS10 trained models were used for NeuSomatic and NeuSomatic-S;
FIG. 32e shows F1-score (%) performance of different somatic mutation detection methods and NeuSomatic network trained models on the FFPE WGS dataset;
FIG. 32f shows F1-score (%) performance of different somatic mutation detection methods and NeuSomatic network trained models on the FFPE WES dataset;
FIGS. 33a and 33b are charts analyzing the impact of training on the target sample for nine replicates from different datasets using (FIG. 33a) NeuSomatic and (FIG. 33b) NeuSomatic-S. For each sample, two sample specific models were used: one only trained on the target sample, and the other with an additional 10% of the training data from SEQC-WGSSpike model;
FIG. 33c shows F1-score (%) performance for the SEQC-WGS-Spike model and two sample-specific NeuSomatic network trained models;
FIGS. 34a-34d show performance analysis of different INDEL sizes, different VAF distributions, and on difficult regions. FIGS. 34a and 34b show performance analysis for different INDEL sizes across SEQC-II datasets using different callers and different training approaches. Negative INDEL sizes reflect deletion. FIG. 34c shows performance analysis for mutations with different VAF ranges across SEQC datasets using different callers. FIG. 34d show performance analysis of different somatic callers on difficult regions including tandem repeats (TR) of different sizes and segmental duplications;
FIG. 35 illustrates performance analysis for mutations with different VAF ranges across SEQC datasets using different callers;
FIGS. 36a and 36b show performance analysis for mutations with different VAF ranges across SEQC datasets using different training approaches for (FIG. 36a) NeuSomatic and (FIG. 36b) NeuSomatic-S;
FIGS. 37a-37c show performance analysis for mutations with different VAF ranges on Tumor/Normal titration dataset using different (FIGS. 37a) callers and (FIGS. 37b and 37c) training approaches for (FIG. 37b) NeuSomatic and (FIG. 37c) NeuSomatic-S;
FIG. 38 show performance comparison on whole genome versus the difficult genomic regions including tandem repeats (TR) of different sizes and segmental duplications using different callers;
FIGS. 39a and 39b show performance analysis of different training approaches on difficult regions including tandem repeats (TR) of different sizes and segmental duplications for (FIG. 39a) NeuSomatic and (FIG. 39b) NeuSomatic-S;
FIGS. 40a and 40b show performance comparison on whole genome versus the difficult genomic regions including tandem repeats (TR) of different sizes and segmental duplications using different training approaches for (FIG. 40b) NeuSomatic and (FIG. 40c) NeuSomatic-S;
FIGS. 41a-41c show performance analysis on difficult regions including tandem repeats (TR) of different sizes and segmental duplications on Tumor/Normal titration dataset using different (FIG. 41a) callers and (FIGS. 41b and 41c) training approaches for (FIG. 41b) NeuSomatic and (FIG. 41c) NeuSomatic-S; and
FIG. 42 shows the violin-plot comparison of the VAF distribution of private FN calls on WGS dataset.

### DETAILED DESCRIPTION

The present disclosure describes, among other things, a method for germline variant calling, which can include obtaining a reference sequence, a plurality of sequence reads, and a position of a candidate variant within the sequence reads; obtaining augmented sequence reads by inserting one or more spaces in one or more sequence reads; obtaining an augmented reference sequence by inserting one or more spaces in the reference sequence; converting a segment of the augmented sequence reads around the candidate variant into a sample matrix; converting a segment of the augmented reference sequence around the candidate variant into a reference matrix; providing the sample matrix and the reference matrix to a trained neural network; and obtaining, at the output of the trained neural network, prediction data related to a variant within the plurality of sequence reads. The disclosed systems and methods allow for capturing of important variant signals directly from the raw data and to consistently achieve high accuracy for different sequencing technologies, sample purities, and sequencing strategies such as whole-genome vs. target enrichment.

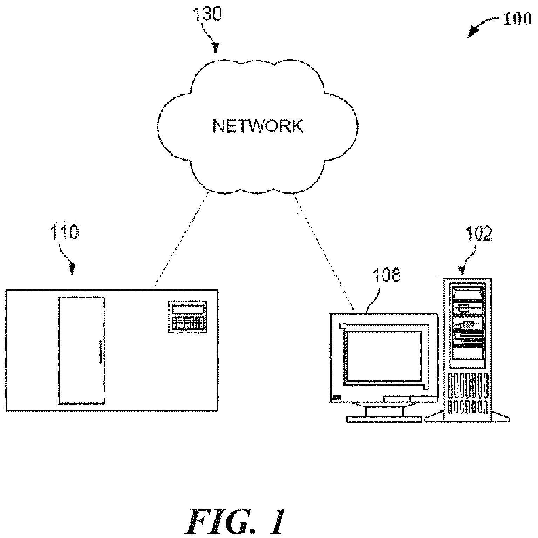

FIG. 1 sets forth an illustrative system 100 including a sequencing device 110 communicatively coupled to a computing system 102. Sequencing device 110 can be coupled to computing system 102 either directly (e.g., through one or more communication cables) or through network 130, which may be the Internet or any other combination of wide-area, local-area, wired, and/or wireless networks. In some embodiments, computing system 102 may be included in or integrated with the sequencing device 110. In some embodiments, sequencing device 110 may sequence a sample containing genetic material and produce resulting sequencing data. The sequencing data can be sent to computing system 102 (e.g., through network 130) or stored on a storage device and at a later stage transferred to computing system 102 (e.g., through network 130). In some embodiments, computing system 102 may or may not include a display 108 and one or more input devices (not illustrated) for receiving commands from a user or operator (e.g. a technician or a geneticist). In some embodiments, computing system 102 and/or sequencing device 110 can be accessed by users or other devices remotely through network 130. Thus, in some embodiments various methods discussed herein may be run remotely on computing system 102.

Computing system 102 may include one computing device or a combination of a number of computing devices of any type, such as personal computers, laptops, network servers (e.g., local servers or servers included on a public/private/hybrid cloud), mobile devices, etc., where some or all of the devices can be interconnected. Computing system 102 may include one or more processors (not illustrated), each of which can have one or more cores. In some embodiments, computing system 102 can include one or more general-purpose processors (e.g., CPUs), special-purpose processors such as graphics processors (GPUs), digital signal processors, or any combination of these and other types of processors. In some embodiments, some or all processors in computing system can be implemented using customized or customizable circuitry, such as application specific integrated circuits (ASICs) or field programmable gate arrays (FPGAs). Computing system 102 can also in some embodiments retrieve and execute non-transitory computer-readable instructions stored in one or more memories or storage devices (not illustrated) integrated into or otherwise communicatively coupled to computing system 102. The memory/storage devices can include any combination of non-transitory computer readable storage media including semiconductor memory chips of various types (DRAM, SRAM, SDRAM, flash memory, programmable read-only memory) and so on. Magnetic and/or optical disks can also be used. The memories/storage devices can also include removable storage media that can be readable and/or writeable; examples of such media include compact disc (CD), read-only digital versatile disc (e.g., DVD-ROM, dual-layer DVD-ROM), read-only and recordable Blu-ray^{®} disks, ultra-density optical disks, flash memory cards (e.g., SD cards, mini-SD cards, micro-SD cards, etc.), and so on. In some embodiments, data and other information (e.g. sequencing data) can be stored in one or more remote locations, e.g., cloud storage, and synchronized with other the components of system 100.

### Germline Variant Calling

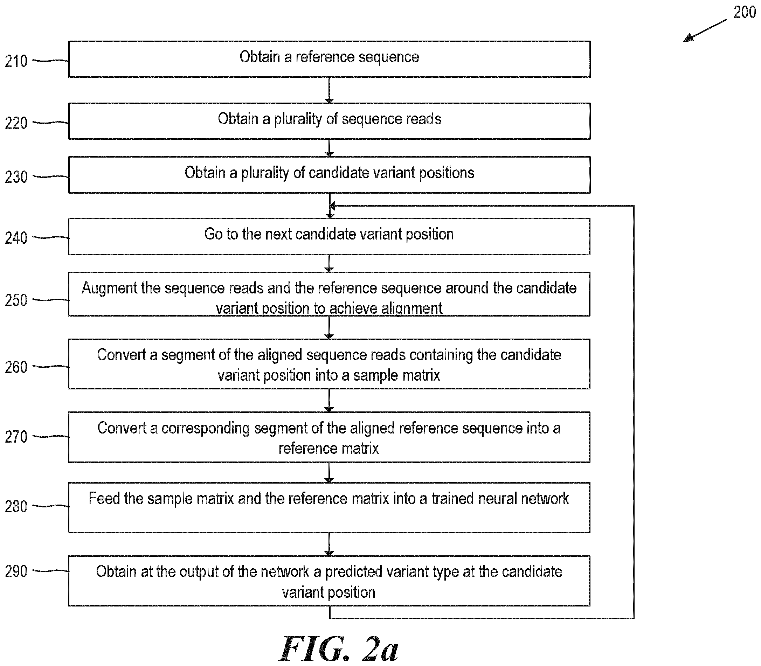

FIG. 2a illustrates an exemplary method 200 of germline variant calling, in accordance with some embodiments. Method 200 may be implemented, for example, in the form of software (i.e., a set of instructions stored in one or more non-transitory mediums accessible and executable by one or more processors, e.g., of computing system 102), or in the form of firmware, hardware, or any combination thereof.

Method 200 may begin at step 210 where a reference sequence may be obtained. The reference sequence may be obtained, for example, from one or more private or public repositories such as the Reference Sequence (RefSeq), an open-access, annotated and curated collection of nucleotide base sequences built by the National Center for Biotechnology Information (NCBI) or the NCBI Genomes FTP site storing a set of complete genomes of different organisms. It should be understood that in some embodiments, a specific copy of the reference sequence may be stored locally (e.g., in a memory of computing system 102), while in other embodiments the reference sequence may be obtained from a remote server, e.g., through network 130. Furthermore, in some embodiments, the entire reference sequence may be obtained while in other embodiments one or more sections of the reference sequence may be obtained - e.g., only the section(s) that is/are associated with a particular assay. Thus, a "reference sequence" as used herein refers generally to one or more sections of the reference sequence, which may or may not include the entire reference sequence.

At step 220, a plurality of sequence reads corresponding to a genetic sample (e.g., a sample comprising a patient's DNA or RNA material) as sequenced by sequencing device 110 can be obtained. As discussed above, the sequence reads can be obtained either directly from sequencing device 110, or from one or more local or remote volatile or non-volatile memories, storage devices, or databases communicatively coupled to computing system 102. The obtained sequence reads can be already pre-processed (e.g., pre-aligned) or they can be "raw," in which case method 200 may also include a preprocessing (e.g., pre-aligning) step (not illustrated). Also, while in some embodiments, entire sequence reads (as generated by sequencing device 110) can be obtained, in other embodiments only sections of the sequence reads can be obtained. Thus, "obtaining a sequence read," as used herein, refers generally to obtaining one or more sections of one or more (e.g., adjacent) sequence reads.

At step 230, a plurality of candidate variant positions within the sequence read can be obtained. In some embodiments, this includes scanning the plurality of obtained sequence reads, comparing them to the obtained reference sequence, and determining a set of one or more positions within the sequence read that appear to include variants of some type (e.g., insertion or deletion variants (INDELs), single-nucleotide variants (SNVs), or structural variants (SVs)). Finding the candidate variant position can include, for example, checking all positions in the reference and determining positions for which at least one of the sequence reads differ from the reference. In determining the candidate positions some filters can also be used, for example filters on the number or the percentage of reads that must different from the reference for a particular position to be considered a candidate variant position. In other embodiments, the sequence reads could have already been scanned and analyzed (e.g., by a separate software and/or hardware module), in which case the plurality of candidate positions can be obtained from a memory or a database coupled to computing system 102. At step 240, the method can proceed to the next candidate variant position from the plurality of identified candidate variant positions.

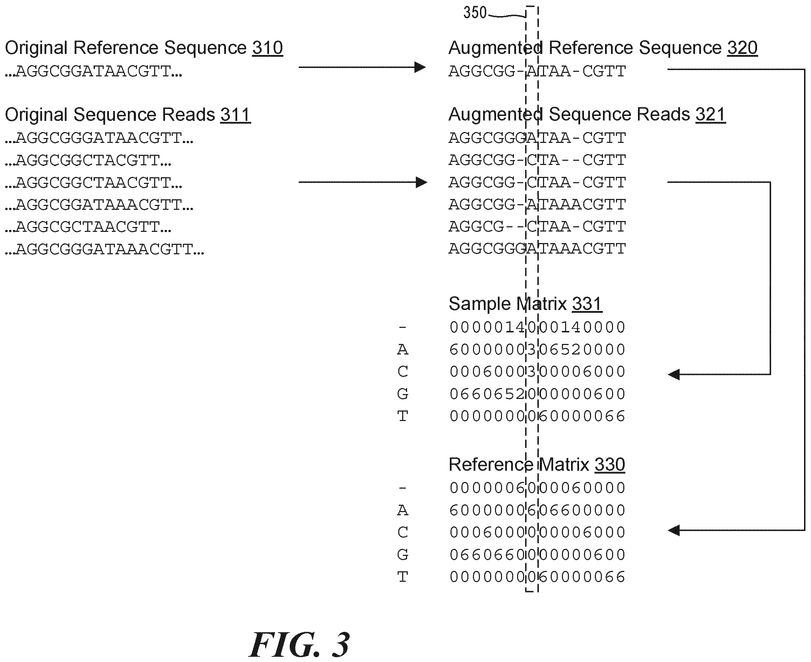

At step 250, the sequence reads and the reference sequence around the current candidate variant position are augmented to achieve a precise multiple sequence alignment (MSA). This step can be better illustrated in conjunction with FIG. 3, which shows a plurality of exemplary sequence reads 311, an exemplary reference sequence 310, and an exemplary candidate variant position 350. As illustrated in FIG. 3, augmenting reference sequence 310 and sequence reads 311 may include inserting one or more gaps or spaces (e.g., spaces 325) within reference sequence 310 and/or within one or more sequence reads 311, thereby producing an augmented reference sequence 320 and an augmented plurality of sequence reads 321 precisely aligned to augmented reference sequence 320.

Specifically, as illustrated in FIG. 3, in some embodiments, the augmentation may include: determining or detecting an inserted base in a particular sequence read; inserting a space at the position of the inserted base in the reference sample; inserting a space at the same position in every sequence read in which no insertion was detected at the same position (which in some cases corresponds to all sequence reads other than the particular sequence read where insertion was detected); and repeating the process for each detected inserted base in each sequence read. It is appreciated that in places where a potential insertion of two bases or longer is detected, two or more spaces can be inserted, respectively. It is appreciated that other suitable techniques can be used for inserting spaces in the sequence reads and the reference sequence such that at the end a precise multiple sequence alignment (MSA) can be achieved, meaning that all bases within the augmented sequence reads (or at least those locations where no deletion or insertion occurred) are precisely aligned to each other and to the augmented reference sequence.

In some embodiments, the augmentation can also include detecting a deleted base in a particular sequence read and inserting a space in that sequence read at the position of the potential deletion variant. It is appreciated that in the case of a detected deleted base, the reference sequence need not be augmented.

It is further appreciated that "inserting spaces" may or may not include an actual modification of the memory where the sequence reads and the reference sequences are stored, and may instead be an inherent or implied part of other operations (e.g., other steps of method 200) achieving the same end result. For example, instead of modifying the original location of the sequence reads and reference sequence, the sequence reads and the reference sequence can be selectively copied from the original location into a separate memory location in a manner that would cause the new copy to store spaces in all the right positions. "Storing a space" as used herein can mean storing any random or predefined value (e.g., 0) other than the four values corresponding to the four types of nucleotide bases (A, C, T, and G). As another example, spaces may not be physically inserted at any step, but may be algorithmically accounted for when the sequence reads and the reference sequence are converted into count matrices, as described below.

In some embodiments, augmenting the sequence reads and the reference sequence around the candidate variant position can mean augmenting only a portion of a variable or predefined length that includes the candidate variant position. In other embodiments, however, the entire length of available sequence reads and reference sequence can be augmented at once, in which case step 250 can be performed only once (e.g., before step 240 or even before step 230) and not be repeated for every candidate variant position.

At step 260, a segment (i.e., a window) of the augmented sequence reads can be converted (or "summarized") into a sample matrix. The segment can be of a fixed length and may or may not place the candidate variant position in the middle. For instance, in the example illustrated in FIG. 3, the augmented segment is 16 columns (positions) long, and includes 7 columns to the left and 8 columns to the right of candidate variant position 350, placing candidate variant position 350 substantially in the middle of the augmented segment.

In some embodiments, the sample matrix can be a count matrix having the same number of columns as the segment being converted. The matrix can include one row for each type of nucleotide base (A, C, G, and T), each row representing the number of times the respective nucleotide base appeared at each position (column) within the augmented sequence reads. Referring to the example of FIG. 3, row "A" in sample matrix 331 includes values "6000000306520000," indicating that base "A" appeared 6 times at the first position within the segment of augmented reads; 3 times at the eighth position; 6 times at the tenth position; 5 times at the eleventh position; twice at the twelfth position; and 0 times (did not appear) at any other position within the segment.

In some embodiments, the sample matrix can also include a row representing the number of times a space (represented in FIG. 3 as "-") appeared at each position within the augmented sequence reads. For example, in FIG. 3, the first row of sample matrix 331 (marked as "-") includes values "0000014000140000," indicating that the augmented sequence reads include 1, 4, 1, and 4 spaces at positions 6, 7, 11, and 12 within the segment, respectively. It is appreciated that while the space row "-" is shown as the first row of the matrix in the example of FIG. 3, in other embodiments, the space row can be placed at other places (e.g., last) within the matrix. It is also appreciated that the row/column notation is arbitrary and that in other embodiments the notation can be reversed, meaning that sample matrix could have 5 columns (A, C, T, G, -) and the number of rows corresponding to the length of the segment. Accordingly, the matrix can be generally described as having at least five "lines" (rows or columns), one line representing counts of each nucleotide type, and at least one line representing space counts within the augmented sequence reads.

At step 270, a segment of the augmented reference sequence corresponding to the segment of sequence reads) can be similarly converted into a reference matrix having the same dimensions as the sample matrix. For example, in FIG. 3 augmented reference 320 is converted into reference matrix 330. Similarly to sample matrix 331, reference matrix 330 can have four rows indicating the number of occurrences of each type of nucleotide at each position within the augmented reference sequence, and a space row indicating the number of spaces at each position within the augmented reference sequence. It is appreciated, however, that in other embodiments, reference matrix 330 can have a different summary/representation of the different bases of the reference sequence, than the one illustrated in FIG. 3.

In the embodiment illustrated in FIG. 3, reference matrix 320 is also normalized to have the same range of values as sample matrix 331. As illustrated in FIG. 3, the normalization may include multiplying each count by a normalization factor, such as the total number of sequence reads (in this example, by 6). It is appreciated that in other embodiments, instead of normalizing the reference matrix, normalization can be performed on the sample matrix, or on both matrices. In yet other embodiments, the matrices may not need to be normalized at all. It is also appreciated that step 270 does not need to follow step 260 and can in some embodiments be performed before or in parallel to step 260.

At step 280, the reference matrix and the sample matrix can be provided as inputs into a trained deep neural network, and at step 290, an output of the trained deep neural network can be obtained, where the output can include various predictions related to a variant included in the sequence reads represented by the sample matrix, as discussed in more detail below.

It should also be understood by a person skilled in the art that unless clearly indicated to the contrary, the number and the order of the steps in method 200 is not necessarily limited to the number and order of steps as illustrated in FIG. 2a. Thus, in different embodiments, some steps of method 200 can be reordered or performed in parallel; some steps can be made optional or omitted; and some additional steps can be added without departing from the spirit and scope of this disclosure.

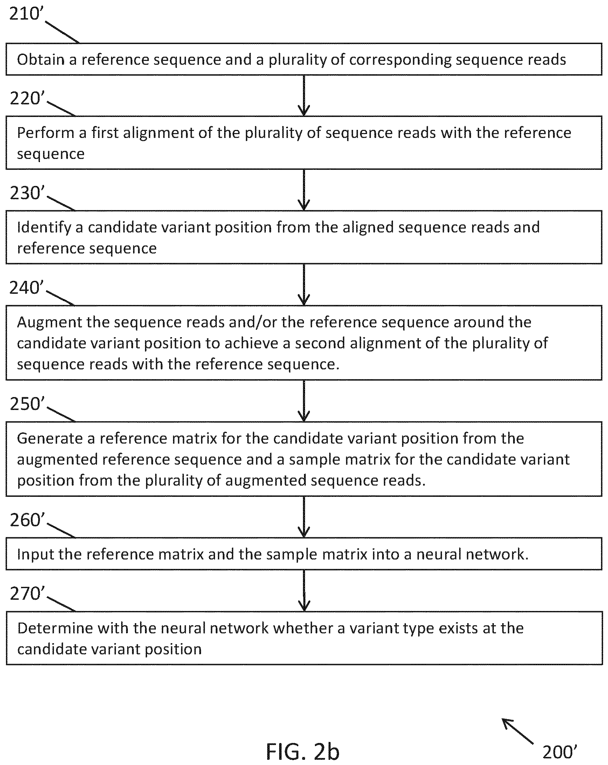

For example, FIG. 2b illustrates another embodiment where at step 210', a reference sequence and a plurality of corresponding sequence reads are obtained. The reference sequence and the plurality of corresponding sequence reads can be obtained in any order (i.e., sequentially or simultaneously). Next, in step 220', the reference sequence and the plurality of corresponding sequence reads are aligned. In some embodiments, the reference sequence and the plurality of corresponding sequence reads may be obtained in an aligned format, which may eliminate the need for performing the first alignment step. At step 230', a candidate variant position is identified from the aligned sequence reads and reference sequence. At step 240', the sequence reads and/or the reference sequence are augmented around the candidate variant position to achieve a second alignment of the plurality of sequence reads with the reference sequence, in the same manner as described above (i.e. by inserting gaps to account for insertions and/or deletions).

In some embodiments, a window of about 2 to 5, 10, 15, 20, 25, 30, 35, 40, 45, 50, 55, 60, 65, 70, 75, 80, 85, 90, 95, or 100 bases on either of the candidate variant position can be augmented and aligned. In some embodiments, a window of at least about 5, 10, 15, 20, 25, 30, 35, 40, 45, 50, 55, 60, 65, 70, 75, 80, 85, 90, 95, or 100 bases on either side of the candidate variant position can be augmented and aligned. In some embodiments, a window of no more than about 5, 10, 15, 20, 25, 30, 35, 40, 45, 50, 55, 60, 65, 70, 75, 80, 85, 90, 95, or 100 bases on either side of the candidate variant position can be augmented and aligned.

At step 250', a reference matrix for the candidate variant position from the augmented reference sequence and a sample matrix for the candidate variant position from the plurality of augmented sequence reads are then generated. As described above, the matrices include the frequencies of each base or gap (i.e. A, C, G, T, or gap) at each sequence position. At step 260', the reference matrix and the sample matrix are input into a trained neural network.

At step 270', the neural network determines whether a variant type exists at the candidate variant position. For example, the neural network can comprise a plurality of convolutional layers that process the reference matrix and the sample matrix and generate an output that is processed by one or more classifiers and regressors (i.e. a mutation type of classifier, and length classifier, and a position regressor) that determine the variant type, size, and position. In some embodiments, the neural network can include up to about 5, 10, 15, 20, 25, 30, 35, 40, 45, 50, 55, 60, 65, 70, 75, 80, 85, 90, 95, or 100 layers. In some embodiments, the convolutional layers can be structured into blocks. In some embodiments, the number of blocks is about half the number of layers.

If the reference sequence and the corresponding sequence reads include more than one variant candidate position, steps 230' to 260' can be repeated for each variant candidate position. In addition, steps 210' to 260' can be repeated by obtaining additional reference sequences and additional corresponding sequence reads.

Although the example illustrated in FIGS. 2a and 2b are described in a section for germline variant calling, these examples can be adapted for somatic variant calling. Conversely, the embodiments described in the somatic variant calling section may also be adapted for the germline variant calling. For example, the plurality of sequence reads described in FIG. 2b, can be subdivided into two groups, normal sequence reads and tumor sequence reads. The normal sequence reads can be obtained from a sample of normal tissue from the subject, while the tumor sequence reads can be obtained from a sample of tumor tissue from the subject. Instead of a generating a single sample matrix, a normal matrix and a tumor matrix would be generated along with the reference matrix. The rest of the method would proceed in a similar manner.

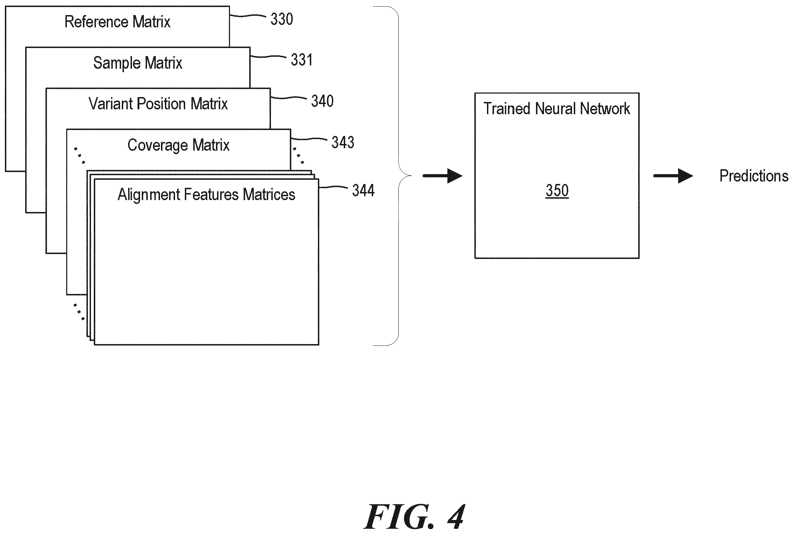

FIG. 4 illustrates an exemplary embodiment in which reference matrix 330 and sample matrix 331 (along with other optional inputs discussed below) are fed into a trained deep neural network 350, which then outputs one or more predictions related to the sequence reads represented by sample matrix 331.

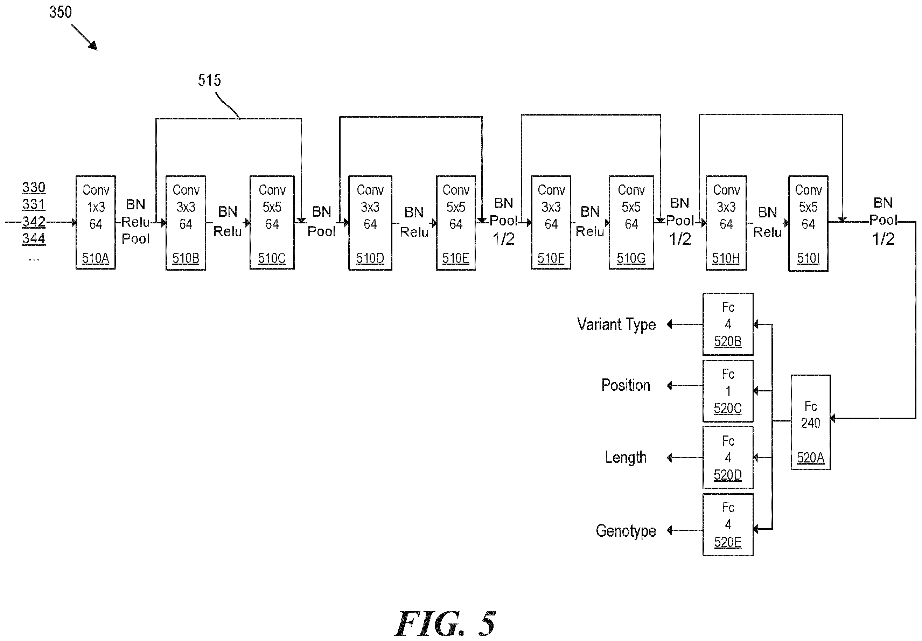

In some embodiments, network 350 can be a convolutional neural network (CNN), an illustrative example of which is shown in FIG. 5. As illustrated in FIG. 5, network 350 may include a plurality of serially connected convolutional layers (510A-510I) eventually feeding into a first fully connected layer 520A, the output of which feeds into four separate fully connected layers 520A-520C that output various predictions. In the example of FIG. 5, different convolutional layers 510 may have different filter sizes (e.g., 1x3, 3x3, or 5x5) and may be interconnected through additional processing layers (not explicitly shown), such as Rectifying Linear Units (ReLUs), pooling layers, batch normalization (BN) layers, etc. Furthermore, as illustrated in FIG. 5, in some embodiments, an input to a certain layer can sometimes be connected via an identity shortcut connection 515 to an output of a subsequent layer in order to help maintain the signal in deeper layers of the network.

In some embodiments, network 350 can output one or more predictions associated with a candidate variant within the segment of augmented sequence reads. As illustrated in the example of FIG. 5, one of such prediction can be obtained at the output of a fully connected layer 520B and can indicate whether the candidate variant position corresponded to a variant and if so the type of that variant. More specifically, in some embodiments, the prediction can include at least four probability values associated with four possible types of variants: NONE (no variant, i.e., a false-positive call); SNP/SNV (single nucleotide polymorphism/variant); INS (insertion variant); and DEL (deletion variant). Based on these probability values it can be determined which type of variant is the most likely, as well as the confidence level of the particular prediction.

In some embodiments, neural network 350 can also output (e.g., at the output of fully connected layer 520C) the predicted position of the variant within the segment of augmented sequence reads represented by sample matrix 331. In other embodiments, this output can be omitted and it can be assumed that the position of the variant is known (e.g., at the center of the segment) based on the manner in which the segment of sequence reads is selected for a given candidate variant, as described above.

In some embodiments, neural network 350 can also output (e.g., at the output of fully connected layer 520D) a predicted length of the variant. For example, a length of "1" can be outputted if the predicted variant is one base long (e.g., a SNP variant or a one-base DEL or INS variant); a length of "2" can be outputted if the predicted variant is a two-base long DEL or INS variant; and so forth. In some embodiments, if the output indicates that a variant is 2 bases long or longer, a post processing step can resolve the exact sequence being inserted or deleted.

In some embodiments, neural network 350 can also output (e.g., at the output of fully connected layer 520E) a predicted genotype associated with the variant, representing, for example, the probabilities with the variant being 1) a homozygous reference (a non-variant); 2) a heterozygous variant (where only one of maternal or paternal copies has a variant); 3) a homozygous variant (where both copies have the same variant); or 4) other (where each copy has a different variant). It is appreciated that in other embodiments, some of these outputs can be omitted and/or additional outputs can be added.

Referring now to the network's inputs, in addition to reference matrix 330 and sample matrix 331 discussed above, in some embodiments other inputs can be provided to further improve the accuracy of the predictions and/or to expand the amount of information that can be obtained at the network's output. As illustrated in the example of FIG. 4, additional inputs may include, for example, a variant position matrix 340, a coverage matrix 343, and one or more alignment feature matrices 344.

In some embodiments, all input matrices can be provided into network 350 as one large three-dimensional matrix. The dimensions of the matrix can be, for example, 5 × s × k, where 5 corresponds to the number of row/columns (-, A, C, T, G); s corresponds to the length of the segment (e.g., 16); and k corresponds to the number of different two-dimensional (e.g., 5 × s) matrices described above. In some embodiments, k can be as high as 30, or even higher.

Variant position matrix 340 may comprise a two dimensional representation of the candidate variant position within the reference and sample matrices. For examples, the matrix can have five rows (-, A, C, T, G) and each column can represent a position within the segment. For example, variant position matrix 340 may include one value (e.g., 1) in all rows in the column corresponding to the candidate variant position, and another value (e.g., 0) in all rows of all other columns.

Coverage matrix 343 may represent the coverage or depth of the particular segment of the sequence reads. For example, in some embodiments, coverage matrix 343 may include the same value in all of its elements, the value representing the coverage/depth (e.g., the average coverage/depth) of the different reads within the segment. In other embodiments, coverage matrix 343 may include different values at different columns, each value representing the respective coverage/depth at each column. Alignment feature matrices 344 can represent various metrics associated with the quality of the sequence reads and their alignment. Such metrics can include, for example, base qualities, mapping qualities, strand bias, clipping information, and so forth. These alignment features can help improve the prediction accuracy by providing more context to sequence reads, for instance, by informing the network about the quality of the sequence bases and/or of their mapping. Additional inputs to network 350 (not illustrated for brevity) may include various data related to known variants. Such data can be obtained, for example, from public and/or private knowledge bases such as dbSNP, COSMIC, ExAC, etc.

It is appreciated that FIG. 5 shows only one exemplary configuration of network 350 and that neural networks having other suitable configurations/architectures can be used to analyze augmented sequence reads (represented by the sample matrix) and to predict the type of variant and its other characteristics, without departing from the scope and spirit of the present disclosure. Such alternative configurations may include fewer layers, additional layers, layers having different parameters, additional inputs or outputs, fewer inputs or outputs, and so forth). Moreover, in some embodiments, network 350 may not be a convolutional neural network (CNN), but may instead be another type of a deep neural network (DNN), i.e., another type of artificial neural network (ANN) that has multiple layers between the input and output layers, without departing from the scope and spirit of the present disclosure.

### Somatic Variant Calling

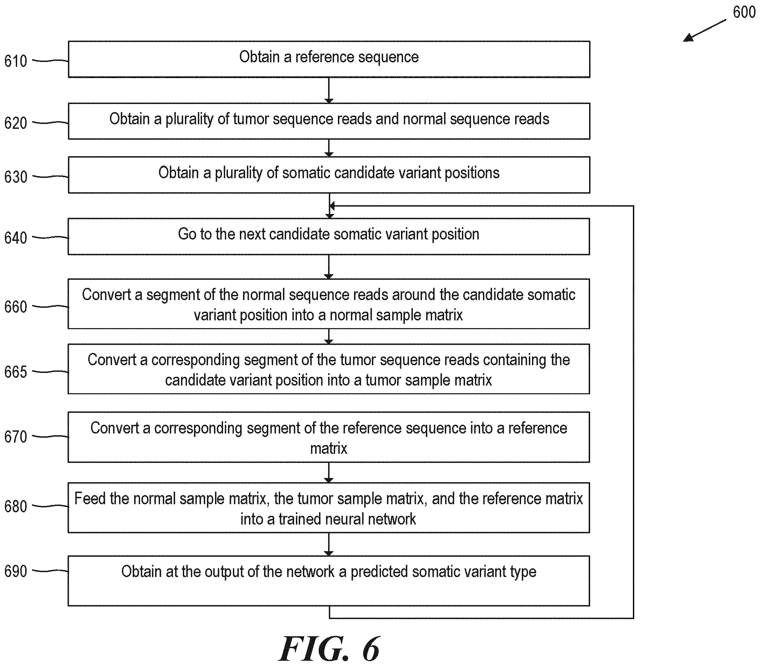

In some embodiments, neural network 350 can also be trained to perform somatic variant calling. FIG. 6 illustrates an exemplary method 600 for somatic variant calling using trained neural network 350.

At step 610, method 600 includes obtaining a reference sequence, for example, in a manner similar to that described above in connection with step 210 of method 200. At step 620, method 600 includes obtaining a plurality of tumor sequence reads and a plurality of corresponding normal (non-tumor) sequence reads. For example, in some embodiments, tumor sequence reads can include sequencing results of a tumor (cancerous) tissue of a patient, and normal sequence reads can include sequencing results of a normal (non-cancerous) tissue taken from the same patient. In other embodiments, the normal sequence reads can include sequencing results of a normal tissue taken from a different patient. Both types of sequence reads can be obtained, for example, in a manner similar to that described above in connection with step 220 of method 200.

At step 630, a plurality of candidate somatic variant positions can be obtained - either by obtaining pre-determined variant positions from a memory or a public/private database, a set of whitelist candidate positions, or by performing a comparison between the tumor sequence reads and the reference sequence, for example, in the manner described above in connection with step 230 of method 200.

At step 640, the method proceeds to one of the plurality of candidate somatic variant positions. At step 660, a segment of the normal sequence reads around the candidate somatic variant position is converted into a normal sample matrix. Similarly, at step 665, a segment of the tumor sequence reads around the candidate somatic variant position (substantially corresponding in length and location to the segment of normal sequence reads) is converted into a tumor sample matrix. At step 670, a segment of the reference sequence around the candidate somatic variant position (substantially corresponding in length and location to the segment of normal sequence reads) is converted into a reference matrix.

In some embodiments, before the above three segments are converted into their respective reference matrices, each of them can be augmented using the augmentation technique described above in connection with step 250 of method 200. In other embodiments, a different type of augmentation technique can be performed on the segments prior to their conversion into matrices; for example, a k-mer collapsing approach can be used where homopolymers of lengths L > k are collapsed to length k, where k is any number greater or equal to one. In yet other embodiments, the augmentation step can be omitted altogether, and the original, unprocessed sequence reads and reference sequence can be converted into their respective matrices directly.

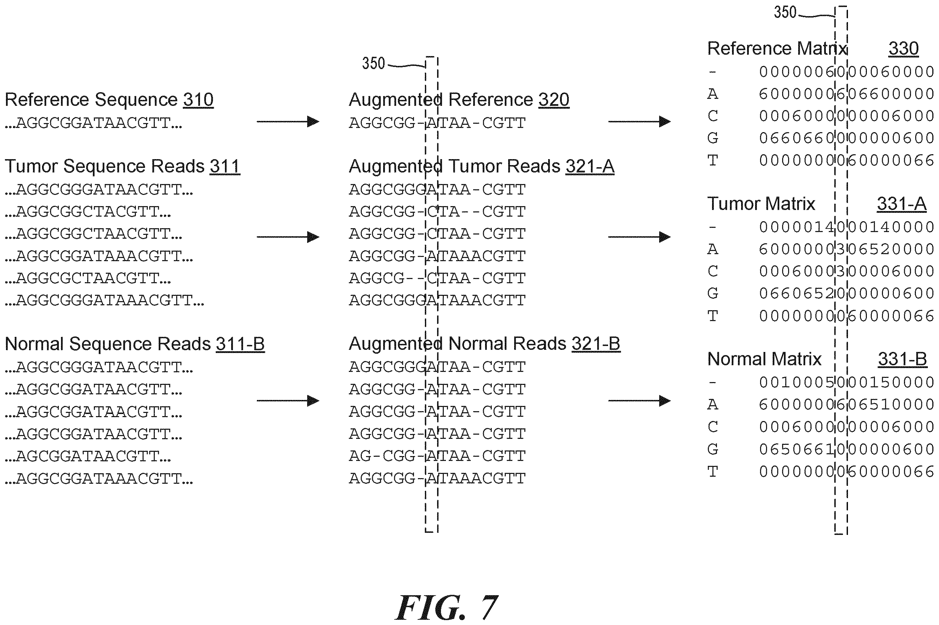

In the example illustrated in FIG. 7, augmentation similar to that performed at step 250 of method 200 is performed. In this example, segments of reference sequence 310, tumor sequence reads 311-A, and normal sequence reads 311-B are first augmented to produce augmented reference sequence 320, augmented tumor sequence reads 321-A, and augmented normal sequence reads 321-B, respectively, and are then converted into reference matrix 330, tumor sample matrix 331-A, and normal sample matrix 331-B, respectively.

Referring now back to method 600, at step 680, the three matrices are fed (provided as inputs) into a trained neural network, and at step 690, an output of the trained neural network is obtained, where the output includes at least a prediction (a probabilistic estimate) of the somatic variant type contained in the tumor sequence read (e.g., at the candidate somatic variant position or its vicinity).

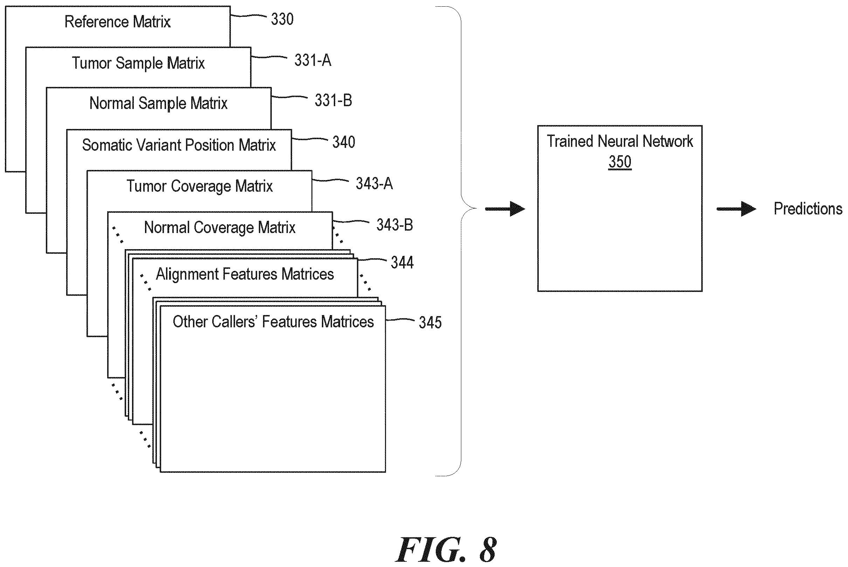

FIG. 8 shows an exemplary diagram of trained neural network 350 being used for somatic variant calling. In this example, network 350 receives as its input reference matrix 330, tumor sample matrix 331-A, and normal sample matrix 331-B.

It is appreciated that while FIG. 8 shows all three types of matrices being inputted into network 350, in some embodiments, reference matrix 330 and/or normal sample matrix 331-B can be omitted and not be provided to network 350, and network 350 could still perform somatic variant calling. In embodiments where normal sample matrix is omitted, obtaining normal sequence reads can be omitted from step 620 of method 600, and step 660 of converting the normal sequence reads into a matrix can be omitted altogether.

In some embodiments, in order to further improve the accuracy of prediction and/or to expand the amount of information that can be obtained at the network's output, the network can also receive as inputs additional matrices such as somatic variant position matrix 340 representing the position of the candidate somatic variant; tumor coverage matrix 343-A and normal coverage matrix 343-B representing the respective coverages of the tumor sequence reads and normal sequence reads segments; and one or more alignment features matrices 344, the matrices representing various features of the sequence reads in a manner similar to that described above in connection with FIG. 4. In addition, while not illustrated in FIG. 8 for brevity, in some embodiments, data related to known variants can be obtained from public and/or private knowledge bases such as dbSNP, COSMIC, ExAC, etc., and provided as input(s) to network 350.

In some embodiments, to further improve the accuracy of the somatic variant calling, neural network 350 can also be provided one or more other callers' features matrices 345 at its input. These matrices can represent (e.g., summarize) one or more features obtained from one or more other (e.g., third-party) variant calling applications or algorithms that have already processed the same segment of sequence reads. Such features can include variant types, lengths, and positions predicted by other applications/algorithms, as well as quality scores, mapping scores, variant significance scores, and other alignment features. As discussed above in connection with FIG. 4, in some embodiments, all input matrices being fed into network 350 can be "combined" and provided as one large three-dimensional matrix.

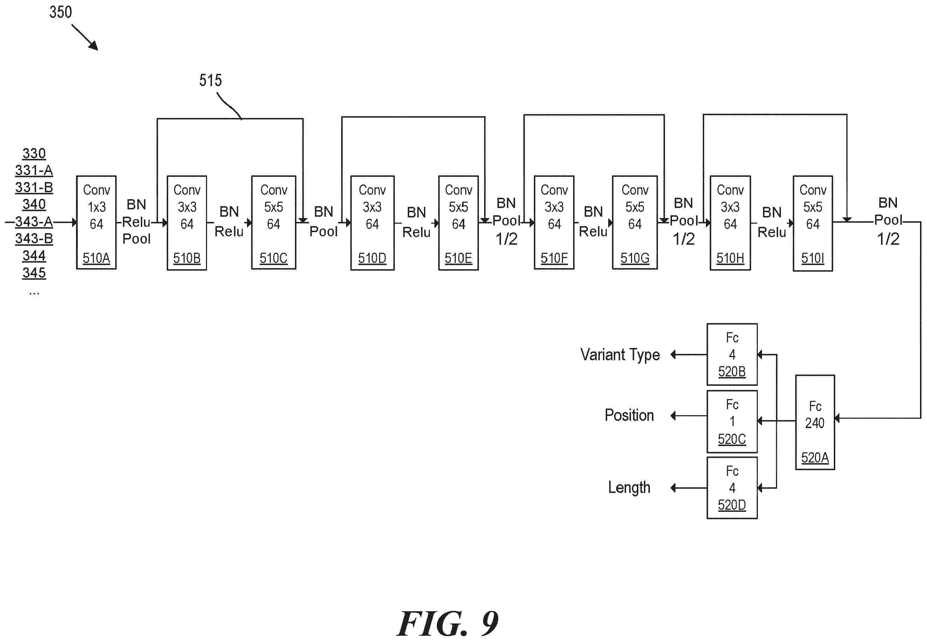

FIG. 9 shows an exemplary trained neural network 350 receiving the various inputs described above and outputting predictions indicating at least the variant type, position, and length of a somatic variant contained within a segment of somatic sequence reads represented by somatic sample matrix 331-A at its input. In some embodiments, network 350 can also include fully connected layer 520E (shown in FIG. 5) that outputs the variant's genotype, but in case of a somatic variant calling the genotype is not important and can therefore be omitted from the network.

Based on the examples of FIG. 9 and FIG. 5 it is appreciated that in some embodiments, network 350 having the same or substantially the same architecture can be used to predict both germline and somatic variants, depending on the type of data the network has been trained on (as discussed below) and depending on the type of inputs provided to it. In some embodiments, however, the architecture of network 350 can be modified and optimized for either germline or somatic variant calling.

### Neural Network Training

It is appreciated by a person skilled in the art that before neural network 350 could start performing accurate germline or somatic variant calling, it first needs to be trained on germline or somatic training data (i.e., training sequences), respectively. The training can include, for example, performing all of the steps of methods 200 or 600 on a large number training sequences, but then also providing to the network "ground truth" data (e.g., actual known variant types and their positions, lengths, genotypes, etc.) to enable the network to gradually minimize its output errors by adjusting its trainable filters and other trainable parameters after every run through the process known as "back propagation."

In some embodiments, network 350 can be trained on genomes having well characterized ground truth variants, such as the NA12878 genome. In some embodiments, instead or in addition to real genomes, various simulation based strategies can be used to train the network. For example, to train the network for germline variant calling, synthetic samples with a given set of variants can be simulated using the method described in Mu, J. C. et al. ("VarSim: a high-fidelity simulation and validation framework for high-throughput genome sequencing with cancer applications." Bioinformatics 31, 1469-1471 (2015)). As another example, to train the network for somatic variant calling, it can be fed normal samples in which random variants have been spiked, e.g., using the method described in Eving, A. et al. ("Combining tumor genome simulation with crowdsourcing to benchmark somatic single-nucleotide-variant detection," Nature methods (2015)). Alternatively or in addition, two different normal germline samples with different proportions can be mixed to generate synthetic tumor/normal training samples. As another example, virtual tumor/normal training samples with desired allele frequency distributions can be generated by switching reads between two germline samples at variant locations.

### Example 1

### Introduction

Somatic mutations are critical signatures in cancer genesis, progression, and treatment. Accurate detection of somatic mutations is challenging due to tumor-normal cross contamination, tumor heterogeneity, sequencing artifacts, and coverage. In general, effectively filtering false-positive calls, which are introduced by the aforementioned issues, and precisely keeping hard-to-catch true-positive calls, which may occur with low allele-frequency (AF) or occur in low-complexity regions, are crucial for an accurate somatic mutation detection algorithm.

To date, a range of tools have been developed to address the somatic mutation detection problem including MuTect2¹, MuSE², VarDict³, VarScan2⁴, Strelka2⁵, and SomaticSniper⁶. These tools employ different statistical and algorithmic approaches, which perform well in certain cancer or sample types for which they were designed; however, they are limited in generalization to a broader range of sample types and sequencing technologies and thus may exhibit suboptimal accuracy in such scenarios^{7,8,9}. In our earlier work, SomaticSeq¹⁰, we used an ensemble approach to maximize the sensitivity by integrating algorithmically orthogonal methods. It also used machine learning to integrate almost a hundred features to keep the precision high, leading to an accuracy improvement over all individual methods. Nevertheless, the machine-learning backbone used in SomaticSeq relies on a set of extracted features for the mutations' locations. As a result, it cannot fully capture the raw information in the genomic contexts of the somatic mutations to further distinguish true somatic mutations from background errors, limiting its performance in challenging situations, such as low-complexity regions and low tumor purity.

Here we address the limitation of generalizability and complexity of statistical modeling of tumor sequencing data by leveraging deep Convolutional Neural Networks (CNNs). CNNs have recently shown significant performance in classification problems from different domains including germline variant calling^{11,12,13} and skin cancer classification¹⁴. Even so, applying CNNs to the challenging problem of somatic mutation detection has not been explored. The only previous deep learning based attempt¹⁵ was to apply a six-layer fully connected neural network to a set of manually extracted features. This approach lacks the power provided by the CNN architecture, which is to learn feature representations directly from the raw data using patterns seen in local regions. Besides, due to the complexity of fully connected networks, it has less generalizability and scalability as seen in CNNs.

We introduce NeuSomatic, the first CNN-based approach for somatic mutation detection that can effectively leverage signals derived from sequence alignment, as well as from other methods to accurately identify somatic mutations. Unlike other deep learning based methods that are focused on germline variants, NeuSomatic is addressing a bigger unmet need in terms of accuracy due to the complexity of tumor samples. It can effectively capture important mutation signals directly from the raw data and consistently achieve high accuracy for different sequencing technologies, sample purities, and sequencing strategies such as whole-genome vs. target enrichment.

### Results

### NeuSomatic overview

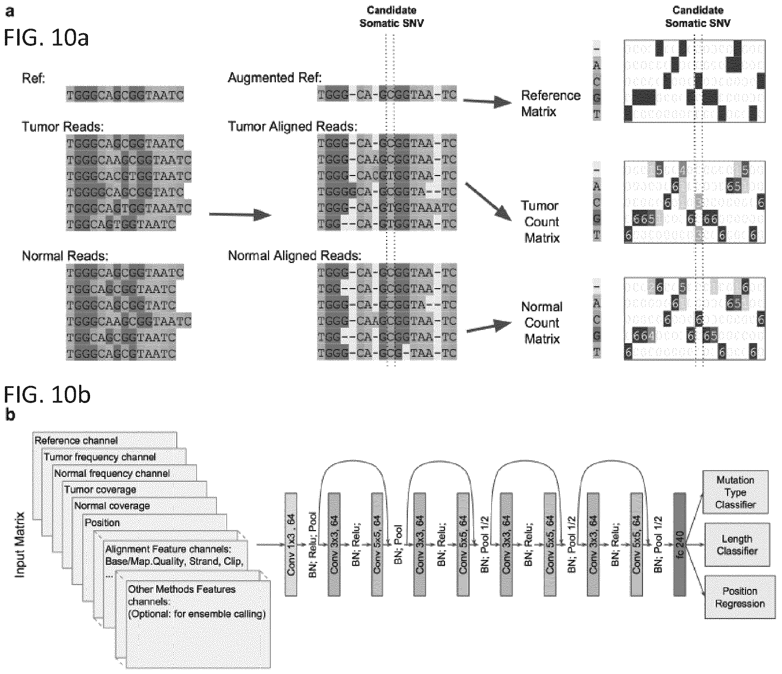

The inputs to NeuSomatic's network are candidate somatic mutations identified by scanning the sequence alignments for the tumor sample as well as the matched normal sample (FIGS. 10a-lOd). Somatic mutations reported by other methods can also be included in this list of candidates. For each candidate locus, we construct a 3-dimensional feature matrix M (of size k × 5 × 32), consisting of k channels each of size 5 × 32, to capture signals from the region centered around that locus. Each channel of the matrix M has five rows representing the four nucleotide bases as well as the gapped base ('-'), and 32 columns representing the alignment columns around the candidate location.

FIG. 10a illustrates a toy example of input matrix preparation for a given candidate somatic SNV. Sequence alignment information in a window of seven bases around the candidate somatic mutation is extracted. The reference sequence is then augmented by adding gaps to account for insertions in the reads. The augmented alignment is then summarized into the reference matrix, the tumor count matrix, and the normal count matrix. The count matrices record the number of A/C/G/T and gap (`-`) characters in each column of the alignment, while the reference matrix records the reference bases in each column. The count matrices are then normalized by coverage to reflect base frequencies in each column. Separate channels are reserved to record the tumor and normal coverages.

FIG. 10b illustrates the input 3-dimensional matrix and the proposed NeuSomatic network architecture. The input matrix consists of reference channel, tumor and normal-frequency channels, coverage and position channels, followed by several channels summarizing the alignment features. When used in ensemble mode, NeuSomatic also includes additional channels for other individual methods features. NeuSomatic network architecture consists of nine convolutional layers structured in four blocks with shortcut identity connections. We use two softmax classifiers and one regressor on the final layer to predict the mutation type, size, and position.

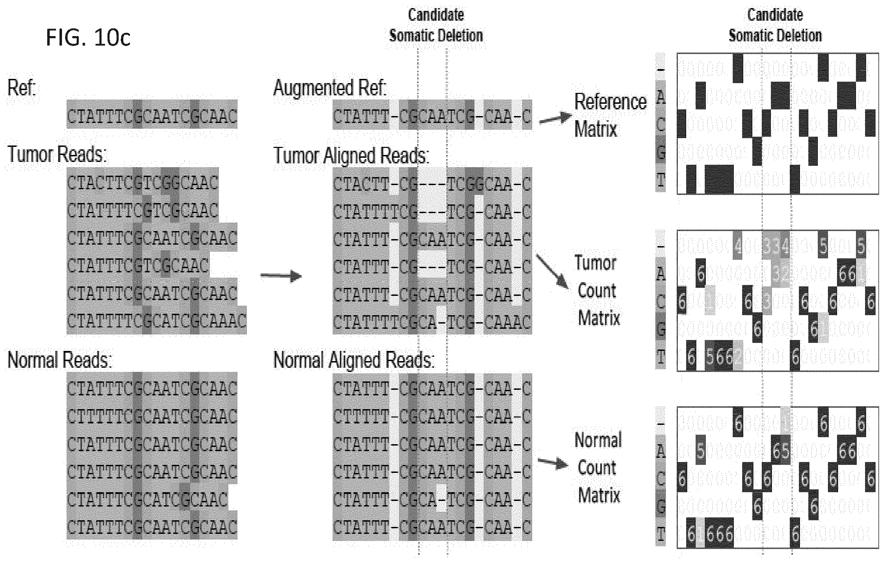

FIG. 10c illustrates a toy example of input matrix preparation for a given candidate somatic deletion. Sequence alignment information in a window of 7 bases around the candidate somatic mutation is extracted. The reference sequence is then augmented by adding gaps to account for insertions in the reads. The augmented alignment is then summarized into the reference matrix, the tumor count matrix, and the normal count matrix. The count matrices record the number of A/C/G/T and gap ('-') characters in each column of the alignment, while the reference matrix records the reference bases in each column. The count matrices are then normalized by coverage to reflect base frequencies in each column. Separate channels are reserved to record the tumor and normal coverages.

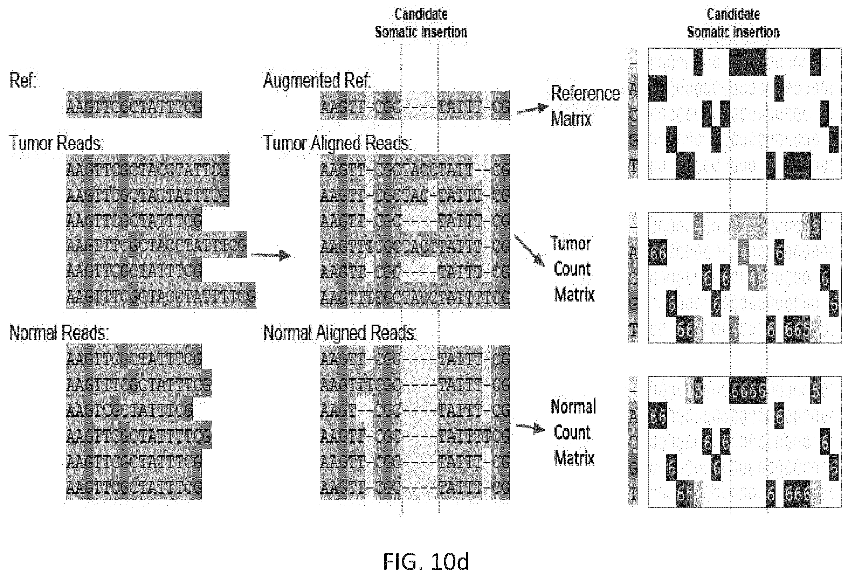

FIG. 10d illustrates a toy example of input matrix preparation for a given candidate somatic insertion. Sequence alignment information in a window of 7 bases around the candidate somatic mutation is extracted. The reference sequence is then augmented by adding gaps to account for insertions in the reads. The augmented alignment is then summarized into the reference matrix, the tumor count matrix, and the normal count matrix. The count matrices record the number of A/C/G/T and gap ('-') characters in each column of the alignment, while the reference matrix records the reference bases in each column. The count matrices are then normalized by coverage to reect base frequencies in each column. Separate channels are reserved to record the tumor and normal coverages.

The first three channels, respectively, are the reference, tumor-frequency, and normal-frequency channels that summarize the reference bases around the candidate locus, as well as the frequency of different bases in that region. We augment the reference sequence around the candidate locus with gaps corresponding to the insertions in the read alignments (FIGS. 10a, 10c, and 10d) in order to capture the insertions. Thus, each column of tumor and normal-frequency matrices represents the frequency of A/C/G/T/gap bases in the corresponding multiple sequence alignment (MSA) column of the tumor and normal samples, respectively. The remaining channels summarize other features, such as coverage, base quality, mapping quality, strand-bias, and clipping information for reads supporting different bases. If NeuSomatic is used in the ensemble mode, we also use additional channels for features reported by the individual somatic mutation detection methods. With this concise, yet comprehensive structured representation, NeuSomatic can use the necessary information in tumor, normal, and reference to differentiate difficult to catch somatic mutations with low AF from germline variants, as well as sequencing errors. This design also enables the use of convolutional filters in the CNN to capture contextual patterns in the sub-blocks of the matrix.

To compare to other CNN approaches used in genomics problems, Deep Variant" uses read pileup as the input for germline variant calling. In contrast, we use base frequency summary for each column as the input to our network. This simplifies the CNN structure, allowing a substantially more efficient implementation. For example, Deep Variant takes ~1000 CPU core-hours to call germline variants for a 30× whole-genome sample¹⁶, whereas the stand-alone version of NeuSomatic can detect somatic mutations from 30× tumor-normal pair samples in ~156 CPU core-hours, despite handling two (tumor-normal) samples instead of one and looking for candidates at lower somatic AFs than germline 50 or 100% AF. Another germline variant calling method, Clairvoyante¹², uses three channels to summarize base counts for allele counts, deletions, and insertions at the center of the window. In contrast, we summarize all these events in a single base frequency matrix using the reference augmentation approach described earlier, which can clearly represent all the insertion and deletion (INDEL) events across the window.

NeuSomatic employs a novel CNN structure that predicts the type and length of a candidate somatic mutation given the feature matrix M (FIG. 10b). The proposed CNN consists of nine convolutional layers structured in four blocks with shortcut identity connections inspired by ResNet¹⁷ but with a different formation to adapt to the proposed input structure. We use two softmax classifiers and one regressor on the final layer. The first classifier identifies whether the candidate is a non-somatic call, SNV, insertion, or deletion. The second classifier predicts the length of the somatic mutation with four classes (0 indicating non-somatic, or lengths from 1, 2, or greater than 2), and the regressor predicts the location of the somatic mutation. Using the output of these classifiers we identify the set of somatic mutations. If the lengths of INDELs are predicted to be larger than 2, we perform a simple post-processing step on reads overlapping that position to resolve the INDEL sequence from the read alignment CIGAR string. This has been shown to perform well for data generated by Illumina sequencers. For higher error rate sequencing data, more complex local realignment post-processing is conducted to resolve the INDEL sequence.

Since NeuSomatic can be used in stand-alone and ensemble modes, we use NeuSomatic-S to denote the stand-alone mode, while reserving NeuSomatic to denote the ensemble mode. We compared NeuSomatic and NeuSomatic-S against the state-of-the-art somatic mutation detection methods including MuTect2¹, MuSE², SomaticSniper⁶, Strelka2⁵, VarDict³, and VarScan2⁴, and against the ensemble approach, SomaticSeq¹⁰. We compared and contrasted performance using multiple synthetic and real datasets. We report below, the synthetic datasets in increasing order of somatic mutation detection difficulty considering the AF of somatic mutation in the datasets.

### Comparison on the Platinum sample mixture dataset

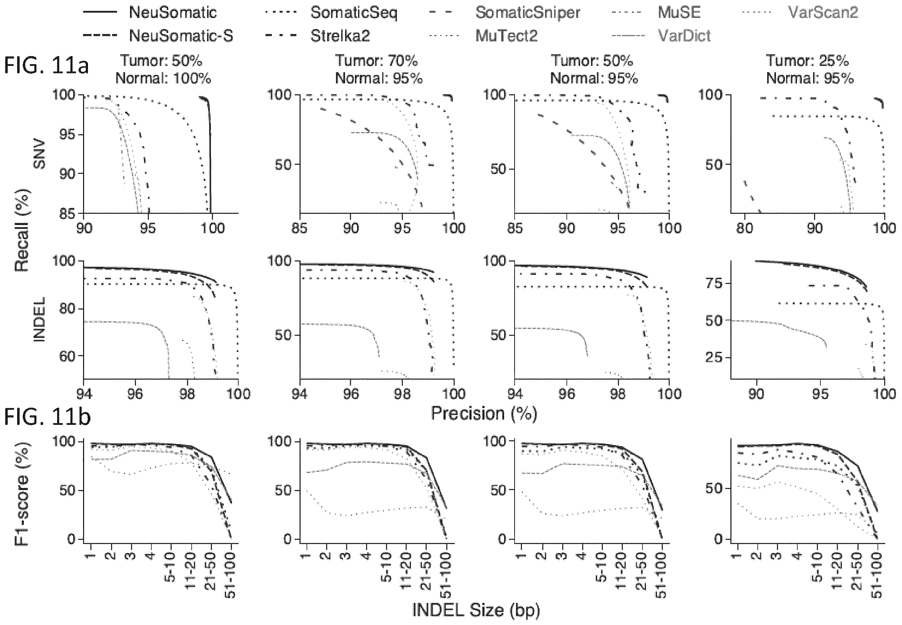

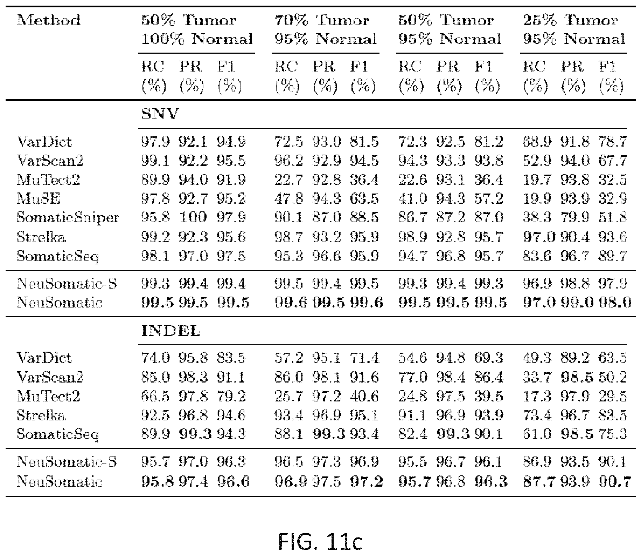

For the first synthetic dataset, as in previous studies^{5,10} we mixed two normal Platinum Genomes¹⁸ samples, NA12877 and NA12878, at 70:30, 50:50, and 25:75 tumor purity ratios to create three tumor contamination profiles, and at 5:95 ratio to create a contaminated normal sample. We also included a test with 100% pure normal and 50% pure tumor. We used the germline variants in NA12878, which were reference calls in NA12877 as truth set for the evaluation. Both NeuSomatic-S and NeuSomatic significantly outperformed all other methods (FIGS. 11a and 11b and 11c). NeuSomatic's performance improvement over other approaches increased with lower, more challenging tumor purities (25:75 mixture). In summary, NeuSomatic yielded up to 99.6 and 97.2% F1-scores for SNVs and INDELs, respectively, overall and an improvement of up to 7.2% over the best method in the lowest sample purity for this dataset. For the sample with 50% tumor purity, reducing normal purity from 100 to 95% had minor impact on NeuSomatic's performance (<0.3%), whereas it caused ~3% decrease in SomaticSeq accuracy.

FIGS. 11a and 11b are charts for the performance analysis of the Platinum two sample mixture dataset. In this dataset, four tumor and normal purity scenarios (50% T:100% N, 70% T:95% N, 50% T:95% N, and 25% T:95% N) are used. FIG. 11a are graphs for a precision-recall analysis: the confidence or quality scores are used to derive the precision-recall curves. The highest F1-score achieved by each algorithm is printed on the curve and marked with a solid circle. FIG. 11b are graphs for a performance analysis of INDEL accuracy (F1-score) for different INDEL sizes. FIG. 11c is a table that shows the performance of different somatic mutation detection methods on Platinum two sample mix dataset. For each method we report the precision, recall and F1-score for the quality score threshold in precision-recall curve which achieves highest F1. (RC: Recall, PR: Precision, F1: F1-score).

### Comparison on the ICGC-TCGA DREAM challenge datasets

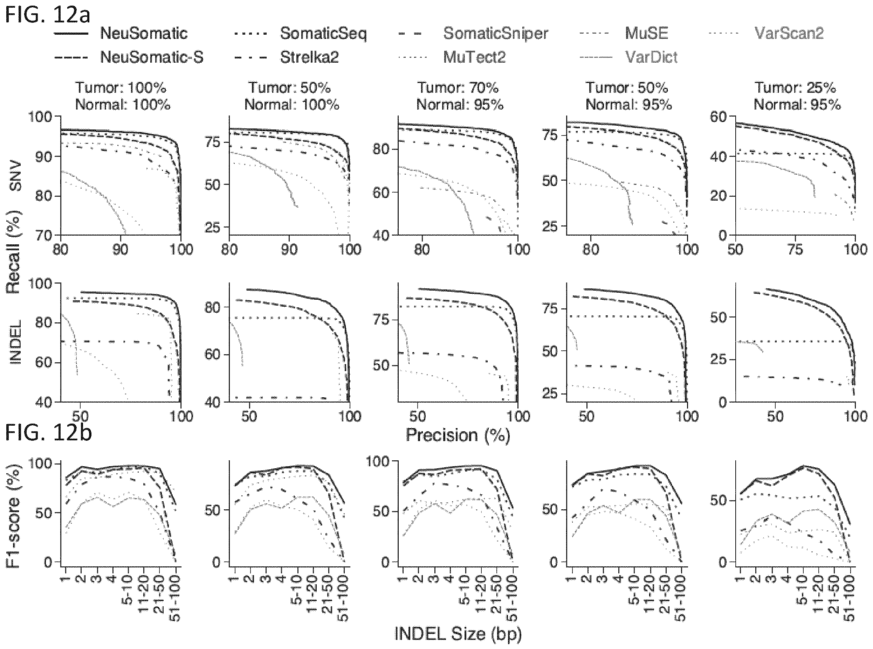

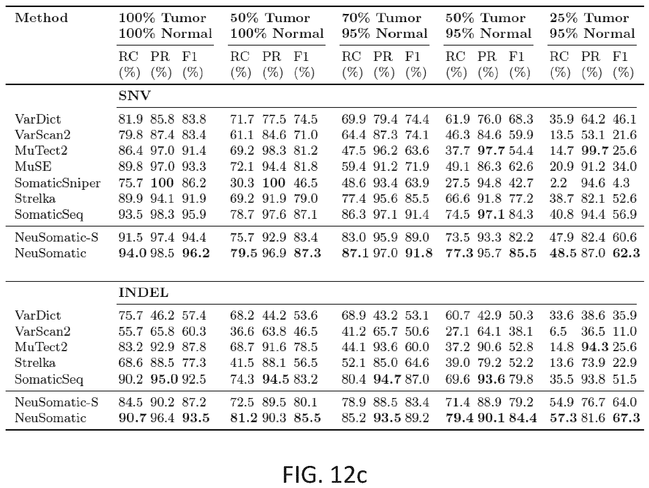

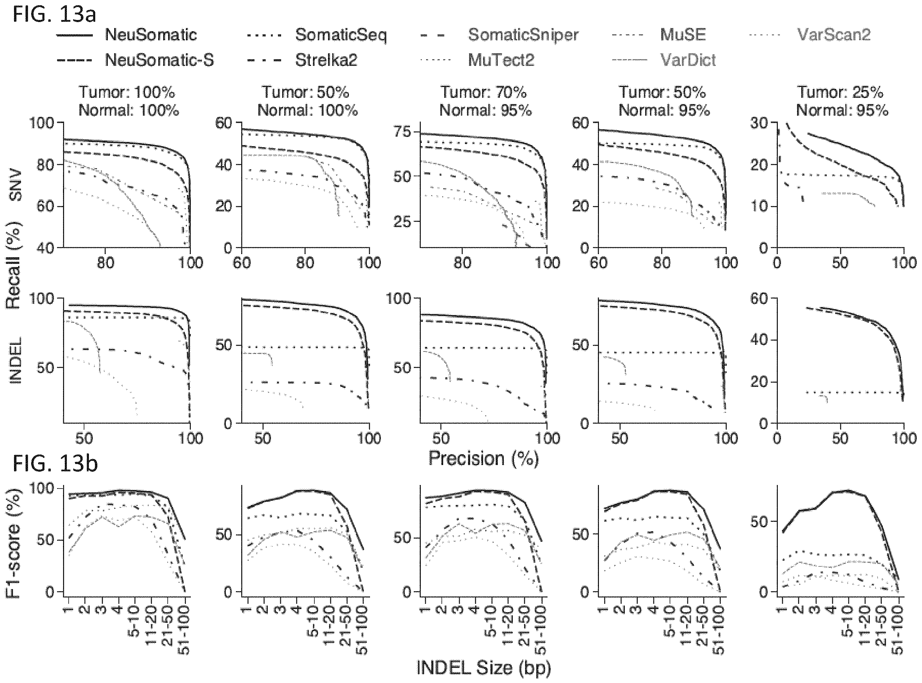

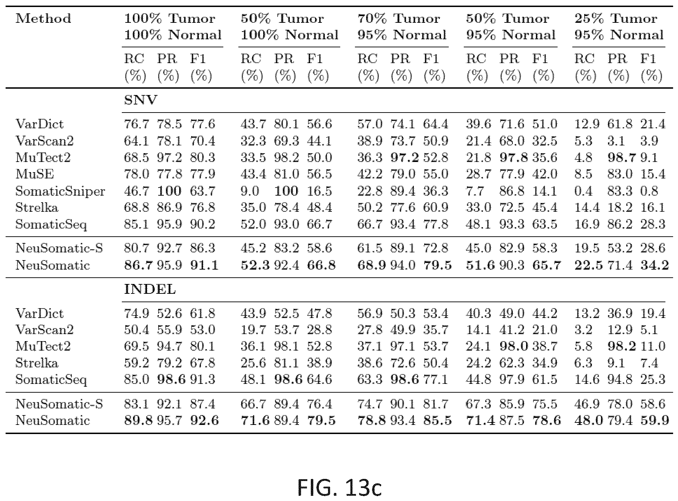

For the second synthetic dataset, we used the ICGC-TCGA DREAM Challenge Stage 3 and Stage 4 datasets¹⁹, which were constructed by computationally spiking mutations into a healthy genome of a paired normal sample with different AFs (See Methods). We mixed the tumor and normal samples to create five tumor/normal purity senarios. NeuSomatic-S outperformed all other stand-alone methods for both Stage 3 and Stage 4 datasets by over 8% for SNVs and 22% for INDELs on average (FIGS. 12a, 12b, 12c, 13a, 13b; 13c). This performance improvement increased with decreasing tumor purity. We further observed that NeuSomatic (the ensemble mode) clearly outperformed both SomaticSeq and NeuSomatic-S, even though NeuSomatic-S still outperformed SomaticSeq in more challenging scenarios, such as SNVs in the 25:75 mixture and INDELs in the 25:75 and 50:50 mixtures. In summary, NeuSomatic yielded up to 96.2 and 93.5% F1-scores for SNVs and INDELs, respectively, overall and an improvement of up to 34.6% over the best method in the lowest sample purity. For the sample with 50% tumor purity, reducing normal purity from 100 to 95% had minor impact on NeuSomatic's performance (~1.2% on average), whereas SomaticSeq and Strelka2 had >3% decrease in F1-score.

FIGS. 12a and 12b are graphs for performance analysis of the DREAM Stage 3 dataset. In this dataset, five tumor and normal purity scenarios (100% T:100% N, 50% T:100% N, 70% T:95% N, 50% T:95% N, and 25% T:95% N) are used. FIG. 12a are graphs for precision-recall analysis: the confidence or quality scores are used to derive the precision-recall curves. The highest F1-score achieved by each algorithm is printed on the curve and marked with a solid circle. Fig. 12b are graphs for performance analysis of INDEL accuracy (F1-score) for different INDEL sizes. FIG. 12c is a table that shows the performance of different somatic mutation detection methods on Dream Challenge Stage 3 dataset. For each method we report the precision, recall and F1-score for the quality score threshold in precision-recall curve which achieves highest F1. (RC: Recall, PR: Precision, F1: F1-score).

FIGS. 13a and 13b are graphs for performance analysis of the DREAM Stage 4 dataset. In this dataset, five tumor and normal purity scenarios (100% T:100% N, 50% T:100% N, 70% T:95% N, 50% T:95% N, and 25% T:95% N) are used. FIG. 13a are graphs for precision-recall analysis: the confidence or quality scores are used to derive the precision-recall curves. The highest F1-score achieved by each algorithm is printed on the curve and marked with a solid circle. FIG. 13b are graphs for performance analysis of INDEL accuracy (F1-score) for different INDEL sizes. FIG. 13c is a table that shows the performance of different somatic mutation detection methods on Dream Challenge Stage 4 dataset. For each method we report the precision, recall and F1 score for the quality score threshold in precision-recall curve which achieves highest F1. (RC: Recall, PR: Precision, F1: F1-score).

### Comparison on the Platinum tumor spike dataset

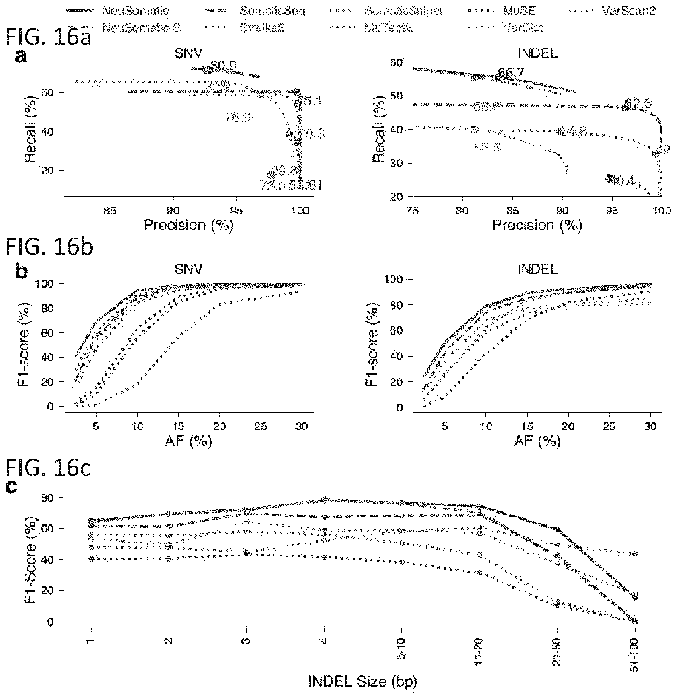

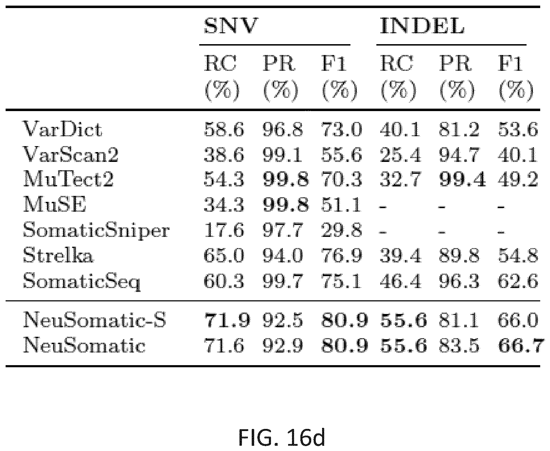

For the third synthetic dataset, as in previous studies^{1,10}, we constructed a tumor sample by spiking reads from NA12878 into NA12877 in variant positions ofNA12878 with spike in frequencies sampled from a binomial distribution with means [0.05, 0.1, 0.2, 0.3] and used an independent set of NA12877 reads as pure normal. Note that, unlike earlier strategy, which mixed samples in fixed proportions yielding somatic mutations at fixed AFs, this mixing approach generated them at varying AFs ranging from 0.025 to 0.3. NeuSomatic yielded 80.9 and 66.7% F1-scores for SNVs and INDELs, respectively, overall and an improvement of up to 4% over the best method (FIGS. 16a-16d). For low AF somatic mutations, the performance improvement was even higher (11% improvement for AF = 0.025 and 8% improvement for AF = 0.05) (Fig. 16b). FIG. 16a shows a precision-recall analysis where the confidence or quality scores are used to derive the precision-recall curves. The highest F1-score achieved by each algorithm is printed on the curve and marked with a solid circle. FIG. 16b shows a performance analysis for different AFs. FIG. 16c shows a performance analysis of INDEL accuracy (F1-score) for different INDEL sizes. FIG. 16d shows for each method the precision, recall and F1-score for the quality score threshold in precision-recall curve which achieves highest F1. (RC: Recall, PR: Precision, F1: F1-score).

### Comparison on the whole-exome and targeted panels

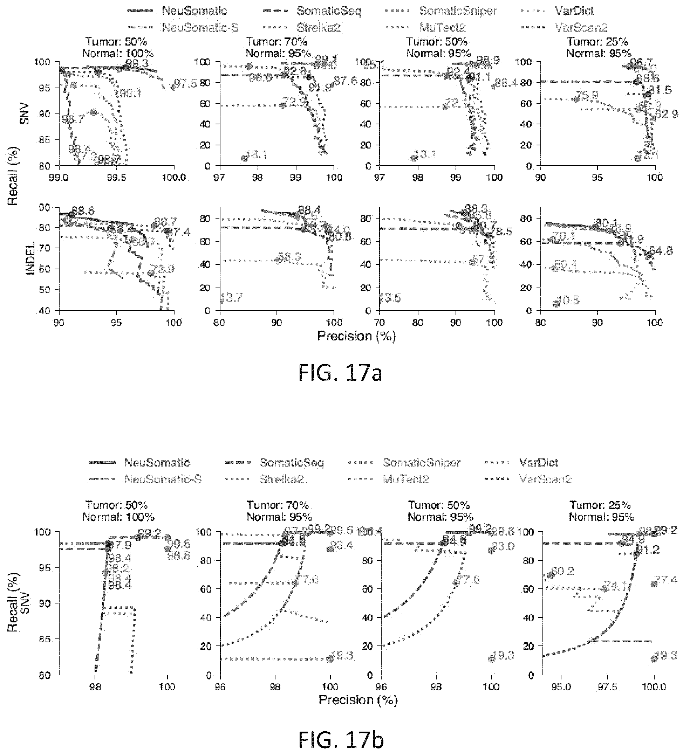

To assess the performance of NeuSomatic on different target enrichments, we used a whole-exome and a targeted panel dataset from the Ashkenazi Jewish trio²⁰ (FIGS. 17a-17d). We trained NeuSomatic and SomaticSeq on the whole-exome dataset and applied the trained model on both the whole-exome and the panel. For whole-exome, NeuSomatic achieved up to 99.3 and 88.6% F1-scores for SNVs and INDELs, respectively. For the targeted panel, NeuSomatic and NeuSomatic-S consistently outperformed other methods with >99.2% F1-score for SNVs.

FIG. 17a illustrates the performance analysis of various methods on the exome sample mixture. In this dataset, four tumor and normal purity scenarios (50%T: 100%N, 70%T:95%N, 50%T:95%N and 25%T:95%N) are used. The confidence or quality scores are used to derive the precision-recall curves. The highest F1-score achieved by each algorithm is printed on the curve and marked with a solid circle. Here the training is on exome data for NeuSomatic, NeuSomatic-S, and SomaticSeq.

FIG. 17b illustrates the performance analysis of various methods on the Target panel sample mixture. In this dataset, four tumor and normal purity scenarios (50%T: 100%N, 70%T:95%N, 50%T:95%N and 25%T:95%N) are used. The confidence or quality scores are used to derive the precision-recall curves. The highest F1-score achieved by each algorithm is printed on the curve and marked with a solid circle. Here the training is on exome data for NeuSomatic, NeuSomatic-S, and SomaticSeq.

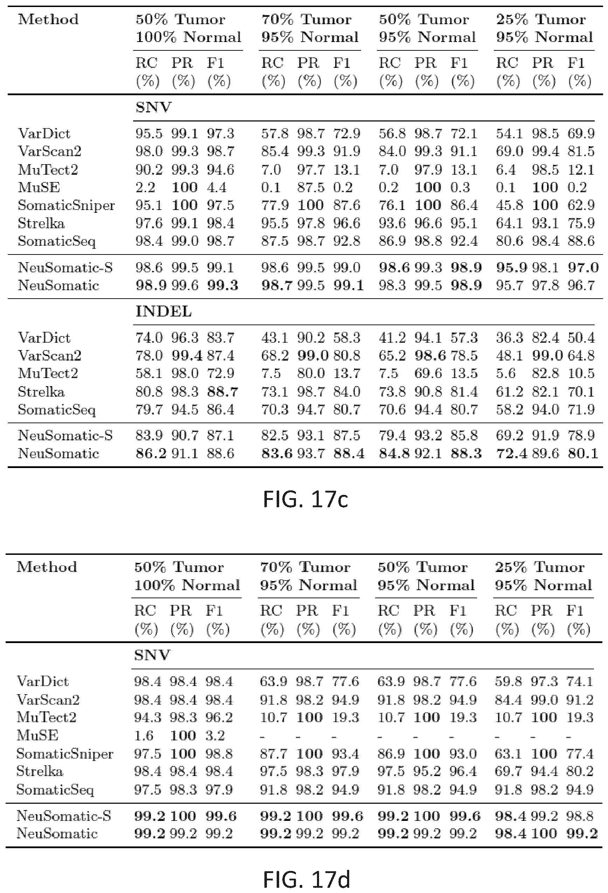

FIG. 17c illustrates the performance of different somatic mutation detection methods on whole-exome sample mix dataset. For each method we report the precision, recall and F1-score for the quality score threshold in precision-recall curve which achieves highest F1. (RC: Recall, PR: Precision, F1: F1-score).

FIG. 17d illustrates the performance of different somatic mutation detection methods on targeted panel dataset. For each method we report the precision, recall and F1-score for the quality score threshold in precision-recall curve which achieves highest F1. (RC: Recall, PR: Precision, F1: F1-score).

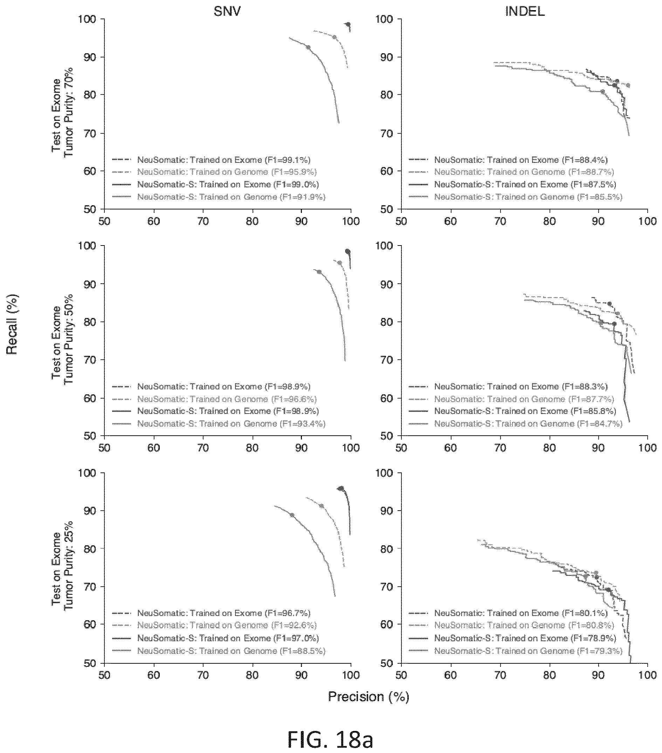

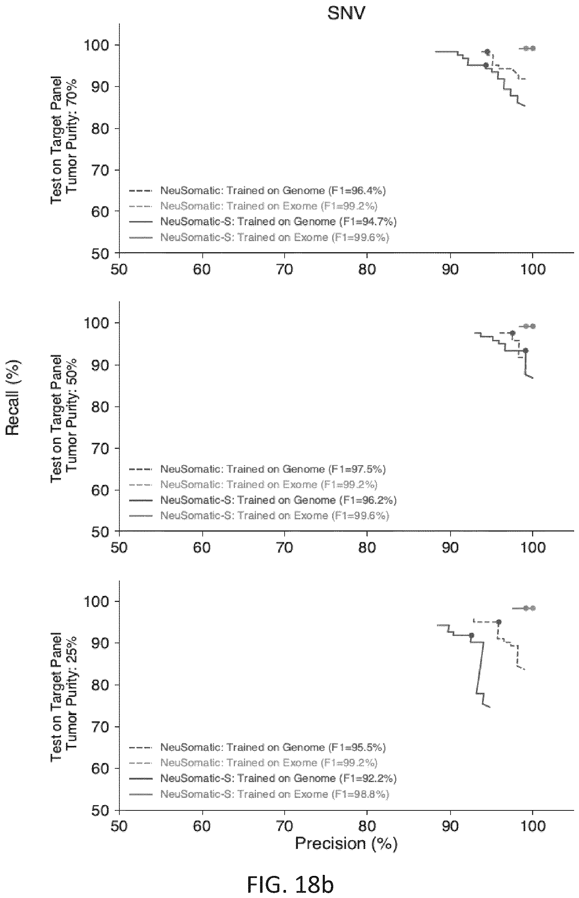

Applying the model trained on whole-genome Platinum-mixture data on both target enrichment sets yielded similar performance, which confirmed the robustness of NeuSomatic (FIGS. 18a and 18b). Similar to other datasets, for the sample with 50% tumor purity, reducing normal purity from 100 to 95% on whole-exome dataset could minimally reduce NeuSomatic's F1-score (~0.3% on average), whereas SomaticSeq and Strelka2 had >5% decrease in F1-score.

FIG. 18a illustrates the performance analysis of using models trained on whole-genome (Platinum data, genome mixture) and whole-exome (HG003-HG004 exome mixture) to test on exome mixture dataset. The confidence or quality scores are used to derive the precision-recall curves. The highest F1-score achieved by each algorithm is indicated in the legend and marked with a solid circle on the curve.

FIG. 18b illustrates the performance analysis of using models trained on whole-genome (Platinum data, genome mixture) and whole-exome (HG003-HG004 exome mixture) to test on target panel mixture dataset. The confidence or quality scores are used to derive the precision-recall curves. The highest F1-score achieved by each algorithm is indicated in the legend and marked with a solid circle on the curve.

### Comparison on the PacBio dataset

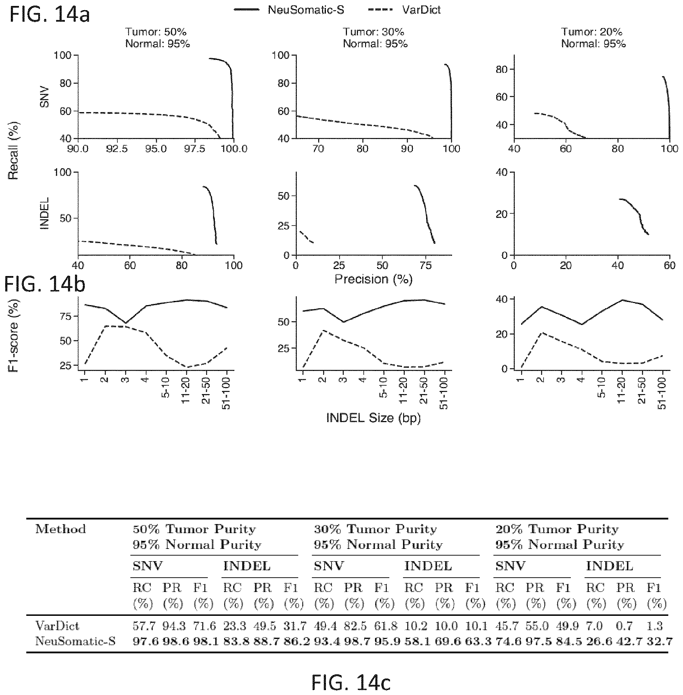

We further evaluated NeuSomatic's performance on reads with high error rates, particularly those from the long-read sequencing platforms. We used tumor-normal pair samples simulated with 20, 30, and 50% AF somatic mutations based on the raw PacBio reads (FIGS. 14a, 14b, and 14c). NeuSomatic identified somatic SNVs and INDELs with F1-scores of up to 98.1 and 86.2%, respectively, which outperformed VarDict³ by up to 34.4% for SNVs and up to 53.2% for INDELs. This analysis confirms the capability of NeuSomatic in detecting somatic mutations even when the sequence reads have high error rate as in PacBio long raw reads.

### Comparison for different INDEL sizes

It is worth noting that NeuSomatic consistently outperformed other methods for various INDEL sizes in different datasets (FIGS. 11b, 12b, 13b, 14b, 16c, and 19). For large (>50 bases) INDELs, since most of the short reads with somatic INDELs are soft-clipped, the INDEL information is lost in the pileup count matrices. For such cases, NeuSomatic benefited from other methods' predictions, since some of the methods like VarDict and MuTect2 used local assembly for their predictions.

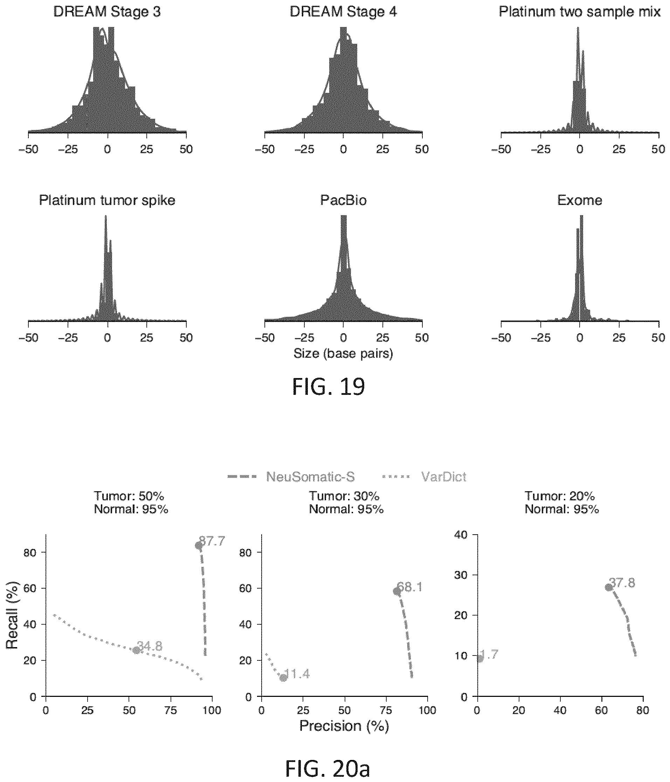

FIGS. 14a and 14b are graphs for performance analysis of different somatic mutation dectection methods on the PacBio dataset. In this dataset, three tumor and normal purity scenarios (70% T:95% N, 50% T:95% N, and 25% T:95% N) are used. FIG. 14a are graphs for precision-recall analysis: the confidence or quality scores are used to derive the precision-recall curves. The highest F1-score achieved by each algorithm is printed on the curve and marked with a solid circle. FIG. 14b are graphs for performance analysis of INDEL accuracy (F1-score) for different INDEL sizes. FIG. 14c shows for each method the precision, recall and F1-score for the quality score threshold in precision-recall curve which achieves highest F1. (RC: Recall, PR: Precision, F1: F1-score). FIG. 19 illustrates the size distribution of ground truth INDELs in Dream Stage 3, Dream Stage 4, Platinum two sample mixture, Platinum tumor spike, PacBio, and exome datasets. Negative sizes correspond to deletions.

### INDEL type and position accuracy

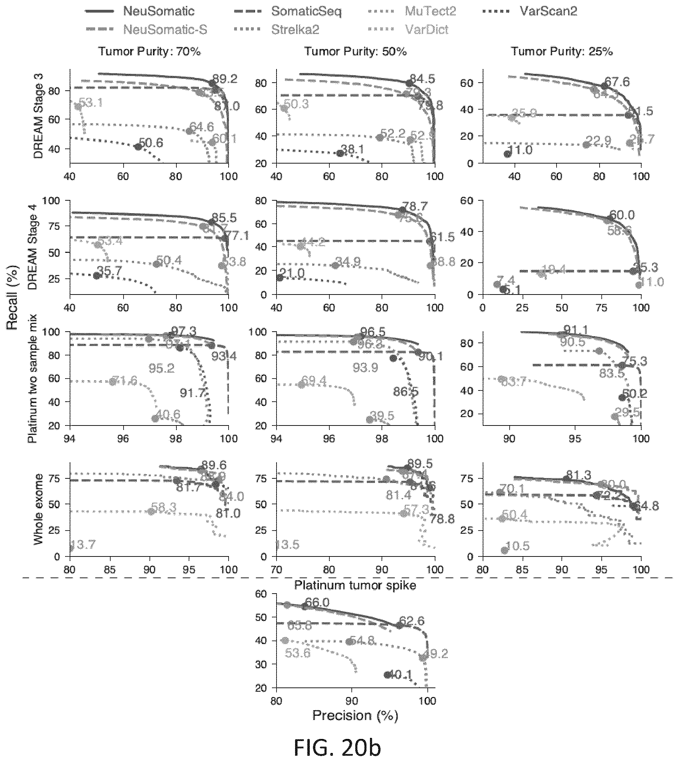

For all datasets discussed, we also assessed the performance of INDEL calling by different somatic mutation detection methods using the more relaxed criterion of simply predicting the positions of the somatic INDELs correctly (and ignoring the exact INDEL sequence). Again, we observed similar superiority of NeuSomatic over other schemes indicating that the main improvements are contributed by the proposed CNN structure and not the post-processing INDEL resolution steps (FIGS. 20a and 20b).

FIG. 20a illustrates the performance analysis of INDELs based on position and type of the predicted somatic mutations (while ignoring the accuracy of the exact predicted INDEL sequence) for PacBio dataset on three tumor purity scenarios (50%, 30% and 20%) and 95% normal purity. The confidence or quality scores are used to derive the precision-recall curves. The highest F1- score achieved by each algorithm is printed on the curve and marked with a solid circle.

FIG. 20b illustrates the performance analysis of INDELs based on position and type of the predicted somatic mutations (while ignoring the accuracy of the exact predicted INDEL sequence) for Dream Stage 3, Dream Stage 4, Platinum two sample mixture, whole-exome, and Platinum tumor spike datasets. For the first four datasets, three tumor purity scenarios (70%, 50% and 25%) are used while normal sample has 95% purity. The confidence or quality scores are used to derive the precision-recall curves. The highest F1- score achieved by each algorithm is printed on the curve and marked with a solid circle.

### Read coverage analysis

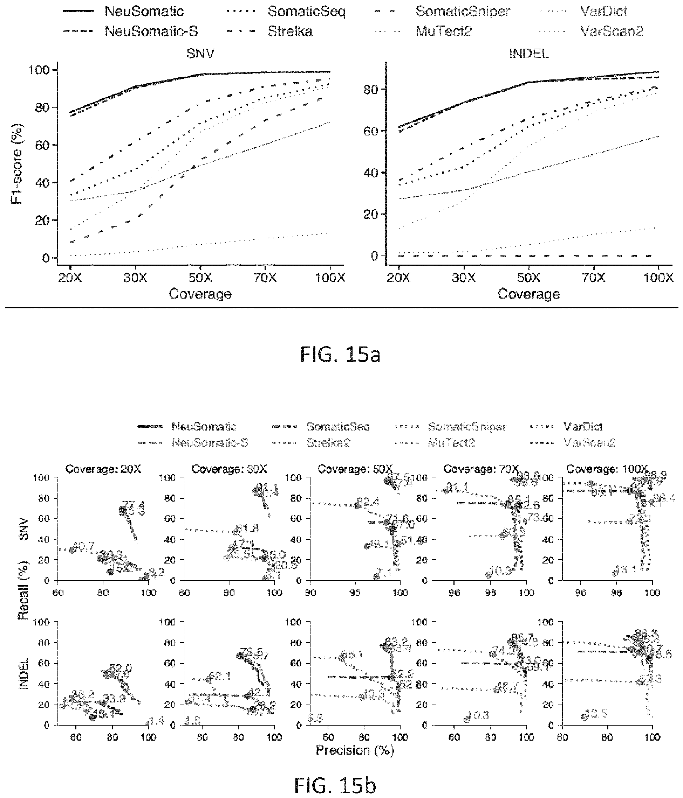

To evaluate the impact of sequence coverage on different techniques, we downsampled the whole-exome dataset to obtain samples with sequence coverages in the range of 20× and 100× (FIGS. 15a and 15b). NeuSomatic consistently outperformed other techniques for different coverages. The improvement increased as the problem became more challenging for lower coverages samples. In addition, reducing the coverage from 100× to 50× had very minimal impact (∼1.5% for SNVs and -5% for INDELS) on NeuSomatic, whereas SomaticSeq's F1-score dropped by ~20% for both SNVs and INDELs, and Strelka2's F1-score dropped by ~13% for SNVs and ~15% for INDELs. This analysis revealed both the robustness of NeuSomatic to coverage perturbations, as well as its advantage in challenging scenarios, which could be seen at lower coverages.

FIG. 15a are graphs for performance analysis of the sequence coverage impact on the whole-exome sample mixture dataset. In this example, tumor has 50% purity and normal has 95% purity. Y-axis illustrates the highest F1-score achieved by each algorithm for sample alignments coverages ranging from 20× to 100×.

FIG. 15b are graphs for performance analysis of the sequence coverage impact on the whole-exome sample mixture dataset. In this example, tumor has 50% purity and normal has 95% purity. Tumor and normal alignments coverages are ranging from 20x to 100x. The confidence or quality scores are used to derive the precision-recall curves. The highest F1-score achieved by each algorithm is indicated in the legend and marked with a solid circle on the curve.

### Training robustness

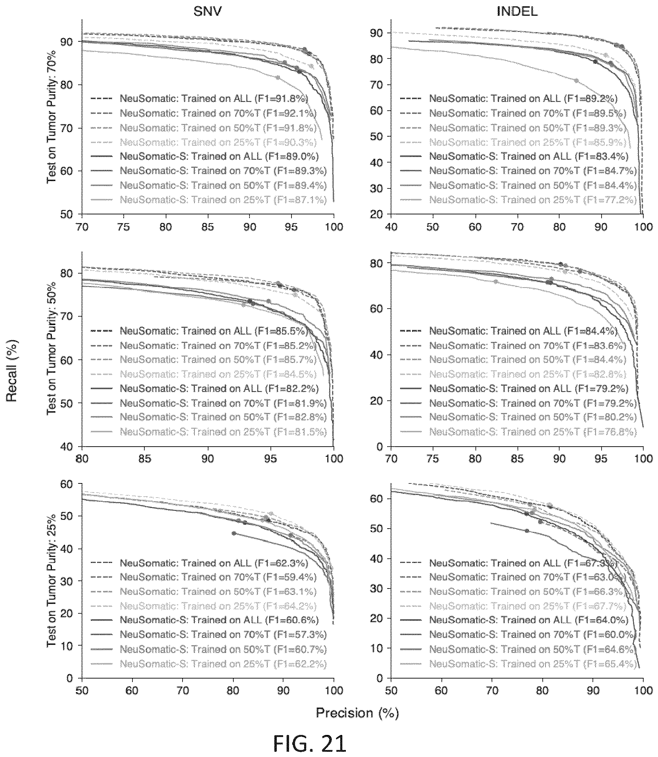

We assessed the robustness of NeuSomatic's training for specific purity by training and testing on different purities for the DREAM Challenge Stage 3 datasets. FIG. 21 shows that performance degrades only marginally even when we trained and tested on very different tumor purities. We also observed that training using data aggregated from multiple tumor purities was as good as training on the target tumor purity (FIG. 21). This suggests that a training set incorporating multiple tumor purities is sufficient to get a model, which is robust to tumor purity variation.

FIG. 21 illustrates the performance analysis of cross sample training for Dream Challenge Stage 3 dataset. We tested each of the samples with tumor purities of 70%, 50%, and 25% with NeuSomatic models trained on different purities, as well as a model trained on collective inputs from all different purities. The confidence or quality scores are used to derive the precision-recall curves. The highest F1-score achieved by each algorithm is printed in the legend and marked with a solid circle on the curve.

### Comparison on real data

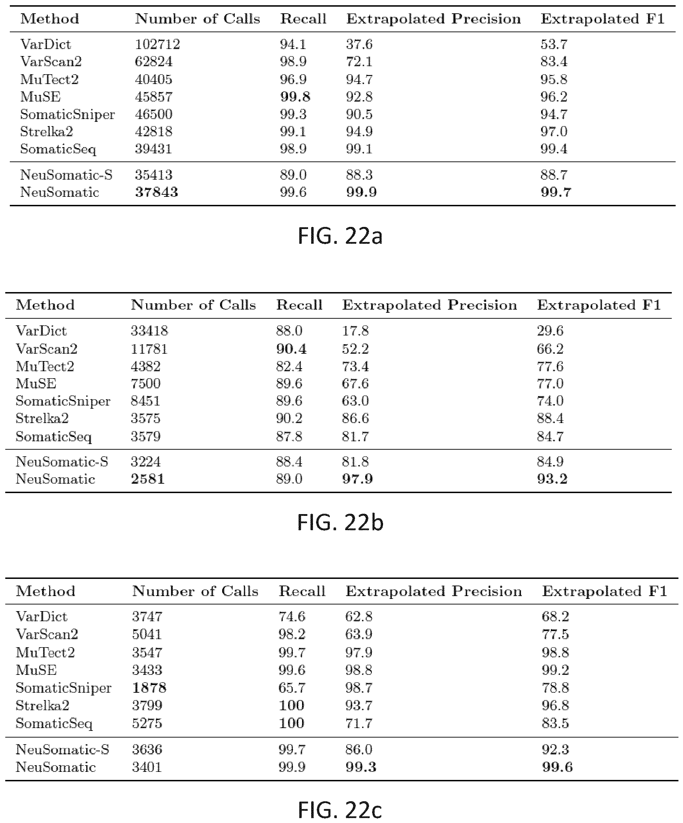

In the absence of a high-quality, comprehensive ground truth dataset for somatic mutations²¹, like the Genome-in-a-Bottle gold set for germline variants²², we would not be able to calculate F1 accuracy outside of synthetic data. Fortunately, there are existing datasets with validated somatic mutations we could take to estimate the accuracy performance of NeuSomatic on real data (See Methods for more details on how to extrapolate the F1-score on real data). We used two datasets: CLL1²³, a chronic lymphocytic leukemia patient whole-genome data with 961 validated somatic SNVs, and COLO-829^{24,25}, an immortal metastatic malignant melanoma cell line-derived whole-genome dataset with 454 validated somatic SNVs. To evaluate NeuSomatic on these two real WGS samples, we used models trained on the DREAM Challenge Stage 3. As shown in FIGS. 22a and 22b, NeuSomatic achieved the highest extrapolated F1-score of 99.7 and 93.2%, respectively, for the COLO-829 malignant melanoma sample and the CLL1 chronic lymphocytic leukemia sample. We also evaluated NeuSomatic on a TCGA^{26,27} whole-exome sequencing (WES) sample of colorectal adenocarcinoma (TCGA-AZ-6601), achieving the highest extrapolated F1-score of over 99.6%(FIG. 22c).

FIG. 22a shows the performance of different somatic mutation detection methods on real dataset COLO-829. FIG. 22b shows the performance of different somatic mutation detection methods on real dataset CLL1. FIG. 22c shows the performance of different somatic mutation detection methods on real dataset TCGA-AZ-6601.

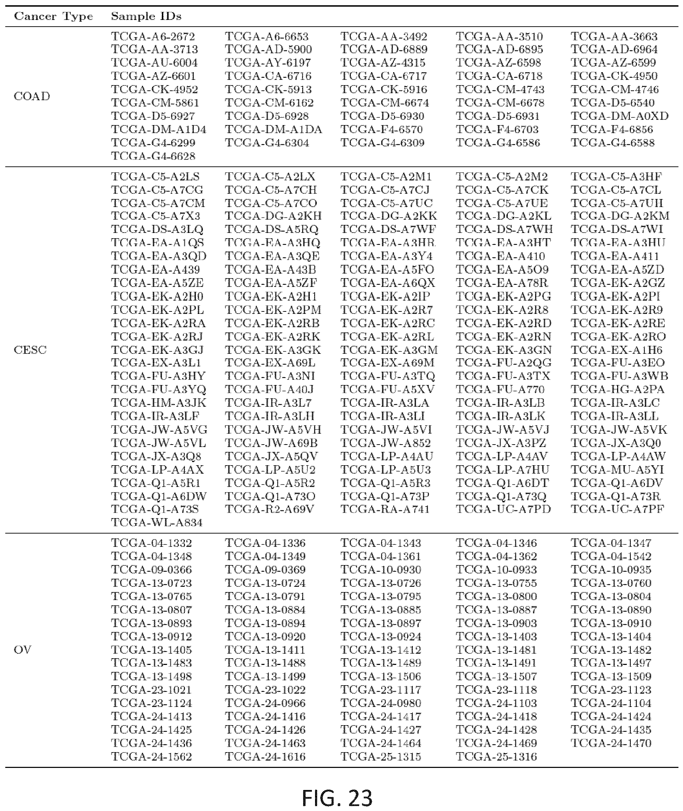

In order to demonstrate NeuSomatic's scalability and cost effectiveness on the cloud, we also processed 261 whole-exome sequenced cancer samples (FIG. 23) from TCGA on the Microsoft Azure cloud platform using both the ensemble and stand-alone modes. These samples were taken across multiple cancer types including colorectal adenocarcinoma, ovarian serus adenocarcinoma, cervical squamous cell carcinoma, and endocervical adenocarcinoma. While the cloud platform enabled us to automatically spin up compute instances on demand, it took, on average, 2.42 hours and 0.72 hours for ensemble and stand-alone modes, respectively, to process each sample. Using Azure's pre-emptible compute instances (the standard H16 instance types were used with 16 cores each) resulted in low per sample processing costs of 0.77 USD and 0.23 USD for the ensemble and stand-alone modes, respectively. We also assessed the accuracy of NeuSomatic on these samples by comparing against the 44,270 validated SNPs across these samples, which provided us with recall rates of 98.9 and 97.2% for ensemble and stand-alone modes, respectively. Thus, NeuSomatic not only can be used on different sequencing technologies or sequencing strategies but also can be run on a variety of compute platforms including a local HPC cluster and on an elastic cloud compute infrastructure.

FIG. 23 is a list of 261 TCGA cancer samples used for Microsoft Azure experiment. Samples are taken across three cancer types: colorectal adenocarcinoma (COAD), ovarian serus adenocarcinoma (OV), and cervical squamous cell carcinoma and endocervical adenocarcinoma (CESC).

### Discussion

NeuSomatic is the first deep learning based framework for somatic mutation detection, which is high-performing and universal. While using the same CNN architecture, it achieves the best accuracy for varying tumor purities across multiple datasets ranging from synthetic to real, across multiple sequencing strategies ranging from whole genome to targeted as well as across multiple sequencing technologies ranging from short reads to high-error long reads. Specifically, for low tumor purities and low allelic frequencies, NeuSomatic significantly outperforms other state-of-the-art somatic mutation detection methods, thus demonstrating its capability in addressing the hard problem. NeuSomatic utilizes an efficient implementation of convolutional neural networks for solving the somatic mutation detection problem with speed and accuracy. It uses a novel summarization of tumor/normal alignment information as a set of input matrices that can effectively capture main signals in the genomic context. Training the proposed CNN architecture on these matrices enables learning feature representations directly from the raw data. The deep features, learned from observed training data, can accurately identify the important mutation signatures that can differentiate true calls from artifacts introduced by sequencing errors, cross contamination, or coverage biases. We believe NeuSomatic advances the state-of-the-art significantly by providing a very broadly applicable approach for somatic mutation detection.

### Methods

### ICGC-TCGA DREAM Challenge data

Stage 3 data consist of a normal sample and a tumor sample constructed by computationally spiking 7,903 SNVs and 7,604 INDELs mutations into a healthy genome of the same normal sample with three different AFs of 50, 33, and 20% to create synthetic but realistic tumoral normal pairs. Stage 4 data have similar formation, but with 16,268 SNVs and 14,194 INDELs in two subclones of 30 and 15% AF. We then constructed an impure normal by mixing 95% normal and 5% tumor reads. We also constructed four tumor mixtures by mixing tumor and normal, respectively, at 100:0, 70:30, 50:50, and 25:75 ratios. Thus, the somatic mutations across these four tumor mixture ratios have AFs ranging from 5 to 50% for Stage 3 dataset, and 3.75 to 30% for Stage 4 dataset.

### Platinum synthetic tumor data

We downloaded 200× Platinum genomes samples NA12878 and NA12877 and their truth germline variants (v2017-1.0)¹⁸ to construct a virtual tumor and normal pair (ENA accession number PRJEB3246). For the normal, we downsampled NA12877 to 50×. For tumor, we constructed three 50× in silico mixture samples with 70, 50, and 25% tumor purities, by independently downsampling NA12877, respectively, at 15×, 25×, and 37.5×, and mixing each with downsampled NA12878 at 35×, 25×, and 12.5×. We use the heterozygous and homozygous variants in NA12878, which are reference calls in NA12877 and are at least five bases apart from NA12877 variants and 300 base apart from each other as the truth set for the training and evaluation steps (1,103,285 SNVs and 174,754 INDELs). Thus, depending on the zygosity of the germline variants in NA12878, somatic mutations across these three tumor mixture ratios have AFs ranging from 12.5 to 70%.

We also generated another 50× virtual tumor sample by randomly spiking reads from a downsampled (to 50× coverage) NA12878 into a downsampled (to 50× coverage) NA12877 data at heterozygous and homozygous variant locations in NA12878, which are reference calls in NA12877. For each variant, we randomly assigned the frequencies of spiked reads from a binomial distribution with means [0.05, 0.1, 0.2, 0.3]. Thus, depending on the zygosity of the variant, the mean somatic mutations AFs ranges from 2.5 to 30%. To avoid ambiguity in the truth set, we only used variants for which the relevant paired-end reads did not overlap any other variants (316,050 SNVs and 46,978 INDELs). This generated a contaminated tumor with reads from NA12878. We also used another independent downsampled (to 50×) data for NA12877 as the pure normal.

For both experiments, FastQ files and truth germline variants were downloaded and aligned with BWA-MEM (v0.7.15)²⁸ followed by Picard MarkDuplicates (v2.10.10) (https://broadinstitute.github.io/picard), and GATK IndelRealigner and Base Quality Score Recalibration (v3.7)²⁹.

### Real tumor-normal pair data

We used the CLL1 chronic lymphocytic leukemia dataset²³ (accession: https://www.ebi.ac.uk/ega/datasets/EGAD00001000023) and the COLO-829 immortal metastatic malignant melanoma cell line dataset^{24,25} (accession: https://www.ebi.ac.uk/ega/studies/EGAS00000000052) to assess our approach on real tumor-normal pair data with published lists of validated somatic mutations.

The COLO-829 dataset consists of 80× whole-genome sequencing tumor sample and its matched normal blood COLO-829BL sample at 60×, with 454 validated somatic SNVs. CLL1 has a whole-genome sequencing tumor sample and a matched normal, respectively, at 53× and 42× coverage, with 961 published somatic SNVs.

The TCGA-AZ-6601^{26,27} dataset is a whole-exome sequencing of a colon adenocarcinoma tumor sample and its matched normal tissue from TCGA. The tumor and normal samples were sequenced at depths of 145× and 165×, respectively. We used 952 validated SNVs in TCGA³⁰ and COSMIC³¹ databases as the ground truth somatic mutations for this sample.

For real data, we compute extrapolated precision as the percentage of predicted somatic mutations that have been called by at least two stand-alone methods, or have been reported as verified somatic mutations in at least two samples of the same cancer type in COSMIC database. We then compute extrapolated F1-score based on the harmonic mean of recall and this extrapolated precision.

### Whole-exome and targeted panel data

To assess NeuSomatic on different target enrichment experiments we used whole-exome datasets from the Ashkenazi Jewish trio²⁰. We downloaded deep-sequenced (200× coverage) whole-exome alignment files for HG003 and HG004 (ftp://ftp-trace.ncbi.nlm.nih.gov/giab/ftp/), along with the high-confidence germline variants (Genome-in-a-Bottle release v3.3.2). We then used mixtures of random 70×, 50×, and 25× downsamples of HG004 and 30×, 50×, and 75× downsamples of HG003, to construct 70, 50, and 25% pure tumor samples, respectively. We also constructed a 95% pure normal by mixing 95× HG003 and 5× HG004 downsampled alignments. For our analysis, we used Agilent SureSelect Human All Exon V5 BED file. The ground truth somatic mutations were identified similar to the Platinum synthetic tumor data (11,720 SNVs, 878 INDELs). Depending on the zygosity of the germline variants in HG004, somatic mutations across these three tumor mixture ratios have AFs ranging from 12.5 to 70%.

For validating the performance on the target panel, we restricted the above alignment and truth data to Illumina's TruSight inherited disease panel BED file (216 SNVs, 5 INDELs). We only evaluated the performance on SNVs due to the limited number of true INDELs in the target panel region.

### PacBio data

For long-reads analysis, we downloaded the high-confidence germline variants (Genome-in-a-Bottle release v3.3.2) for HG002 sample (ftp://ftp-trace.ncbi.nlm.nih.gov/giab/ftp/)²⁰. We built the long-reads error profile using the CHM1 dataset³² (SRA accession SRX533609). We then simulated a 100× pure normal sample using the VarSim simulation framework³³ in combination with the LongISLND in silico long-reads sequencer simulator³⁴. Using a set of random somatic mutations, we also simulated a 100× pure tumor sample with the same error profile. We used NGMLR (v0.2.6)³⁵ to align the sequences. We then mixed a 47.5× downsample of pure normal alignment and 2.5× downsample of the pure tumor alignment to form the 50× normal pair with 95% purity, and mixed 40×, 35×, and 25× independent downsamples of normal, respectively, with 10×, 15×, and 25× downsamples of pure tumor, to construct 50× tumor mixtures of 20, 30, and 50% purity. We restricted the training set to a 120 megabase region in chromosome 1 (with 39,818 truth somatic SNVs and 38,804 truth somatic INDELs), and the testing set to whole chromosome 22 (with 12,201 truth somatic SNVs and 12,185 truth somatic INDELs). Somatic mutations across the three tumor mixture ratios have AFs ranging from 20 to 50%.

### Candidate mutation preparation

As the first step, we scan tumor read alignments to find candidate locations with evidence of mutations. Many of these positions have either germline variants or erroneous calls made due to the complexity of the genomic region, or sequencing artifacts. We apply a set of liberal filters on the set of candidate locations to make sure the number of such locations is reasonable. In general, for SNVs, we required AF ≥0.03 or more than two reads supporting the SNV and Phred scaled base quality score larger than 19 (larger than 14 for real WES dataset) as the minimum requirements. For 1-base INDELs, we required AF ≥0.02 or more than one read support. For INDELs larger than 1-base, we require AF ≥0.03. For the ensemble approach, we also included any somatic mutation detected by other somatic mutation detection methods as input candidate. For the PacBio dataset, we used AF ≥0.1 for SNVs and INDELs larger than 1-base, and AF ≥0.15 for 1-base INDELs.

For the DREAM Challenge dataset, we excluded variants existing in dbSNP³⁶ from the input candidates. For fair comparison, we also filtered dbSNP calls for all other somatic mutation detection tools.

### Input mutation matrix

For each candidate position, we prepare a 3-dimensional matrix M with k channels of size 5 × 32 (FIG. 10a, 10c, and 10d). The five rows in each channel corresponds to four DNA bases A, C, G, and T, and the gap character ('-'). Each of the 32 columns of the matrix represents one column of the alignment.

For each candidate location, we extract the tumor and normal read alignments. As shown in FIG. 10a, we then consider the read alignments of tumor and normal sample to the reference as an MSA. To this end, we augment the reference sequence by adding gaps to the reference sequence, when there is insertion in reads. It must be noted that this process does not need any further realignment of the original read alignments of input BAM files, but only restructuring the alignments into MSA format by assigning additional columns wherever insertions has occurred. If there are multiple distinct insertions in multiple reads after a specific position, we consider them as left-aligned sequences and put them in the same set of columns (See for instance insertions of A and C bases in the ninth column of the toy example in FIG. 10a). With this read representation, we find the frequency of A/C/G/T/- characters in each column and record separate matrices for tumor and normal samples (channels C2 and C3 in matrix M). In channel C1, we record the reference base (or gap) in each column. Channels Ci (4 ≤ i ≤ k) record other alignment signals in tumor and normal samples, such as coverage, base quality, mapping quality, strands, clipping information, edit distance, alignment score, and paired-end information. For instance, for the base quality channel, we have a matrix of size 5 × 32 for each sample, which records the average base quality of reads that have a given base (for a given row) in each column. As another instance, for the edit distance channel, we have a matrix of size 5 × 32 for each sample, which records the average edit distance of reads that have a given base (for a given row) in each column. One channel of matrix M is devoted to specify the column where the candidate is located in. In the current implementation, we used a total of 26 channels in the stand-alone NeuSomatic-S approach.

For the ensemble extension of NeuSomatic, we also included additional channels to capture features reported by each of the six individual methods used. In this implementation, we used 93 additional channels to represent features extracted from other methods, and alignments reported by SomaticSeq. Thus, the ensemble mode of NeuSomatic had 119 input channels for each candidate matrix.

For each candidate location, we report the alignment information in a window of seven bases around the candidate. We reserve 32 columns to take into account the augmented alignment with insertions. In rare cases where we have a large insertion, 32 columns may not be enough to represent the alignment. For such cases, we truncate the insertions so that we can record at least three bases in the vicinity of the candidate.

### CNN architecture

The proposed CNN (FIG. 10b) consists of nine convolutional layers structured as follows. The input matrices are fed into the first convolution layer with 64 output channels, 1 × 3 kernel size and Relu activation followed by a batch normalization and a max-pooling layer. The output of this layer is then fed to a set of four blocks with shortcut identity connection similar to ResNet structure. These blocks consist of a convolution layer with 3 × 3 kernels followed by batch normalization and a convolution layer with 5 × 5 kernels. Between these shortcut blocks, we use batch normalization and max-pooling layers. The output of final block is fed to a fully connected layer of size 240. The resulting feature vector is then fed to two softmax classifiers and a regressor. The first classifier is a 4-way classifier that predicts the mutation type from the four classes of non-somatic, SNV, insertion, and deletion. The second classifier predicts the length of the predicted mutation from the four categories of 0, 1, 2, and ≥3. Non-somatic calls are annotated as zero size mutations, SNVs and 1-base INDELs are annotated as size 1, while 2-base and ≥3 size INDELs are, respectively, annotated as 2 and ≥3 size mutations. The regressor predicts the column of the mutations in the matrix, to assure the prediction is targeted the right position and is optimized using a smooth L1 loss function.

The CNN has less than 900 K parameters, which enables us to have a highly efficient implementation by using large batch sizes. The whole-genome training process took ~8 h on a machine with 8 Tesla K80 Nvidia GPU's.

### CNN training

For DREAM Challenge, Platinum, and target enrichment datasets, we randomly split the genomic regions to 50% training and 50% testing sets. For the PacBio dataset, we trained NeuSomatic on a 120 megabase region on chromosome 1, and tested it on all of chromosome 22.

For each dataset, we combined the generated training input matrices from all different tumor/normal purity scenarios, and used the combined set for training the network. We then applied this unified trained model to test in each individual tumor/normal purity setting.

The DREAM Challenge dataset has 15,507 somatic mutations for Stage 3 and 30,462 somatic mutations for Stage 4. For better network training, we spiked in ~95 K more SNVs and ~95 K more INDELs with similar AF distributions to the original DREAM data into the tumor samples of Stages 3 and 4 using BAMSurgeon¹⁹.

We trained the network using a batch size of 1000 with SGD optimizer with learning rate of 0.01, and momentum of 0.9, and we multiplied the learning rate by 0.1 every 400 epochs.

Since, in general, the input candidate locations have significantly more non-somatic (reference or germline) calls than true somatic mutations, in each epoch we use all the true somatic mutations in the training set and randomly selected non-somatic candidates with twice the number of the true somatic mutations. We used a weighted softmax classification loss function, to balance for the number of candidates in each category. For DREAM Challenge data, since we added more synthetic mutations in the training set, we boosted the weight for the non-somatic category to achieve higher precision on test set.

For assessing synthetic target enrichment datasets, we used whole-exome and whole-genome data as the training set.

To test on real WGS samples CLL1 and COLO-829, we used models trained on DREAM Challenge Stage 3 for SomaticSeq and NeuSomatic. For the real WES sample TCGA-AZ-6601, we prepared a training set using data from another TCGA WES dataset, TCGA-AZ-4315³⁰. We mixed the tumor and normal alignments from this dataset and split the mixture into two equal alignments. We then used one alignment as the pure normal and spiked in ~91 K random SNVs and ~9 K random INDELs into the other alignment using BAMSurgeon to generate a synthetic tumor sample for training. We used models trained on this synthetic tumor-normal WES dataset to test NeuSomatic and SomaticSeq on the real WES dataset, TCGA-AZ-6601. For the experiment on 261 real TCGA samples, we used a similar approach to prepare a training set using 12 TCGA samples. The models trained on this synthetic dataset were used to test on the 261 TCGA samples.

### Hyper-parameter tuning

For hyper-parameter tuning, we used 10% of the genome in the DREAM Challenge Stage 3 experiment and used the derived parameters in all other experiments.

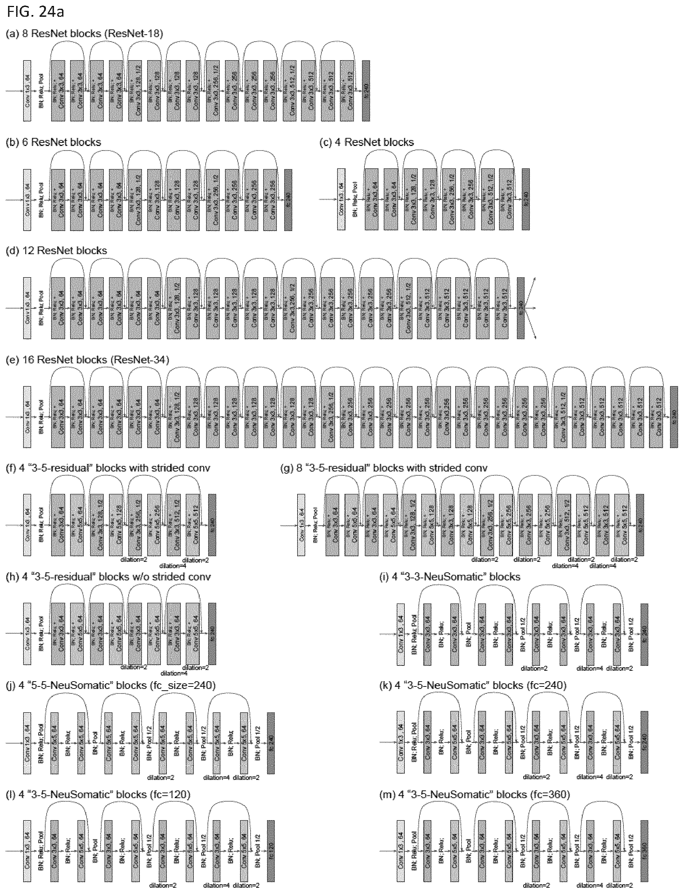

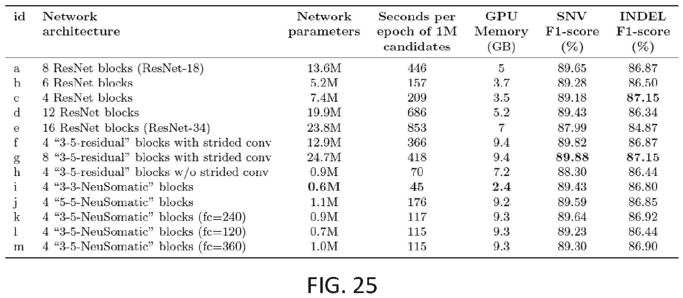

We further explored different network architectures such as the pre-activation ResNet architecture with 4 to 16 ResNet blocks (including ResNet-18 and ResNet-34 architectures) (FIGS. 24a-24e), as well as some variants of the proposed residual NeuSomatic architecture (FIGS. 24f-24m). To evaluate these networks, we split the training data in the DREAM Stage 3 dataset into two halves and used one to train different architectures and the other to evaluate them in the stand-alone mode. FIG. 25 compares these architectures in terms of accuracy, number of network parameters, memory usage, and speed. In general, all these networks can obtain relatively high accuracy compared to the conventional somatic mutation detection approaches. This observation revealed the importance of the proposed data summarization approach, which captures main signals in the genomic context of the candidates and facilitates efficient implementation of convolutional networks on the somatic mutation detection problem. The default ReSNet architectures with two 3 × 3 convolution filters (FIGS. 24a-24e) have lower average accuracy compared to those with the proposed residual blocks (FIGS. 24f-24m). In addition, networks with strided convolution (FIGS. 24a-24g) have larger number of network parameters and run-time requirements. In summary, although each network architecture shows advantages in some of the compared aspects, we selected the proposed NeuSomatic network architecture (FIG. 10b; FIG. 24k) as our default network architecture as a compromise of all these factors, while other networks can easily be adapted by users given their use-cases and time/computational constraints.

FIGS. 24a-24m illustrate the different network architectures tested. FIGS. 24a-24e illustrate ResNet architectures with different number of pre-activation residual blocks with default 3x3 conv layers. Here, strided convolutions are used with channel expansions. FIGS. 24f and 24g illustrate multiple customized residual blocks with 3x3 and 5x5 conv layers and some dilated convolutions. Here, strided convolutions are used with channel expansions. FIG. 24h illustrates four customized residual blocks with 3x3 and 5x5 conv layers and some dilated convolutions. Here, no strided convolutions are used. FIGS. 24i-24m illustrate NeuSomatic residual architecture with different residual blocks and fully-connected sizes.

FIG. 25 shows the performance analysis of the different network architectures shown in FIGS. 24a-24m. Here, all the networks are assessed with batch size of 1000 and after 600 epochs training.

### Other somatic mutation detection algorithms

We used Strelka2 (v2.8.4), Mutect2 (v4.0.0.0), SomaticSniper (v1.0.5.0), MuSE (v1.0rc), VarDict (v1.5.1), VarScan2 (v2.3.7), and SomaticSeq (v2.7.0) somatic mutation detection algorithms in our analysis, with their default settings.

We used VarDict as an alternative approach to NeuSomatic on PacBio data. To enable detecting somatic mutations on high-error rate long reads, we used VarDict with " -m 10000 -Q 1 -q 5 -X 1 -c 1 -S 2 -E 3 -g 4 -k 0 " parameter settings. Besides, as in NeuSomatic, we used AF ≥0.1 for SNVs and AF ≥0.15 for INDELs.

To train SomaticSeq, we also followed the same 50% train/test region splitting as used for NeuSomatic. In addition, as in NeuSomatic, for each dataset we combined the training data from all different tumor/normal purity scenarios to train the SomaticSeq SNV and INDEL classifiers. These unified classifiers were then used to predict in each individual tumor/normal purity setting.

For the precision-recall analysis, somatic mutations were sorted based on the confidence or quality scores assigned by each tool. For MuSE, we used the tier assignments as the sorting criterion. For VarDict, VarScan2, MuTect2, Strelka2, and SomaticSniper we, respectively, used SSF, SSC, TLOD, SomaticEVS, and SSC values reported in the VCF file for sorting. For SomaticSeq and NeuSomatic, we used the somatic mutation quality score in the QUAL field. NeuSomatic reports quality scores for the predicted somatic mutations based on the probability of predictions by CNN.

To analyze performance on real samples, we used the PASS somatic calls from different methods (For VarDict we restricted to calls with StrongSomatic status). For NeuSomatic, we used 0.97 as the quality score threshold for WGS and 0.6 for WES.

### Computational complexity

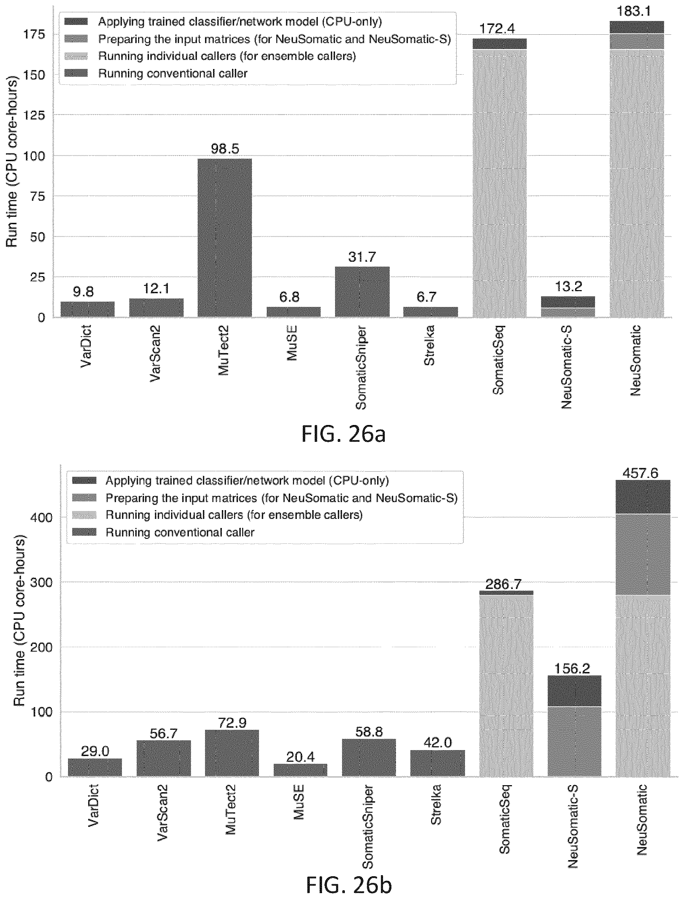

For whole-genome data, scanning 30× tumor and normal alignments to find candidates, extracting features, and preparing the input matrices can take ~3.9 h on a dual-14 core Intel Xeon CPU E5-2680 v4 2.40 GHz machine. The whole-genome training process can take ~8 h on a machine with 8 Tesla K80 Nvidia GPU's (-90 s per epoch of size 580,000). Depending on the cutoff AF on candidate somatic mutations, computing the network predictions for the candidate mutations on a 30× whole-genome data can take ~35 min (with AF cutoff of 0.05, 3.9 M candidates) to ~100 min (with AF cutoff of 0.03, 11.5 M candidates) with 8 Tesla K80 Nvidia GPUs. For 125× whole-exome data, the whole scanning, preparation, and computing the network predictions can take ~30 min. The end-to-end run times for predicting somatic mutations on a 125× whole-exome dataset and a 30× whole-genome dataset using NeuSomatic ensemble and stand-alone approaches (in CPU-only mode) were compared with other somatic mutation detection techniques in FIGS. 26a and 26b.

FIG. 26a illustrates a run-time comparison of different somatic mutation detection algorithms. CPU core-hours are shown for predicting somatic mutations on a 125x whole-exome sequencing dataset. FIG. 26b illustrates a run-time comparison of different somatic mutation detection algorithms. CPU core-hours are shown for predicting somatic mutations on a 30x whole-genome sequencing dataset.

### Code availability

NeuSomatic is written in Python and C++. Its deep learning framework is implemented using PyTorch 0.3.1 to enable GPU application for training/testing. The source code is available at https://github.com/bioinform/neusomatic under the Creative Commons Attribution-NonCommercial-ShareAlike 4.0 International license. The results in this paper were based on NeuSomatic v0.1.3.

### Reporting summary

Further information on experimental design is available in the Nature Research Reporting Summary linked to this article.

### Data availability

Sequence data for this study were collected from various sources, i.e., the European Nucleotide Archive (accession: PRJEB3246; https://www.ebi.ac.uk/ena), the Sequence Read Archive (accession: SRX1026041; https://www.ncbi.nlm.nih.gov/sra), the International Cancer Genome Consortium (project: ICGC-TCGA DREAM Mutation Calling Challenge, controlled access: https://icgc.org/), The Cancer Genome Atlas (accessions: TCGA-AZ-6601, TCGA-AZ-4315; controlled access: https://gdc.cancer.gov/), the European Genome-phenome Archive (accessions: EGAS00000000052, EGAD00001000023; controlled access: https://www.ebi.ac.uk/ega/), and the Genome-in-a-Bottle (accessions: HG002, HG003, HG004; ftp://ftp-trace.ncbi.nlm.nih.gov/giab/ftp/). Synthetic data were generated from the above datasets using the scripts at https://github.com/bioinform/neusomatic/blob/paper/etc/data_scripts.zip. All other relevant data are available upon request.

### References

1. Cibulskis, K. et al. Sensitive detection of somatic point mutations in impure and heterogeneous cancer samples. Nat. Biotechnol. 31, 213 (2013).
2. Fan, Y. et al. MuSE: accounting for tumor heterogeneity using a sample-specific error model improves sensitivity and specificity in mutation calling from sequencing data. Genome Biol. 17, 178 (2016).
3. Lai, Z. et al. VarDict: a novel and versatile variant caller for next-generation sequencing in cancer research. Nucleic Acids Res. 44, e108-e108 (2016).
4. Koboldt, D. C. et al. VarScan 2: somatic mutation and copy number alteration discovery in cancer by exome sequencing. Genome Res. 22, 568-576 (2012).
5. Kim, S. et al. Strelka2: fast and accurate calling of germline and somatic variants. Nat. Methods 15, 591-594 (2018).
6. Larson, D. E. et al. SomaticSniper: identification of somatic point mutations in whole genome sequencing data. Bioinformatics 28, 311-317 (2011).
7. Wang, Q. et al. Detecting somatic point mutations in cancer genome sequencing data: a comparison of mutation callers. Genome Med. 5, 91 (2013).
8. Alioto, T. S. et al. A comprehensive assessment of somatic mutation detection in cancer using whole-genome sequencing. Nat. Commun. 6, 10001 (2015).
9. Roberts, N. D. et al. A comparative analysis of algorithms for somatic SNV detection in cancer. Bioinformatics 29, 2223-2230 (2013).
10. Fang, L. T. et al. An ensemble approach to accurately detect somatic mutations using SomaticSeq. Genome Biol. 16, 197 (2015).
11. Poplin, R. et al. A universal SNP and small-indel variant caller using deep neural networks. Nat. Biotechnol. 36, 983 (2018).
12. Luo, R., Sedlazeck, F. J., Lam, T.-W. & Schatz, M. Clairvoyante: a multi-task convolutional deep neural network for variant calling in single molecule sequencing. https://www.biorxiv.org/content/early/2018/04/28/310458 (2018).
13. Simple convolutional neural network for genomic variant calling with tensorflow. https://towardsdatascience.com/simple-convolution-neural-network-for-genomic-variant-calling-with-tensorflow-c085dbc2026f (2017).
14. Esteva, A. et al. Dermatologist-level classification of skin cancer with deep neural networks. Nature 542, 115-118 (2017).
15. Torracinta, R. et al. Adaptive somatic mutations calls with deep learning and semi-simulated data. https://www.biorxiv.org/content/early/2016/10/04/079087 (2016).
16. Running DeepVariant. https://cloud.google.com/genomics/docs/tutorials/deepvariant (2018).
17. He, K., Zhang, X., Ren, S. & Sun, J. Deep residual learning for image recognition. Proc. IEEE conf. comput. vis. pattern recognit. 770-778 (2016) http://openaccess.thecvf.com/content_cvpr_2016/html/He_Deep_Residual_Leaming_CVPR_201 6_paper.html.
18. Eberle, M. A. et al. A reference data set of 5.4 million phased human variants validated by genetic inheritance from sequencing a three-generation 17-member pedigree. Genome Res. 27, 157-164 (2017).
19. Ewing, A. D. et al. Combining tumor genome simulation with crowdsourcing to benchmark somatic single-nucleotide-variant detection. Nat. Methods 12, 623 (2015).
20. Zook, J. M. et al. Extensive sequencing of seven human genomes to characterize benchmark reference materials. Sci. Data 3, 160025 (2016).
21. Xu, C. A review of somatic single nucleotide variant calling algorithms for next-generation sequencing data. Comput. Struct. Biotechnol. J. 16, 15-24 (2018).
22. Zook, J. M. et al. Integrating human sequence data sets provides a resource of benchmark SNP and indel genotype calls. Nat. Biotechnol. 32, 246 (2014).
23. Puente, X. S. et al. Whole-genome sequencing identifies recurrent mutations in chronic lymphocytic leukaemia. Nature 475, 101 (2011).
24. Morse, H. G. & Moore, G. E. Cytogenetic homogeneity in eight independent sites in a case of malignant melanoma. Cancer Genet. Cytogenet. 69, 108-112 (1993).
25. Pleasance, E. D. et al. A comprehensive catalogue of somatic mutations from a human cancer genome. Nature 463, 191 (2010).
26. Network, C. G. A. et al. Comprehensive molecular characterization of human colon and rectal cancer. Nature 487, 330 (2012).
27. Grasso, C. S. et al. Genetic mechanisms of immune evasion in colorectal cancer. Cancer Discov. 8, 730-749 (2018).
28. Li, H. Aligning sequence reads, clone sequences and assembly contigs with BWA-MEM. https://arxiv.org/abs/1303.3997 (2013).
29. Van der Auwera, G. A. et al. From FastQ data to high-confidence variant calls: the genome analysis toolkit best practices pipeline. Curr. Protoc. Bioinforma. 43, 10-11 (2013).
30. Grossman, R. L. et al. Toward a shared vision for cancer genomic data. N. Engl. J. Med. 375, 1109-1112 (2016).
31. Forbes, S. A. et al. COSMIC: somatic cancer genetics at high-resolution. Nucleic Acids Res. 45, D777-D783 (2016).
32. Chaisson, M. J. et al. Resolving the complexity of the human genome using single-molecule sequencing. Nature 517, 608 (2015).
33. Mu, J. C. et al. VarSim: a high-fidelity simulation and validation framework for high-throughput genome sequencing with cancer applications. Bioinformatics 31, 1469-1471 (2014).
34. Lau, B. et al. LongISLND: in silico sequencing of lengthy and noisy datatypes. Bioinformatics 32, 3829-3832 (2016).
35. Sedlazeck, F. J. et al. Accurate detection of complex structural variations using single-molecule sequencing. Nat. Methods 15, 461-468 (2018).
36. Sherry, S. T. et al. dbSNP: the NCBI database of genetic variation. Nucleic Acids Res. 29, 308-311 (2001).

### Example 2

Accurate detection of somatic mutations is challenging but critical to the understanding of cancer formation, progression, and treatment. We recently proposed NeuSomatic, the first deep convolutional neural network based somatic mutation detection approach and demonstrated performance advantages on in silico data. In this study, we used the first comprehensive and well-characterized somatic reference samples from the SEQC-II consortium to investigate best practices for utilizing deep learning framework in cancer mutation detection. Using the high-confidence somatic mutations established for these reference samples by the consortium, we identified strategies for building robust models on multiple datasets derived from samples representing real scenarios. The proposed strategies achieved high robustness across multiple sequencing technologies such as WGS, WES, AmpliSeq target sequencing for fresh and FFPE DNA input, varying tumor/normal purities, and different coverages (ranging from 10× - 2000×). NeuSomatic significantly outperformed conventional detection approaches in general, as well as in challenging situations such as low coverage, low mutation frequency, DNA damage, and difficult genomic regions.

### Introduction

Somatic mutations are critical cancer drivers. Accurate somatic mutation detection enables precise diagnosis, prognosis, and treatment of cancer patients¹. Several tools have been developed to identify somatic mutations from next-generation sequencing technology²⁻¹¹. In general, conventional techniques pinpoint somatic mutations from background noise, germline variants, and/or cross-contaminations through various statistical/algorithmic modeling approaches employing a set of hand-crafted sequencing features extracted in the tumor-normal paired sequencing data²⁻¹⁰. Those manually designed approaches are not easily generalizable beyond the specific cancer types, sample types, or sequencing strategies for which they were developed.

Recently, a deep learning based somatic mutation detection approach, called NeuSomatic, has been proposed that uses a convolutional neural network (CNN) to learn feature representations directly from raw data¹¹. NeuSomatic uses a novel summarization of tumor/normal alignment information as a set of input matrices that can be adopted to efficiently train models that learn how to effectively differentiate true somatic mutations from artifacts. The network models trained by NeuSomatic can capture important mutation signals directly from read alignment and genomic-context without manual intervention. NeuSomatic, thus, provides a framework that can easily be applied to different problem statements including diverse sequencing technologies, cancer types, tumor and normal purities, and mutation allele frequencies, through training on proper data. Moreover, it can be implemented as a stand-alone somatic mutation detection method or with an ensemble of existing methods to achieve the highest accuracy. NeuSomatic has been shown to significantly outperform conventional techniques on in silico data sets. Given the lack of thoroughly-characterized benchmark samples with known "ground-truth" somatic mutations, performance evaluation on real samples was limited to a partial sensitivity analysis on a small number of validated somatic variants. Thus, despite the advantages seen by implementing NeuSomatic's CNN-based framework on in silico data, the accuracy and reproducibility of NeuSomatic on real cancer samples have not been comprehensively evaluated to date.

Recently, the Somatic Mutation Working Group in the FDA-led Sequencing Quality Control Phase II (SEQC-II) consortium developed reference matched tumor-normal samples: a human triple-negative breast cancer cell line (HCC1395) and a matched B lymphocyte derived normal cell line (HCC1395BL)^{12,13}. Using orthogonal sequencing technologies, multiple sequencing replicates, and multiple bioinformatics analysis pipelines, the SEQC-II consortium has developed a well-defined "Gold Set" of somatic Single Nucleotide Variants (SNVs) and insertion/deletions (INDELs) for HCC1395.

As the first comprehensive and well-characterized paired tumor-normal reference cancer samples, this dataset along with the accompanying sequencing data prepared on multiple sites and technologies provides a unique resource to achieve two important purposes. First, using the high-confidence somatic mutation call set developed for these reference samples by the consortium, we performed in-depth analysis of deep learning based somatic mutation detection in a real cancer sample, in comparison with the conventional schemes. Second, we explored various model-building strategies using SEQC-II data to train the CNN in NeuSomatic and identified effective training approaches on multiple data sets derived from samples representing real scenarios. We evaluated the proposed strategies on whole genome sequencing (WGS), whole exome sequencing (WES), and AmpliSeq targeted sequencing datasets with converges ranging from 10 × - 2000 ×. The WGS and WES data were derived from Formalin-Fixed Paraffin-Embedded (FFPE) and fresh DNA, using three library preparation protocols with various input amounts, and from multiple platforms/sequencing sites. Using tumor and normal titration, we evaluated different approaches for 5%- 100% tumor purities, 5% of contaminated match normal, and with 10 × - 300 × WGS coverage. The proposed strategies to train and implement the deep learning framework in NeuSomatic achieved high robustness across all the aforementioned real scenarios, and significantly outperformed conventional paired tumor-normal somatic mutation detection approaches.

Our analysis on SEQC-II reference cancer samples demonstrates that the deep learning scheme implemented in NeuSomatic helps to overcome the main challenges in somatic mutation detection which are not easy to resolve using conventional techniques. The deep learning models and strategies derived from our study can thus provide the research community with actionable best-practice recommendations for robust cancer mutation detection.

### Results

### Reference samples and datasets

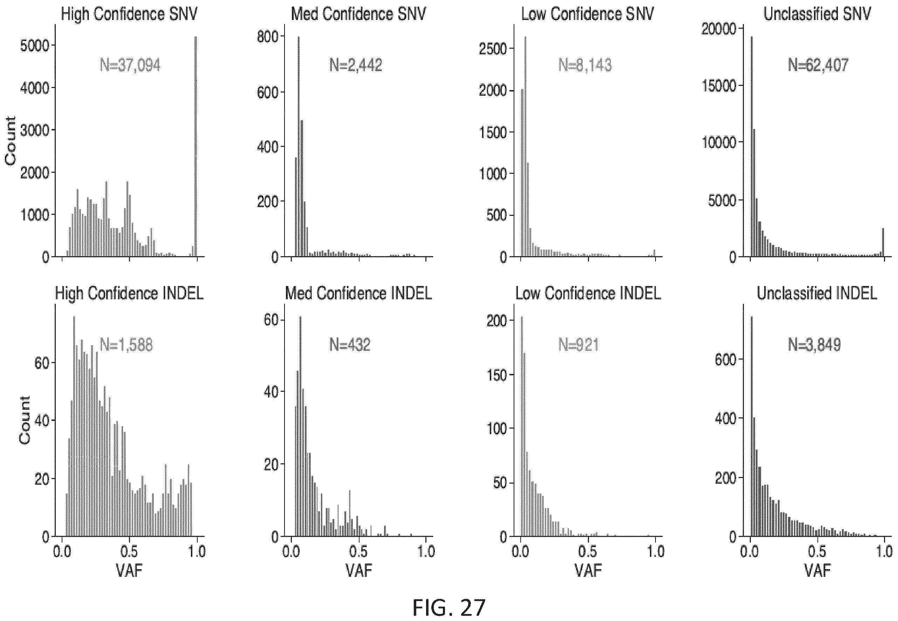

For full-spectrum analysis of the somatic mutation detection problem, we used the first comprehensive whole-genome characterized reference tumor-normal paired breast cancer cell lines (HCC1395 and HCC1395BL), developed by the Somatic Mutation Working Group of the SEQC-II consortium^{12,13}. We leveraged high-confidence somatic mutations (39,536 SNVs and 2,020 INDELs) derived by the consortium as our ground truth set, as shown in FIG. 27, which illustrates the VAF distribution of the ground truth SNV and INDEL somatic mutations in the super set of calls for HCC1395 classified by SEQC-II consortium to four confidence levels (High, Med, Low, and Unclassified). High and medium confidence calls are grouped together as the "truth set" of somatic mutations. For a broad assessment of consistency and reproducibility of predictions, we used a total of 123 replicates from diverse datasets which represent realistic cancer detection applications including real WGS, WES, and AmpliSeq target sequencing with different coverages, tumor purities, and library preparations, on FFPE and fresh DNA sequenced with multiple platforms in six centers (See Methods).

FIG. 27 illustrates VAF distribution of the ground truth SNV and INDEL somatic mutations in the super set of calls for HCC1395 classified by SEQC-II consortium to four confidence levels (High, Med, Low, and Unclassified). High and medium confidence calls are grouped together as the "truth set" of somatic mutations.

### Analysis overview

We evaluated NeuSomatic's deep learning based somatic mutation detection approach using various trained network models and compared it with seven widely-used conventional somatic mutation calling algorithms including MuTect2², MuSE³, SomaticSniper⁴, Strelka2⁵, VarDict⁶, TNscope⁷, and Lancet⁸. We assessed NeuSomatic both in its standalone mode (shown as NeuSomatic-S) and its ensemble mode, where predictions reported by MuTect2, SomaticSniper, VarDict, MuSE, and Strelka2 are also included as input channels in addition to raw data.

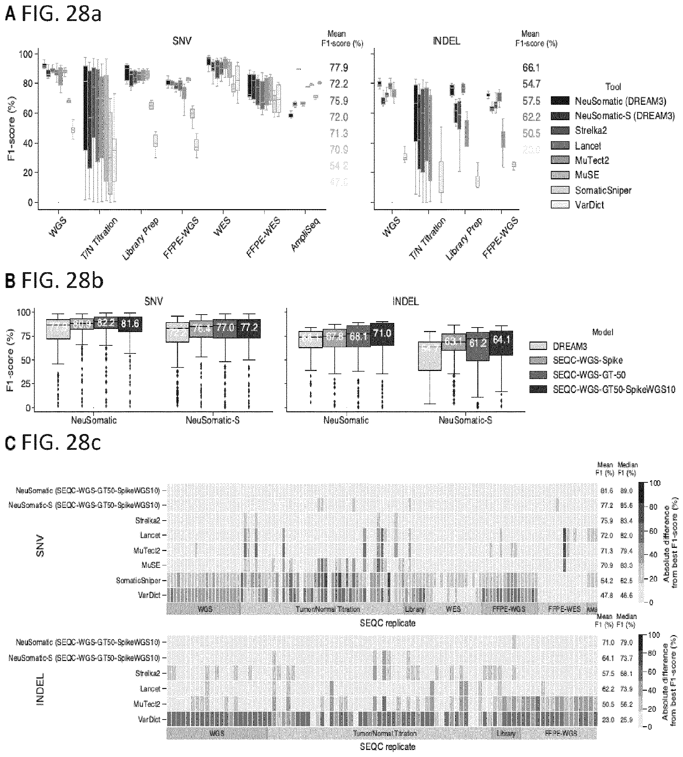

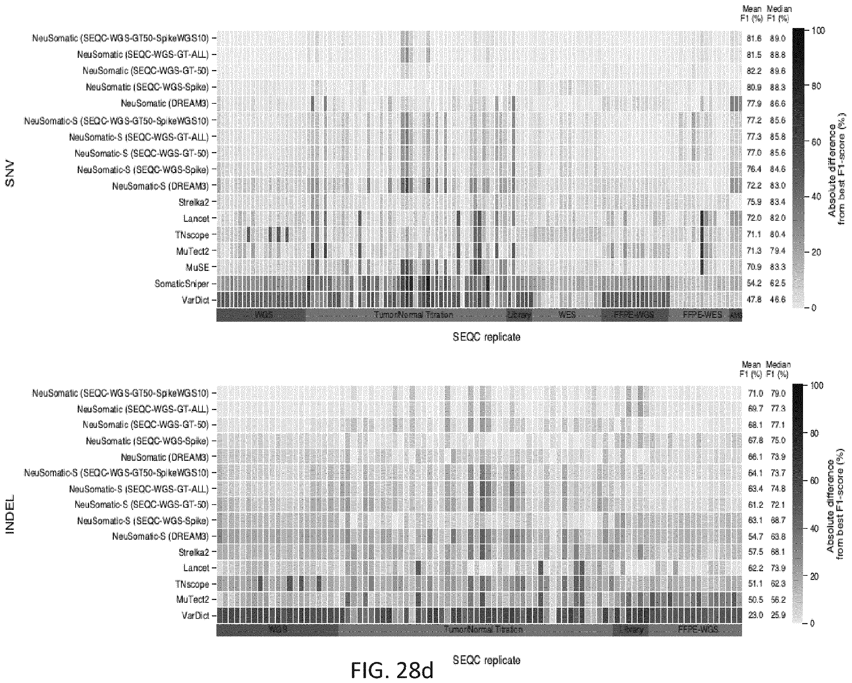

FIGS. 28a-28e show NeuSomatic's overall performance on 123 replicates in SEQC-II dataset. FIGS. 28a shows the NeuSomatic model trained on DREAM challenge stage 3 dataset outperformed other techniques when applied to SEQC-II data. FIG. 28b shows that training different NeuSomatic models using SEQC-II reference samples resulted in additional absolute 4-5% improvement in the mean F1-score. FIG. 28c shows that using a model trained on SEQC-II data, NeuSomatic achieves consistent superiority over other techniques across diverse set of replicates of different purities/coverages in WGS, WES, FFPE, AmpliSeq, and different library-prep datasets. In this subfigure, for each replicate the best F1-score was computed across different approaches. The heatmaps illustrate the absolute difference between the F1-score of any of the somatic mutation detection approaches to the best F1-score. In each panel, the mean F1-score is shown for each approach across 123 replicates. FIG. 28d compares the performance of different techniques across 123 replicates in six datasets for SNVs and INDELs. For each replicate the best F1-score was computed across different approaches. The heatmaps illustrates the absolute difference between the F1-score of any of the somatic mutation detection approaches to the best F1-sScore. The mean F1-score is shown for each approach across 123 replicates.

We used several different training models in our analysis. First, we used the already available model published recently¹¹ which was trained using in silico spike-ins from the DREAM Challenge Stage 3 dataset¹³. Despite the large discrepancy between the sample types, sequencing platforms, coverages, spike-in mutation frequencies, and heterogeneity of the samples used to train the DREAM3 model, this model outperformed other conventional techniques across the real cancer datasets of diverse characteristics by more than ~4% by the mean F1-score averaged across different samples for both SNVs and INDELs (FIG. 28a). Although this superiority supports the robustness of NeuSomatic to the stated variations, it also suggests that the deep learning framework used in NeuSomatic could perform even better through learning the sequencing features and mutation signatures from real cancer samples, especially for predicting INDELs, and for higher coverage PCR-enriched datasets like AmpliSeq.

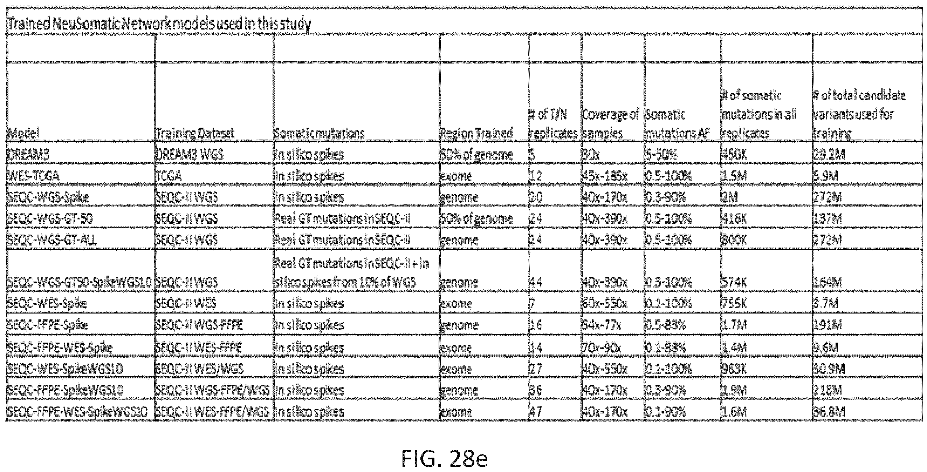

To identify the most effective strategies for building network models using real cancer samples, we evaluated ten more training approaches for NeuSomatic using the SEQC-II reference samples (FIG. 28e).

The first model (SEQC-WGS-Spike) was trained on a set of WGS pairs of in silico tumor-normal replicates, where the in silico tumors were designed by spiking mutations into distinct normal replicates. The second model (SEQC-WGS-GT-50) was trained using the ground truth somatic mutations in HCC1395 on a set of real WGS tumor-normal replicate pairs on 50% of the genome. The third model (SEQC-WGS-GT50-SpikeWGS10) was prepared by adding 10% of the training data from the first model to those for the second model to take advantage of both a high number of spike-in mutations and the realistic somatic mutations. These three models were tested on all datasets. For the specific datasets like FFPE and WES, we also prepared six additional specialized models using a set of synthetic pairs of in silico tumor and normal replicates. For all models, we evaluated the performance on the 50% held-out region of the genome (not used for the SEQC-WGS-GT-50 model). We also trained a model (SEQC-WGS-GT-ALL) using all ground truth mutations similar to the SEQC-WGS-GT-50 model but on the whole genome. Rather than being directly applicable to our WGS dataset where it has seen all true mutations, SEQC-WGS-GT-ALL will be beneficial for performance analysis on other datasets or samples other than HCC1395.

Employing the models trained on SEQC-II samples, we boosted the average DREAM3 model performance by an additional mean F1-score improvement of ~4-5% (FIG. 28b). The proposed model-building strategy was consistently best across various sample types and sequencing strategies, outperforming conventional approaches by more than 5.7% and 7.8% in terms of the mean F1-score for SNVs and INDELs, respectively. Similarly, we observed more than 5.6% improvement compared to the median F1-score of other conventional techniques across all samples (FIGS. 28c and 28d).

### WGS dataset

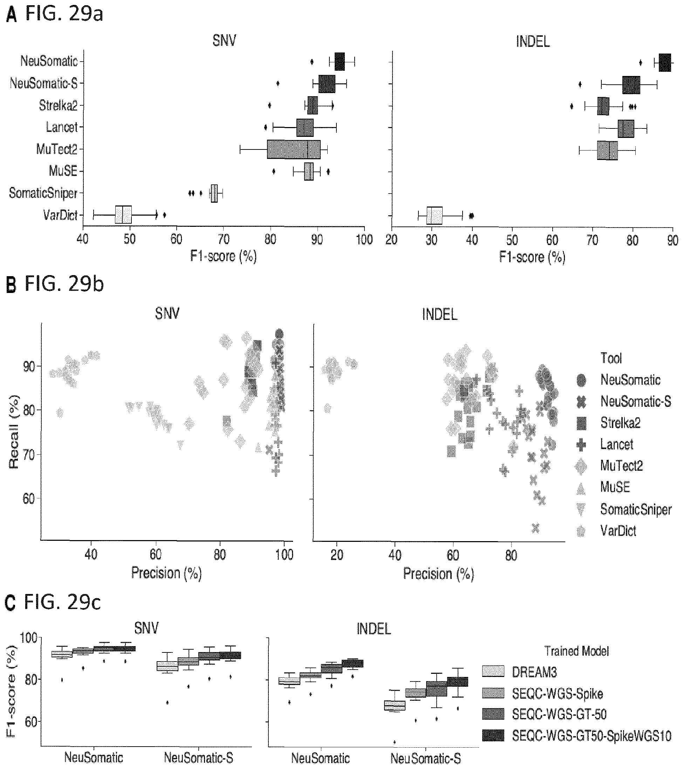

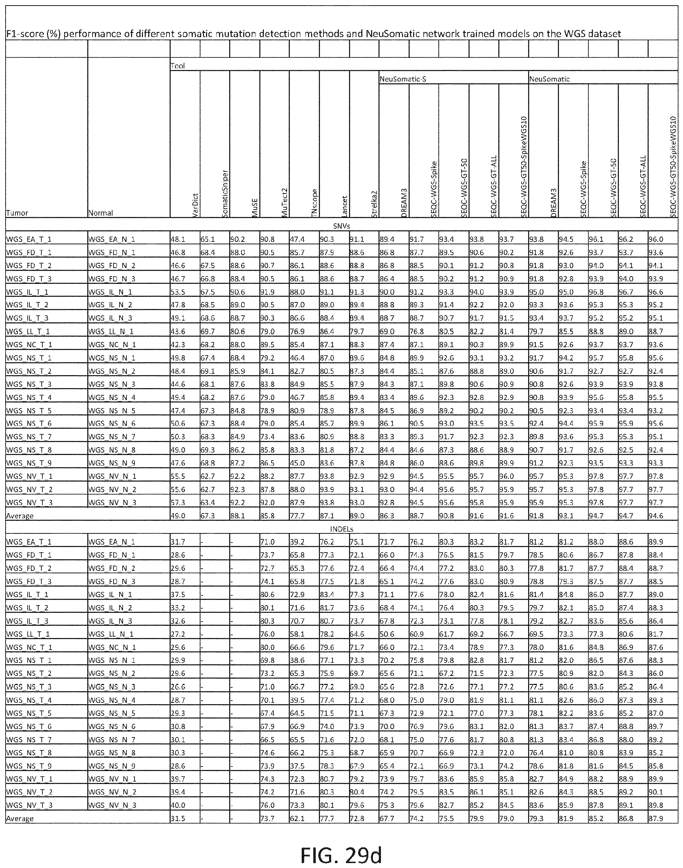

We evaluated the performance of the aforementioned somatic calling techniques and network models on the 21 WGS replicates sequenced in six sequencing centers using the HiSeqX10, HiSeq4000, and NovaSeq S6000 platforms (FIGS. 29a-29d).

The NeuSomatic SEQC-WGS-GT50-SpikeWGS10 model performed consistently better than other schemes with minor variations across replicates, demonstrating robustness and reproducibility (FIG. 29a). NeuSomatic yielded average F1-scores of 94.6% and 87.9% for SNVs and INDELs with more than 5.6% and 10.2% superiority, respectively in SNVs and INDELs over the mean F1-scores of other conventional somatic mutation detection schemes. Precision-recall analysis revealed that the high precision of NeuSomatic compared to other techniques drove this superiority (FIG. 29b). Comparing different model training strategies also revealed that NeuSomatic-S INDEL calling benefit more from training using ground truth somatic mutations, while, in general, we observed significant improvements of up to 11% from using SEQC-II reference samples compared to the DREAM3 model (FIG. 29c).

### Tumor purity and contaminated normal

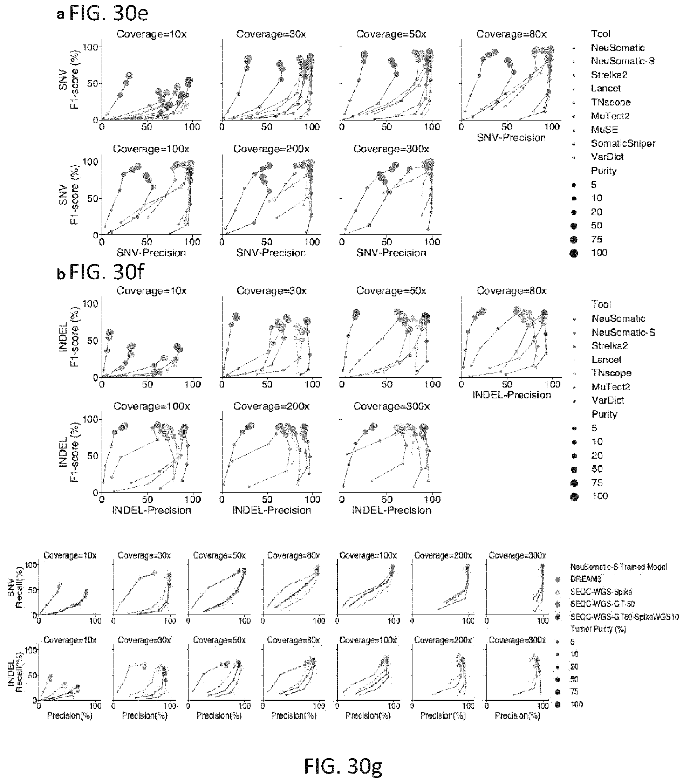

As tumor purity and the contamination of tumor cells in match normal sample would greatly impact mutation detection accuracy, we investigated model robustness on different sequencing depths and sample purities by tumor-normal titration of WGS samples. We first studied tumor samples with 5%-100% purity paired with pure normal samples for 10×-300× coverage ranges (FIGS. 30a-30h). In general, NeuSomatic maintained superiority over other schemes despite tumor purity and coverage variations, which reflected its robustness (FIGS. 30a, 30e, and 30f). It had the largest advantages over conventional schemes for more challenging cases like lower coverage (e.g. ~20% F1-score advantage for a sample with 10× coverage and 100% purity).

We further analyzed the robustness to 5% tumor contamination in the normal sample for tumor sample purities ranging from 10%-100% at 80x coverage (FIG. 30b). NeuSomatic yielded high robustness to tumor-normal cross-contamination with less than 5% median absolute change in F1-score. Among the other techniques with high F1-score at pure normal, Strelka2, also showed high robustness to tumor contamination (8.4% median change in F1-score). MuTect2, MuSE, Lancet, and TNscope, despite having high F1-scores for the pure normal scenario, experienced significant drops of up to ~50% in F1-score when contaminated normal was used.

The models trained on the ground truth variants yielded the highest advantage over the DREAM3 model, in general, mainly due to the higher precision (FIGS. 30c and 30g). The SEQC-WGS-Spike model, trained on in silico tumors, also showed lower precision compared to models trained on the ground truth. In general, INDELs, low purity, and low coverage samples benefited the most from training on SEQC-II data.

Analyzing the F1-scores for different INDEL sizes across various coverages and purity settings revealed NeuSomatic's robustness to size variations for both insertions and deletions (FIG. 30d).

### Library-preparation and DNA input

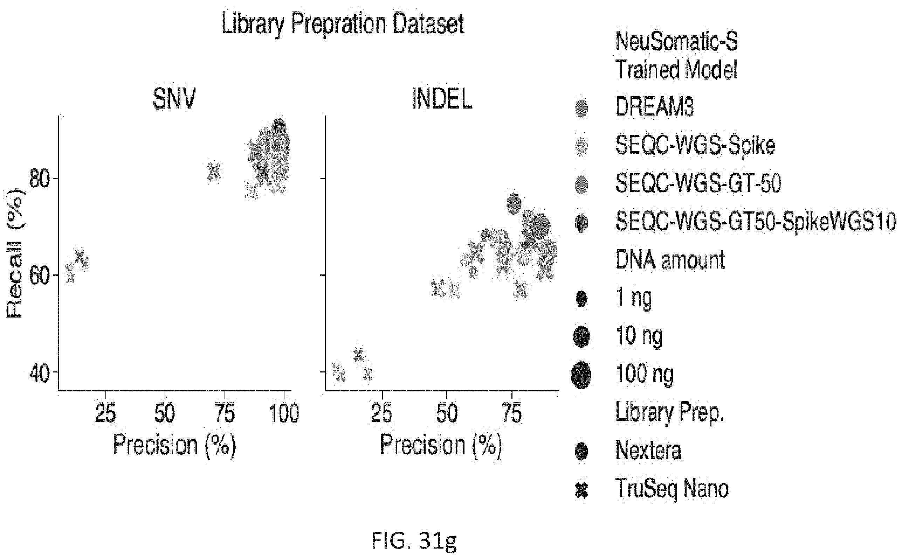

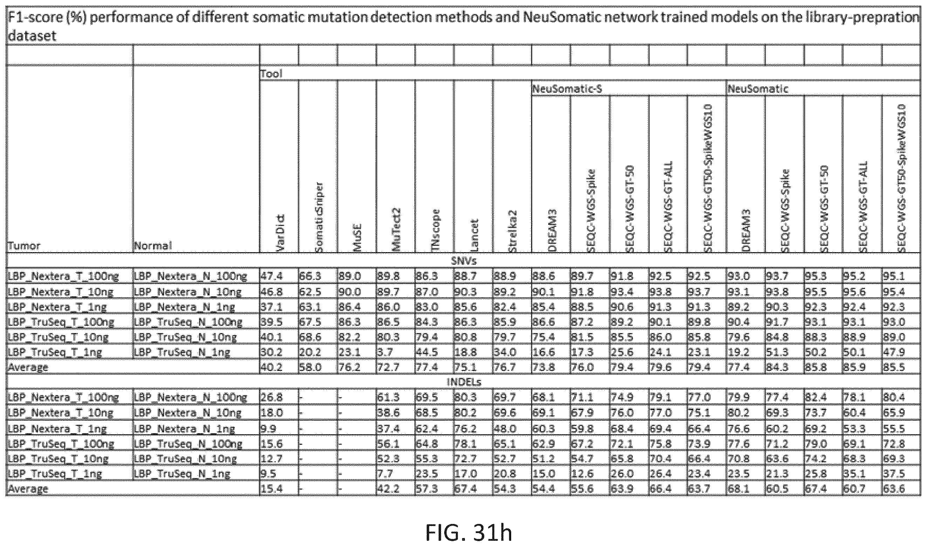

To measure the impact of library preparation on prediction robustness, we used our models to test the six replicates prepared using TruSeq-Nano and Nextera Flex protocols and three DNA input amounts: 1ng, lOng, and 100ng (FIGS. 31a-31f and 31h). NeuSomatic consistently outperformed other techniques across different library preparation approaches. For the 1 ng TruSeq-Nano libraries, all the methods had poor performance due to the limited effective coverage after removal of redundant reads (~7x). On average, NeuSomatic yielded 8.4% F1-score improvement for SNVs over conventional somatic mutation detection techniques. For INDELs, Lancet's assembly-based INDEL caller outperformed the NeuSomatic SEQC-WGS-GT50-SpikeWGS10 model by ~4% (FIG. 31a). In contrast, NeuSomatic's SEQC-WGS-GT50 model achieved similar performance to Lancet for INDELs (FIGS. 31b and 31g). Training on SEQC-II spike-in or ground truth data resulted in overall ~8.4% F1-score improvement for SNVs compared to DREAM3 model. The advantage was more pronounced in more challenging cases including TruSeq-Nano libraries with 1ng input. We also observed that NeuSomatic-S benefited more from these models.

### Captured (WES) and Targeted (AmpliSeq) panels

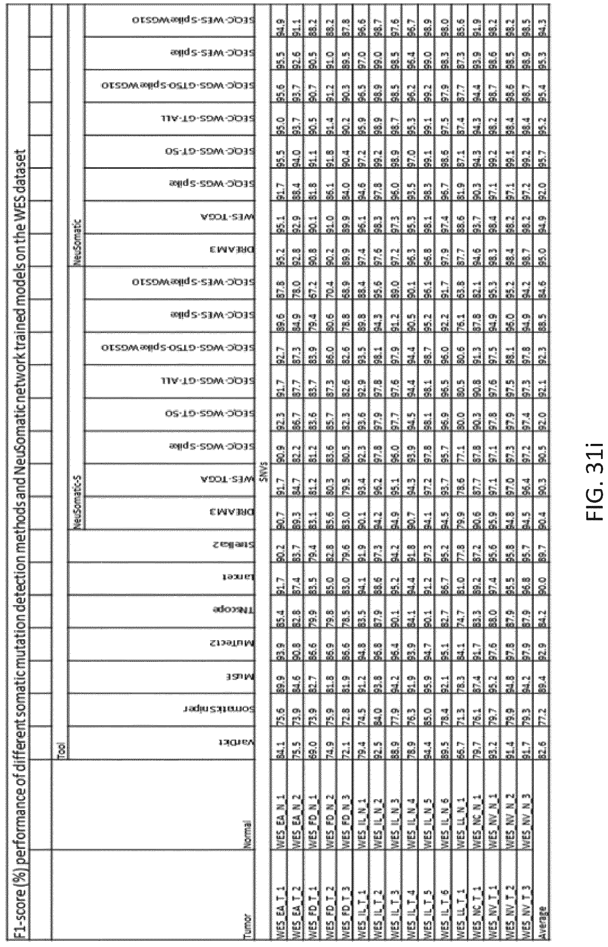

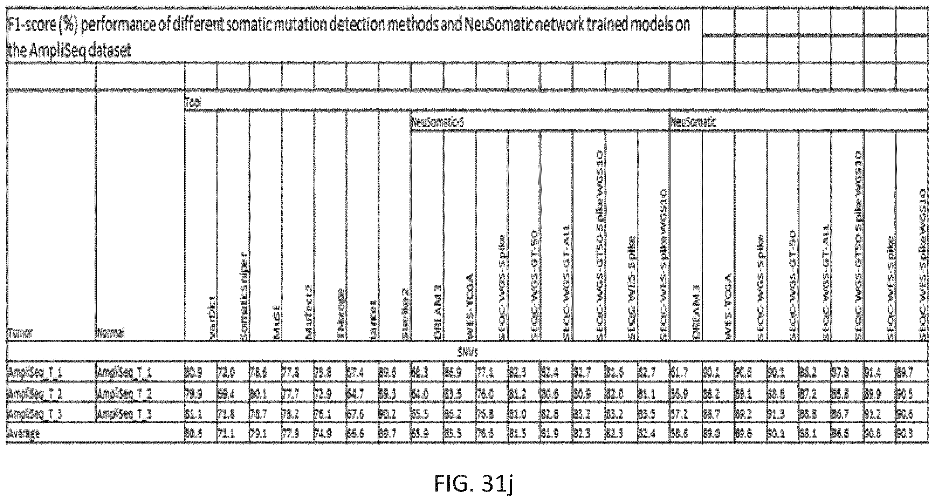

We tested our models on the 16 WES replicates sequenced at six sequencing sites, as well as three replicates in AmpliSeq dataset (FIGS. 31c-31f, 31i, and 31j). Although the models trained on SEQC-II WGS samples had different coverage and platform biases, NeuSomatic performed well on both WES and AmpliSeq datasets with 2000x coverage. For WES, NeuSomatic achieved an average SNV F1-score of 95.4% with more than 2.6% improvement over the mean F1-score of alternative schemes. On the WES dataset, models trained on WES and WGS data performed similarly with ~95% F1-score. On the AmpliSeq dataset, the NeuSomatic SEQC-WG-GT50 and SEQC-WES-Spike models achieved average F1-scores of greater than 90%, which were the highest compared to other models/schemes along with Strelka2.

### Effect of FFPE processing

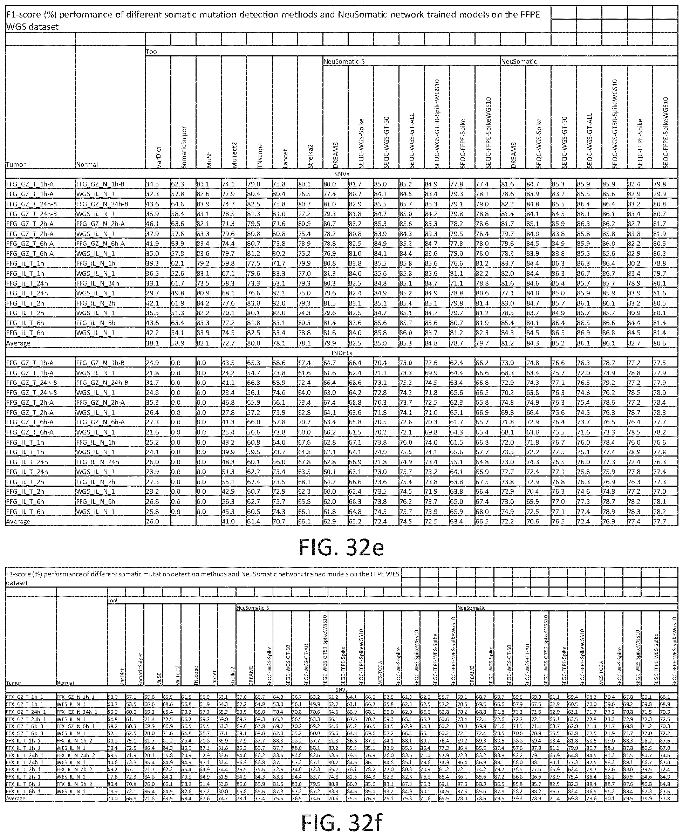

To measure the robustness of NeuSomatic's prediction on FFPE processed samples, we used 8 WGS and 7 WES FFPE replicates, prepared with four different formaldehyde fixation times of 1 hr, 2 hrs, 6 hrs, and 24 hrs. We evaluated each FFPE replicate with both FFPE and fresh normal matched sample. NeuSomatic continued consistently superior performance over the other techniques despite presence of FFPE artifacts and remained largely invariant to fixing time and the matched normal sample used (FIGS. 32a, 32e, and 32f). On WGS FFPE data, NeuSomatic yielded average F1-scores of 86.1% and 76.9%, respectively for SNVs and INDELs, with more than 4% and 6% improvement over the mean F1-score of alternative techniques. Similarly, for FFPE WES data, NeuSomatic yielded an average F1-score of 78.9%, with more than 4% superiority over the mean F1-score of conventional schemes (FIG. 32c).

In general, we observed significant boost over the DREAM3 model when we leveraged SEQC-II samples for training (FIGS. 32b and 32d). The models trained on FFPE samples seemed to improve those trained on fresh sample, only for INDEL prediction using NeuSomatic, but, for SNVs, models trained on fresh samples were superior.

### Sample specific models

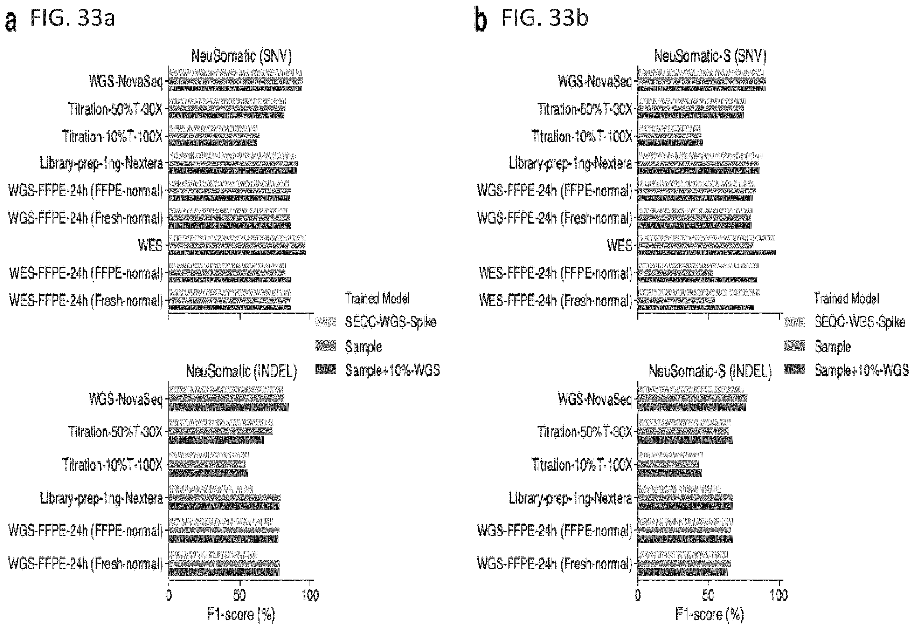

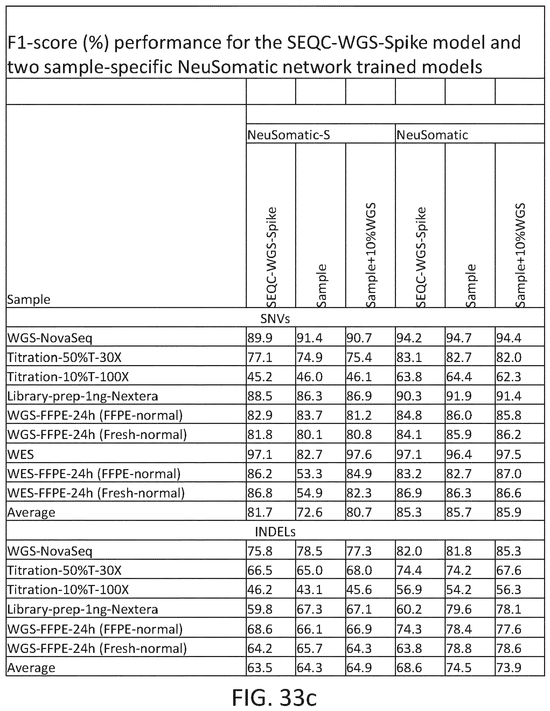

While the universal models trained on SEQC-II have shown to perform consistently better than other conventional somatic mutation detection schemes, here we explored whether sample-specific trained models could give us an additional accuracy boost, using nine replicate pairs across different SEQC-II datasets. For each sample, we trained NeuSomatic and NeuSomatic-S models using the in silico tumor-normal replicates prepared for that sample. We also trained a distinct model by combining 10% of training candidates used for the SEQC-WGS-Spike model and the training data derived for each sample. We compared these two sample-specific models with the universal SEQC-WGS-Spike model (FIGS. 33a-33c). On average, the sample-specific models yielded ~0.5% SNV and ~5% INDEL F1-score improvements over the SEQC-WGS-Spike model for NeuSomatic. The library-prep and FFPE samples benefited the most from sample specific training. For instance, the library-prep sample prepared with Nextera Flex protocol with 1ng DNA amount gained 1.6% and 19.4% absolute F1-score improvements, respectively for SNVs and INDELs. Similarly, the 24h duration FFPE WGS sample with matched fresh normal gained 1.8 and 14.8 percentage points in F1-score improvement, respectively for SNVs and INDELs. For NeuSomatic-S, only INDELs seemed to benefit from sample-specific training.

### INDEL performance

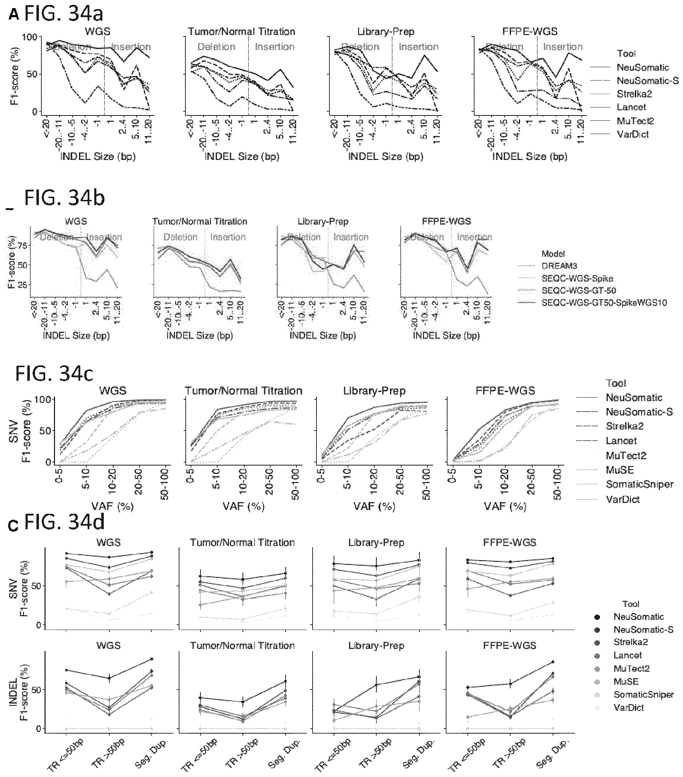

We further evaluated the accuracies for detecting INDELs of different sizes across multiple datasets (FIGS. 34a and 34b). Although NeuSomatic did not explicitly use local assembly for INDEL detection, it still consistently surpassed other approaches including the assembly-based techniques like Lancet across a broad range of INDEL size, coverage, and tumor purity (FIGS. 34a and 30d). Overall, for insertions and deletions of more than 2 base pairs (bps), NeuSomatic, respectively, yielded 24.4% and 6.5% superiority in F1-score over Lancet, the best alternative conventional technique. Comparing INDEL accuracies across different NeuSomatic training approaches, the DREAM3 model suffered mainly from low insertion detection accuracy, whereas the SEQC-II trained models robustly identified both insertions and deletions of different sizes (FIG. 34b).

### Performance analysis for different Variant Allele Frequencies (VAFs)

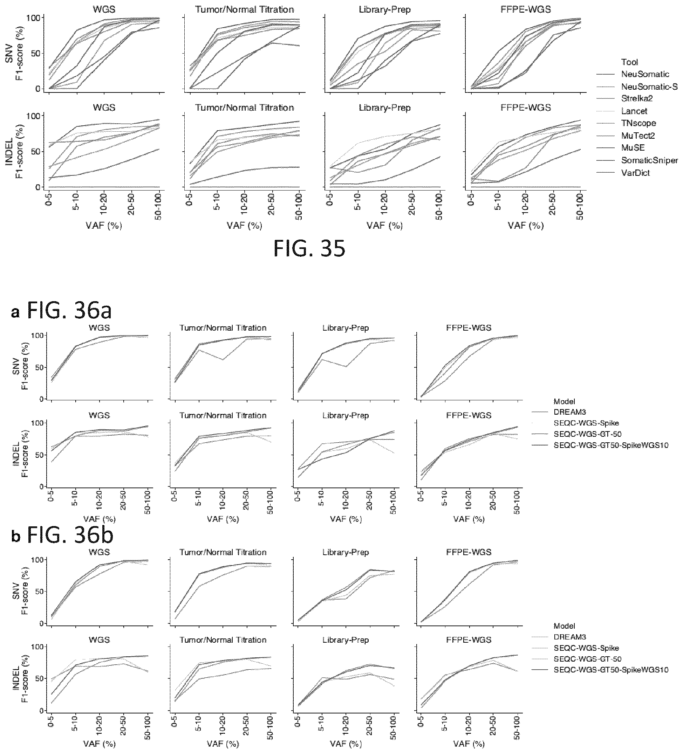

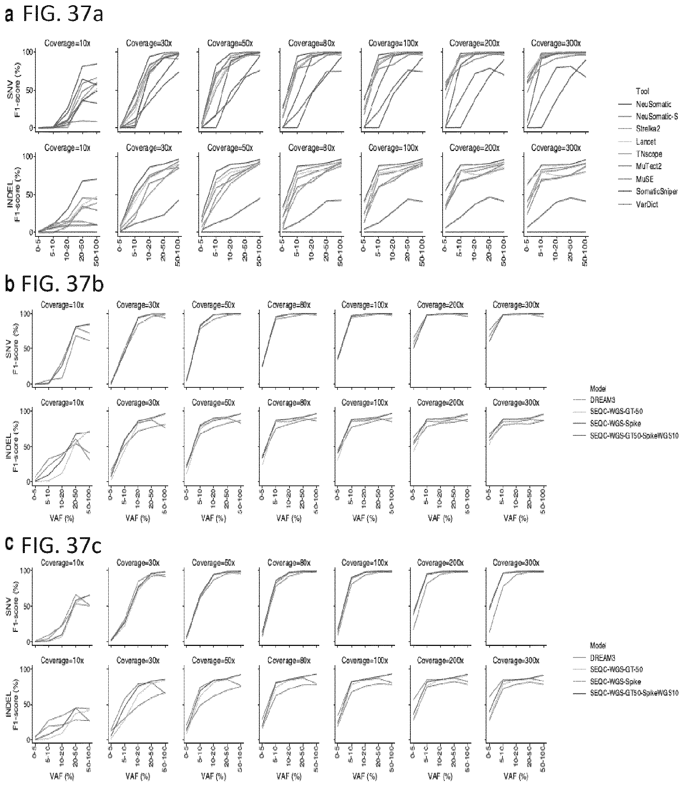

We analyzed the accuracy of different approaches across somatic mutation VAFs (FIGS. 34c and 35-37c). On different datasets, we observe NeuSomatic show an added advantage over other schemes for VAFs less than 20% (FIGS. 34c and 35). We also observed that NeuSomatic has high robustness to VAF variations with consistent predictions for as low as 5% VAF. The SEQC-II trained models clearly performed better than the DREAM3 model for mutations with 5%-20% VAFs (FIGS. 36a and 36b). Similar robustness was observed for all coverage and tumor purity settings (FIGS. 37a-37c).

### Performance on difficult genomic regions

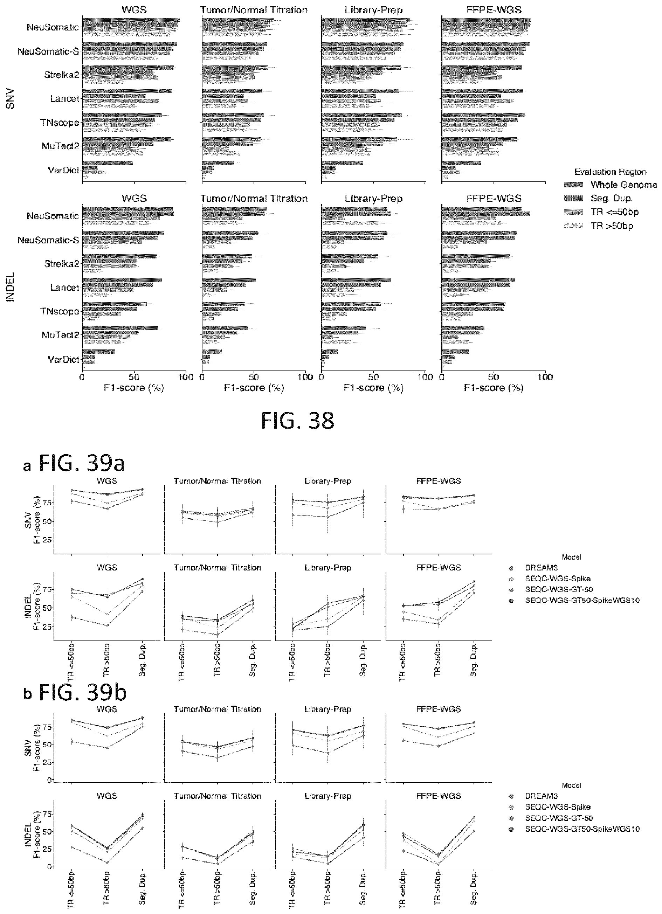

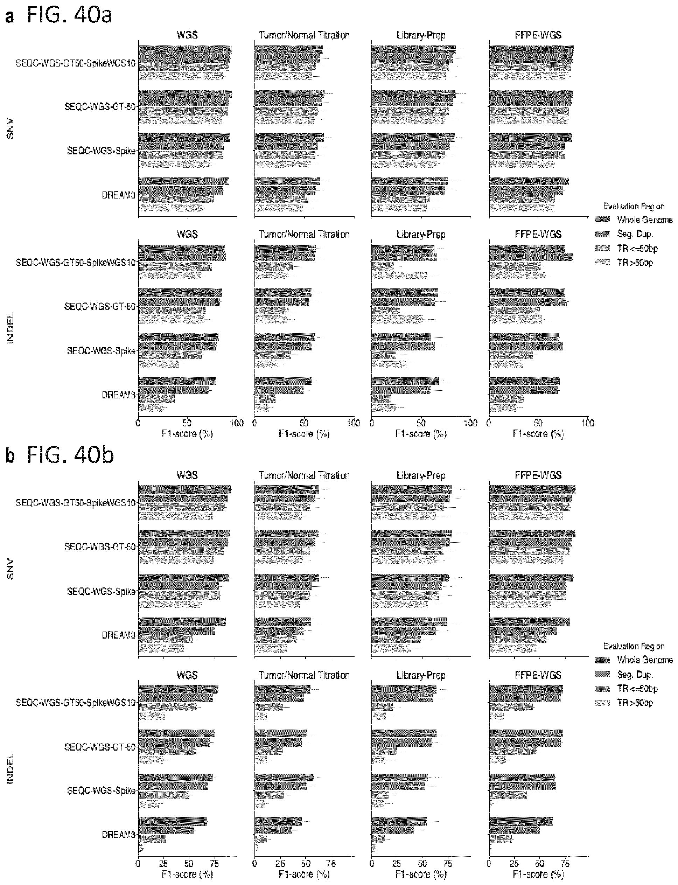

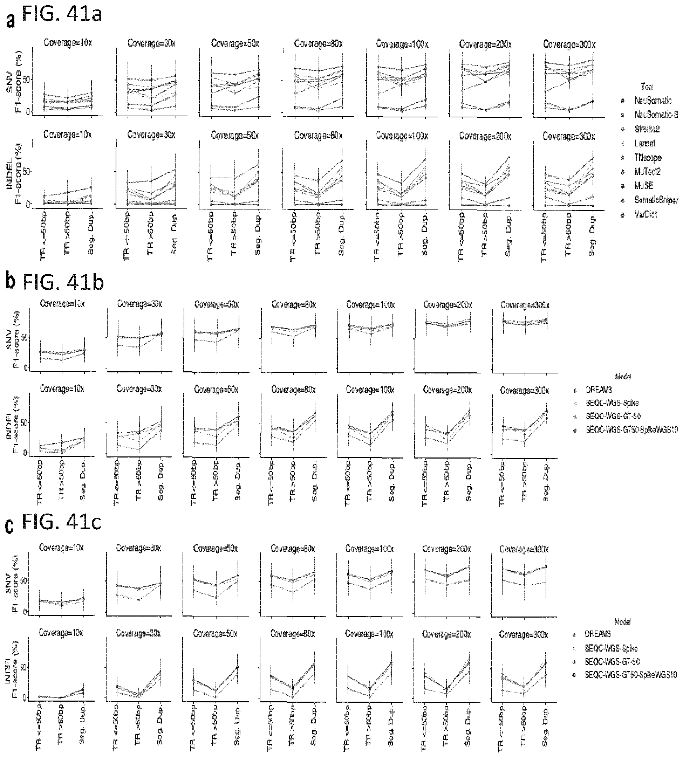

To illustrate robustness to genomic context complexity, we evaluated the performance of different somatic calling schemes on difficult genomic regions including different length tandem repeats (TR) and segmental duplications (FIGS. 34d and 38-41c). While many other schemes showed a significant drop in their performance on difficult genomic regions (FIG. 38), NeuSomatic remained highly robust and yielded even more significant improvement over other schemes compared to the whole genome analysis (FIGS. 34d and 38). On average, for these difficult genomic regions, NeuSomatic yielded more than 15% higher F1-scores than the best alternative conventional somatic caller for both SNVs and INDELs. Training NeuSomatic with SEQC-II samples resulted in large accuracy improvement on these difficult genomic regions, where the largest advantage was in tandem repeat regions using the models trained on ground truth mutations (FIGS. 39a-40b). Similar performance on difficult genomic regions was observed across varying coverage and tumor purity (FIGS. 41a-41c).

### Discussion

We explored the robustness of NeuSomatic's deep learning framework in detecting somatic mutations across a diverse set of experimental settings seen in real WGS, WES, and AmpliSeq samples with different coverages, sample purities, and library preparations, on FFPE and fresh DNA sequenced with multiple platforms in six centers. Experiments confirmed the potential of NeuSomatic in capturing true somatic mutation signatures from the raw data to differentiate true calls from sequencing artifacts. NeuSomatic models trained on SEQC-II reference samples, both using spike-in mutations and the set of ground truth somatic mutations derived by SEQC-II studies, boosted the performance to achieve the highest accuracy. This analysis highlighted model-building best practice strategies, of utility in various scenarios. Compared with the baseline DREAM3 network models, the proposed models derived from SEQC-II reference samples were shown to reduce false positives for both SNVs and INDELs, improve insertion calling accuracies, and enhance somatic detection on difficult genomic regions by learning correct mutation signals.

While NeuSomatic remained robust to tumor contamination in the match normal, many somatic calling approaches like MuTect2, Lancet, MuSE, and TNscope were highly impacted, because they rejected true somatic mutations that had corresponding reads in normal. Thus, their recall rate dropped significantly. NeuSomatic models trained on WGS performed equally well on targeted sequencing data. On the other hand, TNscope employs a machine learning (random forest) model, while Lancet uses localized colored de Bruijn graphs approach to detect mutations. Both tools were designed for genome-wide somatic mutation detection and thus are not suitable for targeted sequencing (typically covers less than 1 MB of the genome).

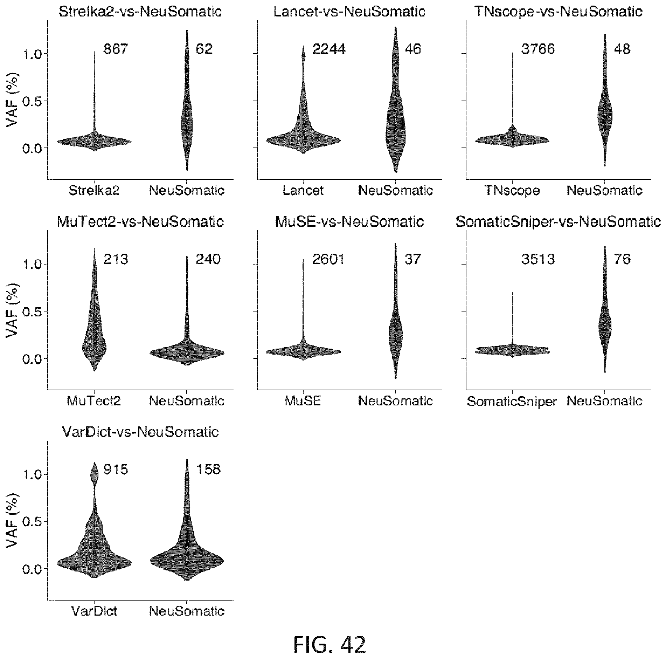

Analyzing prediction accuracies across different specifications including mutation type, INDEL length, VAF, and genomic region revealed that NeuSomatic yielded the largest improvements over other schemes in challenging situations such as low coverage, less than 20% VAF, difficult genomic regions like tandem repeats, INDELS more than 2bp long, FFPE with DNA damage, or contaminated match normal. As a result of confusing sequencing or sample-specific artifacts with the true somatic mutations, conventional schemes had many false positives (and thus lower precision), often seen in low-complexity genomic regions (FIGS. 34d and 38). However, by training on WGS samples from multiple platforms and multiple centers, NeuSomatic learned error-prone genomic contexts and thus consistently enhanced accuracy across different conditions including difficult-to-call low-complexity genomic contexts. Similarly, analysis of missed calls by different approaches revealed that most of the private false negative calls by conventional schemes like Strelka2, which were correctly predicted by NeuSomatic, had low VAF (FIG. 42). This revealed the inferiority of other schemes in detecting low VAF mutations. NeuSomatic, on the other hand, more accurately distinguished challenging low VAF calls from artifacts by learning the correct signature from raw data.

FIG. 42 shows the violin-plot comparison of the VAF distribution of private FN calls on WGS dataset. In each subfigure we compared one of the conventional somatic mutation detection schemes against NeuSomatic. For a X-vs-Y subfigure, we identified X_{FN}, the set of ground truth SNVs which were missed by algorithm X (FN in X) in at least 11 out of 21 WGS replicates. Similarly, we identified Y_{FN}, the set of ground truth SNVs which were missed by algorithm Y (FN in Y) in at least 11 out of 21 WGS replicates. The figure then shows the VAF distribution of private FN calls for X and Y. In other words, the violin-plot shows the VAF distribution of calls in the set X_{FN}/Y_{FN} in blue and the VAF distribution of calls in the set Y_{FN}/X_{FN} in red. For most of the conventional schemes like Strelka2, the private FNs, which were correctly predicted by NeuSomatic, had low VAF which revealed their inferiority of such approaches in detecting low VAF mutations. In each X-vs-Y subfigure, the number of private FNs for X and Y are reported on the top.

### Methods

### SEQC-II tumor-normal sequencing data and ground truth

We used the SEQC-II reference matched samples, a human triple-negative breast cancer cell line (HCC1395) and a matched B lymphocyte derived normal cell line (HCC1395BL) in our analysis. Detailed sample information can be found in the SEQC-II reference samples manuscripts^{12,13}. The SEQC-II somatic mutation working group has established a gold standard truth set of somatic mutations for these samples¹³ (FIG. 27). The truth set was defined using multiple tumor-normal sequencing replicates from different sequencing centers, and orthogonal mutation detection bioinformatics pipelines. To assign confidence scores to the mutation calls and minimized platform, center, and pipeline specific biases, the SomaticSeq¹⁰ machine learning framework was implemented to train a set of classifiers on in silico tumor-normal replicate pairs generated by spiking synthetic mutations into an HCC1395BL alignment file, matched with another HCC1395BL for each replicate pair. Using these classifiers, mutation calls were categorized into four confidence-levels (HighConf, MedConf, LowConf, and Unclassified) based on cross-aligner and cross-sequencing-center reproducibility. HighConf and MedConf calls were grouped together as the "truth set" of somatic mutations (v1.0), which contains a total of 39,536 SNVs and 2,020 INDELs. The truth set for somatic mutations in HCC1395, is available for community use on NCBI's ftp site (ftp://ftp-trace.ncbi.nlm.nih.gov/seqc/ftp/Somatic_Mutation_WG/).

All sequencing data used in this study are publicly available through NCBI's SRA database (SRP162370). For all samples, the FastQ files were first trimmed using Trimmomatic15, and then aligned with BWA-MEM (v0.7.15)16 followed by Picard MarkDuplicates (https://broadinstitute.github.io/picard).

### Training datasets and models

Different training datasets were used to derive effective NeuSomatic CNN models using both in silico tumor replicates, prepared with synthetic spike-in mutations, as well as real tumor replicates with known high confidence truth set of somatic mutations (FIG. 28e):

### DREAM3 model

As the baseline WGS model we employed the DREAM3 model, developed recently¹¹ by training on ICGC-TCGA DREAM Challenge Stage 3 data¹⁴. The Stage 3 dataset consists of a normal sample and a tumor sample constructed by computationally spiking 7,903 SNVs and 7,604 INDELs mutations into a healthy genome of the same normal sample with three different AFs of 50%, 33%, and 20% to create synthetic but realistic tumoral normal pairs. An additional ~95K SNVs and ~95K INDELs were spike-in into the tumor sample¹¹ using BAMSurgeon¹⁷ with similar AF distributions to the original DREAM data for better network training. This model was trained by combining training data (in 50% of the genome) from five different tumor-normal purity setting of 100T: 100N, 50T:100N, 70T:95N, 50T:95N, and 25T:95N11. NeuSomatic and NeuSomatic-S were trained on ~29.2M candidate mutations identified in these five replicate pairs, which includes ~450K candidates with somatic SNV/INDEL labels and ~28.7M candidates, labeled as non-somatic.

### TCGA model

As the baseline WES model, we used the TCGA model developed recently¹¹ by training on a set of 12 TCGA samples¹⁸. The tumor and normal alignments for each of these samples were mixed and split into two equal alignments. One alignment was treated as the normal replicate and the other alignment was used to spike in mutations to construct the tumor replicate. For each sample, ~88K SNVs and ~44K INDELs were spiked in to generate a synthetic tumor sample for training. NeuSomatic and NeuSomatic-S were trained on ~5.9M candidate mutations identified in these 12 replicate pairs, which includes ~1.5M candidates with somatic SNV/INDEL labels and ~4.4M candidates, labeled as non-somatic.

### SEQC-WGS-Spike model

To prepare the training data used to build this model, we employed BAMSurgeon to construct a synthetic tumor by spiking in silico mutations in one of the HCC1395BL replicates and pairing that with distinct HCC1395BL replicates as normal match. Using this approach, we prepared ten in silico tumor-normal pairs. Eight of the ten replicate pairs were selected from WGS replicates sequenced in four different sites with 40×-95× mean coverage. The other two replicate pairs were created by merging multiple NovaSeq sequencing replicates from Illumina to obtain in silico ~220× tumor sample coverage and ~170× normal matched sample coverage. In each in silico tumor we spiked in ~92 K SNVs and ~22 K INDELs. The AFs of the spike-in mutations were randomly selected from a beta distribution (with shape parameters α=2 and β=5). For each of the ten replicate pairs we also constructed an impure normal by mixing 95% normal and 5% tumor reads. So, in total we used 20 in silico tumor-normal pairs to train the SEQC-WGS-Spike model. We then trained NeuSomatic and NeuSomatic-S on ~272M candidate mutations identified in these 20 replicate pairs, which included ~2M candidates with somatic SNV/INDEL labels and ~270M candidates, labeled as non-somatic.

### SEQC-WGS-GT-50 model

This model was constructed using the real WGS tumor-normal replicates accompanied by the SEQC-II HighConf truth set as the ground truth somatic mutations. We used eight WGS tumor-normal replicates as the base samples to train this model. The first seven WGS replicate pairs were from six different sequencing centers on HiSeq and NovaSeq platforms with mean coverage of 40×-95×, and the last one was constructed by combining 9 NovaSeq sequencing replicates from Illumina to get a replicate pair with ~390× coverage. For each of these eight replicate pairs, we constructed two other purity variations, one with 95% normal purity by mixing 95% normal and 5% tumor reads, and the other with 10% tumor purity by mixing 10% tumor and 90% normal reads. So, for each of the replicate pairs we had a version with 100% pure tumor and normal, a version with 100% pure tumor matched with 95% pure normal, and a version with 10% pure tumor matched with 100% pure normal. Thus, in total, we used 24 tumor-normal replicates to train the SEQC-WGS-GT-50 model. To have unbiased evaluation, we only used 50% of the genome to train this model and kept the other 50% for evaluation. To prepare the training and evaluation regions, we split the genome to small regions of size ~92K bps, and randomly select half of the fractured regions for training and the other half for evaluation. We excluded a 5-bases padded region around each of the grey-zone mutations, including calls with LowConf label in the super-set of SEQC-II calls, and those with Unclassified label in the super-set of SEQC-II calls with more than 30% VAF, from the training region. We then trained NeuSomatic andNeuSomatic-S on - 137M candidate mutations identified in these 24 replicate pairs, which included ~416K candidates with somatic SNV/INDEL labels and ~136.5M candidates, labeled as non-somatic.

### SEQC-WGS-GT-ALL model

This model was prepared similar to the SEQC-WGS-GT-50 model but using all HighConf ground truth somatic mutations over the whole genome. So, it cannot be used to evaluate WGS samples which were used for training purposes and also may be biased towards truth set mutations given all have been used for training. This model is still beneficial for performance analysis on other datasets or non-SEQC-II samples. We trained this model for NeuSomatic and NeuSomatic-S on ~272M candidate mutations identified in the 24 replicate pairs used for SEQC-WGS-GT-50 model, which included ~839K candidates with somatic SNV/INDEL labels and ~271M candidates, labeled as non-somatic.

### SEQC-WGS-GT50-SpikeWGS10 model

The training data for this model was prepared by combining 10% of training candidates used for SEQC-WGS-Spike model and all candidates from SEQC-WGS-GT-50. This combination takes advantage of both a high number of spike-in mutations and the real somatic mutation characteristics seen in real tumor-normal data. We trained NeuSomatic and NeuSomatic-S on the combined set of 164M candidate mutations, including ~574K candidates with somatic SNV/INDEL labels and - 163.5M candidates, labeled as non-somatic.

### SEQC-WES-Spike model

Similar to the SEQC-WGS-Spike model, we used BAMSurgeon to construct 7 in silico tumor-normal WES replicates to train this model. The replicate pairs were selected from the WES dataset sequenced in four different sites with 60×-550× mean coverage. In each in silico tumor we spiked in ~97 K SNVs and ~19 K INDELs. The AF of the spike-in mutations were randomly selected from a beta distribution (with shape parameters α=2 and β=5).We then trained NeuSomatic and NeuSomatic-S on ~3.7M candidate mutations identified in these 7 replicate pairs, which included ~755K candidates with somatic SNV/INDEL labels and ~3M candidates, labeled as non-somatic.

### SEOC-FFPE-Spike model

Similar to the SEQC-WGS-Spike model, we used BAMSurgeon to construct 8 in silico tumor-normal WGS FFPE replicates to train this model. The replicate pairs were selected from the FFPE dataset sequenced at two different sites with four different preparation times. In each in silico tumor we spiked in ~92 K SNVs and ~22 K INDELs. The AF of the spike-in mutations were randomly selected from a beta distribution (with shape parameters α=2 and β=5). We also matched each in silico tumor with a fresh WGS replicate to include FFPE tumor versus fresh normal scenarios in our training. So, in total we used 16 in silico tumor-normal pairs to train the SEQC-FFPE-Spike model. We trained NeuSomatic and NeuSomatic-S on ~191M candidate mutations identified in these 7 replicate pairs, which included ~1,7M candidates with somatic SNV/INDEL labels and ~190M candidates, labeled as non-somatic.

### SEQC-FFPE-WES-Spike model

Similar to other spike-in models, we used BAMSurgeon to construct 7 in silico tumor-normal WES FFPE replicates to train this model. The replicate pairs were selected from the WES FFPE dataset sequenced in two different sites and prepared across four varying time intervals. Since we don't have two normal replicates with the same FFPE preparation time and sequencing site for this dataset, we mixed the tumor and normal alignments for each replicate pair and split the mixture into two equal alignments. We then treat one alignment as the normal replicate and spike-in mutations in the other to construct the tumor replicate. In each in silico tumor we spiked in ~90 K SNVs and ~19 K INDELs. The AF of the spike-in mutations were randomly selected from a beta distribution (with shape parameters α=2 and β=5).We also matched each in silico tumor with a fresh WES replicate to include FFPE tumor versus fresh normal scenarios in our training. So, in total, we used 14 in silico tumor-normal pairs to train the SEQC-FFPE-WES-Spike model. We trained NeuSomatic and NeuSomatic-S on ~9.6M candidate mutations identified in these 7 replicate pairs, which included ~ 1,4M candidates with somatic SNV/INDEL labels and ~8.2M candidates, labeled as non-somatic.

### SEQC-WES-SpikeWGS10, SEQC-FFPE-SpikeWGS10, SEQC-FFPE-WES-SpikeWGS10 models

The training data for these models was prepared by combining 10% of training candidates used for SEQC-WGS-Spike model and respectively all candidates from SEQC-WES-Spike, SEQC-FFPE-Spike, and SEQC-FFPE-WES-Spike. This combination takes advantage of both the high number of spike-in WGS mutations, as well as the sample biases for WES and FFPE samples.

### Sample-specific models

In addition to the above general-purpose models, for a set of nine samples across multiple data types, we derived sample-specific models. The selected samples included a WGS sample sequenced by a NovaSeq instrument, a WES sample, a sample prepared with a Nextera Flex library-prep kit with 1ng DNA amount, a 30× WGS sample with 50% tumor purity, a 100× WGS sample with 10% tumor purity, two WGS and two WES FFPE samples each treated with formalin for 24h and matched with fresh/FFPE normal samples. For each sample, we prepared the in silico tumor using random spikes. For the 10% tumor sample, the AF of the spike-in mutations were randomly selected from a beta distribution (with shape parameters α=2 and β=18). For the other samples, a beta distribution (with shape parameters α=2 and β=5) was used to select the AFs. For each sample, we then trained NeuSomatic and NeuSomatic-S models using the in silico tumor-normal replicates. In addition, for each sample we trained a distinct model by combining 10% of training candidates used for SEQC-WGS-Spike model and the training data derived for that sample.

### Somatic mutation detection algorithms

In addition to NeuSomatic, we used seven somatic mutation callers, MuTect2 (4.beta.6)², SomaticSniper (1.0.5.0)⁴, Lancet (1.0.7)⁸, Strelka2 (2.8.4)⁵, TNscope (201711.03)⁷, MuSE (v1.0rc)³, and VarDict (v1.5.1)⁶ and ran each of them using the default parameters or parameters recommended by the user's manual. For SomaticSniper, we used "-q 1 -Q 15 -s 1e-05". For TNscope (201711.03), we used the version implemented in Seven Bridges's CGC with the following command, "sentieon driver -i $tumor_bam -i $normal_bam -r $ref --algo TNscope --tumor_sample $tumor_sample_name --normal sample $normal_sample_name -d $dbsnp $output_vcf". For Lancet, we used "--cov-thr 10 --cov-ratio 0.005 --max-indel-len 50 -e 0.005". The high confidence outputs flagged as "PASS" in the resulting VCF files were applied to our comparison analysis. For VarDict we also restricted to calls with "Somatic" status. Results from each caller used for comparison were all mutation candidates that users would otherwise consider as "real" mutations detected by this caller.

We used NeuSomatic (0.1.4) in both ensemble and standalone mode. For ensemble mode, in addition to the candidate variants identified by "alignment scanning" step of NeuSomatic, we also incorporated calls from five individual callers (MuTect2, SomaticSniper, Strelka2, MuSE, and VarDict) and represented their occurrence as additional input channels for each candidate variant. For NeuSomatic and NeuSomatic-S, we used "--scan_maf 0.01-min_mapq 10 --snp_min_af 0.03 --snp_min_bq 15 --snp_min_ao 3 --ins_min_af 0.02-del_min_af 0.02 --num_threads 28 --scan_window_size 500 --max_dp 100000" during preprocessing step. For training, we used "--coverage_thr 300 --batch_size 8000".

### Difficult genomic regions

We used a set of difficult genomic regions derived by the Genome-in-a-Bottle consortium¹⁹. These regions included tandem repeats, in two different categories of less than 50bp and larger than 50bp repeats, and segmental duplication regions. We evaluated different techniques on these regions to illustrate robustness to genomic context complexity.

### Analysis of false negative calls

To better understand the difference in performance of different techniques, we performed a set of pairwise comparison between the VAF distribution of the false negatives SNVs of NeuSomatic against other schemes on WGS dataset (FIG. 42). In each X-vs-Y analysis, we identified XFN, the set of ground truth SNVs which were missed by algorithm X (False Negative in X) in at least 11 out of 21 WGS replicates. Similarly, we identified YFN, the set of ground truth SNVs which were missed by algorithm Y (False Negative in Y) in at least 11 out of 21 WGS replicates. We then computed the private false negatives of X, defined as XFN / YFN, and the private false negatives of Y, defined as YFN / XFN. For somatic mutations in each of those sets we then computed the distribution of VAF of mutations.

### Evaluation process

For a fair comparison, we evaluated all the models and somatic mutation algorithms on the 50% hold-out genomic region which was not used for training the SEQC-WGS-GT-50 model. This ~1,4GB region contained ~21K SNVs and ~1.3K INDELs from the SEQC-II truth set for HCC1395 which were not used for training any of the NeuSomatic models except for SEQC-WGS-GT-ALL model.

Calls labeled as HighConf and MedConf by SEQC-II consortium for HCC1395 were grouped together as the "truth set" of somatic mutations used herein. We employed this truth set to compute the true positives and false negatives across all replicates of HCC1395 for all pipelines. As recommended by SEQC-II consortium, we also blacklisted LowConf calls as they have low validation rates. Since this truth set provided by the consortium had a VAF detection limit of 5% and depth detection limit of 50×, for calls at higher coverages data or lower VAFs, we cannot ascertain their real somatic status. Thus, for private calls reported by any pipeline that was not in the truth set, we excluded calls which were deemed to be ambiguous for evaluation. In summary, for a tumor replicate in-hand that had a mean coverage of C and a tumor purity of P, if a pipeline had reported a private somatic mutation for this replicate (which was not in the truth set) that has d number of supporting reads, we only labeled this call as false positive if its projected number of supporting reads at 100% purity and 50x coverage was ≥ 3. In other words, this call was false positive if d ≥ 3CP/50; otherwise we exclude this call from evaluation.

For WES and AmpliSeq data since the number of true indels in the evaluation region was very limited, we only reported SNVs evaluations.

### References

1. Xu, C. A review of somatic single nucleotide variant calling algorithms for next-generation sequencing data. Comput. Struct. Biotechnol. J. 16, 15-24 (2018).
2. Cibulskis, K. et al. Sensitive detection of somatic point mutations in impure and heterogeneous cancer samples. Nat. Biotechnol. 31, 213 (2013).
3. Fan, Y. et al. MuSE: accounting for tumor heterogeneity using a sample-specific error model improves sensitivity and specificity in mutation calling from sequencing data. Genome Biol. 17, 178 (2016).
4. Larson, D. E. et al. SomaticSniper: identification of somatic point mutations in whole genome sequencing data. Bioinformatics 28, 311-317 (2011).
5. Kim, S. et al. Strelka2: fast and accurate calling of germline and somatic variants. Nat. Methods 15, 591-594 (2018).
6. Lai, Z. et al. VarDict: a novel and versatile variant caller for next-generation sequencing in cancer research. Nucleic Acids Res. 44, e108-e108 (2016).
7. Freed, D., Pan, R. & Aldana, R.TNscope: Accurate Detection of Somatic Mutations with Haplotype-based Variant Candidate Detection and Machine Learning Filtering. bioRxiv 250647
8. Narzisi, G. et al. Lancet: genome-wide somatic variant calling using localized colored DeBruijn graphs. Communications biology 1, 20 (2018).
9. Koboldt, D. C. et al. VarScan 2: somatic mutation and copy number alteration discovery in cancer by exome sequencing. Genome Res. 22, 568-576 (2012).
10. Fang, L. T. et al. An ensemble approach to accurately detect somatic mutations using SomaticSeq. Genome Biol. 16, 197 (2015).
11. Sahraeian, S. M. E., Liu, R., Lau, B., Podesta, K., Mohiyuddin, M., & Lam, H. Y. Deep convolutional neural networks for accurate somatic mutation detection. Nature communications. 10, 1041 (2019).
12. The Somatic Mutation Working Group of SEQC-II Consortium. Achieving reproducibility and accuracy in cancer mutation detection with whole-genome and whole-exome sequencing. https://doi.org/10.1101/626440 (2019).
13. Fang, L. T. et al. Establishing reference samples for detection of somatic mutations and germline variants with NGS technologies, https://doi.org/10.1101/625624 (2019).
14. Ewing, A. D. et al. Combining tumor genome simulation with crowdsourcing to benchmark somatic single-nucleotide-variant detection. Nat. Methods 12, 623 (2015).
15. Bolger, A. M., Lohse, M. & Usadel, B. Trimmomatic: a flexible trimmer for Illumina sequence data. Bioinformatics 30, 2114-2120 (2014).
16. Li, H. Aligning sequence reads, clone sequences and assembly contigs with BWA-MEM. https://arxiv.org/abs/1303.3997 (2013).
17. Ewing, A. D. et al. Combining tumor genome simulation with crowdsourcing to benchmark somatic single-nucleotide-variant detection. Nat. Methods 12, 623 (2015).
18. Grossman, R. L. et al. Toward a shared vision for cancer genomic data. N. Engl. J. Med. 375, 1109-1112 (2016).
19. Krusche, Peter, Len Trigg, Paul C. Boutros, Christopher E. Mason, M. Francisco, Benjamin L. Moore, Mar Gonzalez-Porta et al. "Best practices for benchmarking germline small-variant calls in human genomes." Nature biotechnology 1 (2019).

Although the present disclosure has been described with reference to a number of illustrative embodiments, it should be understood that numerous other modifications and embodiments can be devised by those skilled in the art that will fall within the spirit and scope of the principles of this disclosure. More particularly, reasonable variations and modifications are possible in the component parts and/or arrangements of the subject combination arrangement within the scope of the foregoing disclosure, the drawings, without departing from the spirit of the disclosure. In addition to variations and modifications in the component parts and/or arrangements, alternative uses will also be apparent to those skilled in the art.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present disclosure.

Throughout this specification unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

As used herein in the specification including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description.

## Claims

1. A computer-implemented method for germline variant calling, the method comprising:
obtaining a reference sequence, a plurality of sequence reads, and a position of a candidate variant within the sequence reads;
obtaining augmented sequence reads by inserting one or more spaces in one or more sequence reads;
obtaining an augmented reference sequence by inserting one or more spaces in the reference sequence;
converting a segment of the augmented sequence reads around the candidate variant into a sample matrix;
converting a segment of the augmented reference sequence around the candidate variant into a reference matrix, wherein the sample matrix comprises:
at least four lines representing four types of nucleotide bases, each line representing the number of bases of the respective nucleotide base type at different positions within the segment of the augmented sequence reads; and
at least one line representing the number of inserted spaces at different positions within the segment of the augmented sequence reads;
providing the sample matrix and the reference matrix to a trained neural network; and obtaining, at the output of the trained neural network, prediction data related to a variant within the plurality of sequence reads,wherein the prediction data related to the variant comprises at least one of:
a predicted type of the variant;
a predicted position of the variant;
a predicted length of the variant; and
a predicted genotype of the variant,
the method further comprising:
detecting one or more inserted bases within the plurality of sequence reads, wherein augmenting the sequence reads and the reference sequence comprises:
for each inserted base detected in any of the sequence reads, inserting a space in the reference sample at the position of the inserted base; and
for each inserted base detected in any of the sequence reads, inserting a space at the position of the inserted base in every sequence read for which no insertions were detected at the position of the inserted base.

2. The method of claim 1, wherein the reference matrix has the same dimensions as the sample matrix and wherein the reference matrix provides a complete representation of the locations of different nucleotide bases and spaces within the augmented reference sequence.

3. The method of claim 1, wherein the trained neural network comprises a trained convolutional neural network.

4. The method of claim 1, further comprising providing to the trained neural network at least one of:
a variant position matrix representing the candidate variant's position within the segment of the augmented sequence reads;
a coverage matrix representing coverage or depth of the segment of the augmented sequence reads;
an alignment feature matrix representing an alignment feature of the augmented sequence reads;
a knowledgeable base matrix representing information about publicly known information about one or more variants.

5. The method of claim 1, wherein the prediction data related to the variant comprises a predicted type of the variant, and wherein the neural network is configured to produce one of a plurality of values for predicted type of the variant, the plurality of values comprising:
a first value indicating a probability that the variant is a false positive;
a second value indicating a probability that the variant is a single-nucleotide-polymorphism variant;
a third value indicating a probability that the variant is a deletion variant; and
a fourth value indicating a probability that the variant is an insertion variant.

## Patentansprüche

1. Computerimplementiertes Verfahren zum Keimbahn-Variant-Calling, wobei das Verfahren Folgendes umfasst:
Erhalten einer Referenzsequenz, einer Vielzahl von Sequenzlesungen und einer Position einer Kandidatenvariante in den Sequenzlesungen;
Erhalten vergrößerter Sequenzlesungen durch Einfügen eines Zwischenraumes oder mehrerer Zwischenräume in eine oder mehrere Sequenzlesung(en);
Erhalten einer vergrößerten Referenzsequenz durch Einfügen eines Zwischenraumes oder mehrerer Zwischenräume in die Referenzsequenz;
Umwandeln eines Segments der vergrößerten Sequenzlesungen um die Kandidatenvariante in eine Probenmatrix;
Umwandeln eines Segments der vergrößerten Referenzsequenz um die Kandidatenvariante in eine Referenzmatrix, wobei die Probenmatrix Folgendes umfasst:
mindestens vier Linien, die vier Typen von Nukleotidbasen darstellen, wobei jede Linie die Anzahl von Basen des jeweiligen Nukleotidbasentyps an verschiedenen Positionen innerhalb des Segments der vergrößerten Sequenzlesungen darstellt; und
mindestens eine Linie, welche die Anzahl eingefügter Zwischenräume an verschiedenen Positionen innerhalb des Segments der vergrößerten Sequenzlesungen darstellt;
Bereitstellen der Probenmatrix und der Referenzmatrix an ein trainiertes neuronales Netz; und Erhalten von Vorhersagedaten bezüglich einer Variante innerhalb der Vielzahl von Sequenzlesungen an der Ausgabe des trainierten neuronalen Netzes, wobei die Vorhersagedaten bezüglich einer Variante mindestens eines von Folgendem umfassen:
einen vorhergesagten Typ der Variante;
eine vorhergesagte Position der Variante;
eine vorhergesagte Länge der Variante; und
einen vorhergesagten Genotyp der Variante,
wobei das Verfahren ferner Folgendes umfasst:
Nachweisen einer oder mehrerer eingefügter Basen innerhalb der Vielzahl von Sequenzlesungen, wobei das Vergrößern der Sequenzlesungen und der Referenzsequenz Folgendes umfasst:
für jede eingefügte Base, die in einer der Sequenzlesungen nachgewiesen wurde, Einfügen eines Zwischenraumes in der Referenzprobe an der Position der eingefügten Base; und
für jede eingefügte Base, die in einer der Sequenzlesungen nachgewiesen wurde, Einfügen eines Zwischenraumes an der Position der eingefügten Base in jeder Sequenzlesung, für die keine Einfügungen an der Position der eingefügten Base nachgewiesen wurden.

2. Verfahren nach Anspruch 1, wobei die Referenzmatrix dieselben Dimensionen hat wie die Probenmatrix und wobei die Referenzmatrix eine vollständige Darstellung der Orte verschiedener Nukleotidbasen und Zwischenräume innerhalb der vergrößerten Referenzsequenz bereitstellt.

3. Verfahren nach Anspruch 1, wobei das trainierte neuronale Netz ein trainiertes neuronales Faltungsnetz umfasst.

4. Verfahren nach Anspruch 1, ferner umfassend das Bereitstellen mindestens eines von Folgendem an das trainierte neuronale Netz:
eine Variantenpositionsmatrix, welche die Position der Kandidatenvariante innerhalb des Segments der vergrößerten Sequenzlesungen darstellt;
eine Deckungsmatrix, welche die Deckung oder Tiefe des Segments der vergrößerten Sequenzlesungen darstellt;
eine Ausrichtungsmerkmalsmatrix, welche ein Ausrichtungsmerkmal der vergrößerten Sequenzlesungen darstellt;
eine Wissensdatenbankmatrix, welche Informationen über öffentlich bekannte Informationen über eine oder mehrere Variante(n) darstellt.

5. Verfahren nach Anspruch 1, wobei die Vorhersagedaten bezüglich der Variante einen vorhergesagten Typ der Variante umfassen, und wobei das neuronale Netz zum Produzieren eines einer Vielzahl von Werten für den vorhergesagten Typ der Variante ausgebildet ist, wobei die Vielzahl von Werten Folgendes umfasst:
einen ersten Wert, der eine Wahrscheinlichkeit angibt, dass die Variante falsch-positiv ist;
einen zweiten Wert, der eine Wahrscheinlichkeit angibt, dass die Variante eine Einzel-Nukleotid-Polymorphismusvariante ist;
einen dritten Wert, der eine Wahrscheinlichkeit angibt, dass die Variante eine Deletionsvariante ist; und
einen vierten Wert, der eine Wahrscheinlichkeit angibt, dass die Variante eine Insertionsvariante ist.

## Revendications

1. Procédé mis en oeuvre par ordinateur pour l'appel de variants de lignée germinale, le procédé comprenant :
l'obtention d'une séquence de référence, d'une pluralité de lectures de séquence et d'une position d'un variant candidat au sein des lectures de séquence ;
l'obtention de lectures de séquence augmentées par insertion d'un ou de plusieurs espaces dans une ou plusieurs lectures de séquence ;
l'obtention d'une séquence de référence augmentée par insertion d'un ou de plusieurs espaces dans la séquence de référence ;
la conversion d'un segment des lectures de séquence augmentées autour du variant candidat en une matrice d'échantillon ;
la conversion d'un segment de la séquence de référence augmentée autour du variant candidat en une matrice de référence, dans lequel la matrice d'échantillon comprend :
au moins quatre lignes représentant quatre types de bases nucléotidiques, chaque ligne représentant le nombre de bases du type de base nucléotidique respectif au niveau de différentes positions au sein du segment des lectures de séquence augmentées ; et
au moins une ligne représentant le nombre d'espaces insérés au niveau de différentes positions au sein du segment des lectures de séquence augmentées ;
la fourniture de la matrice d'échantillon et de la matrice de référence à un réseau neuronal entraîné ; et l'obtention, au niveau de la sortie du réseau neuronal entraîné, de données de prédiction liées à un variant au sein de la pluralité de lectures de séquence, dans lequel les données de prédiction liées au variant comprennent au moins un parmi :
un type prédit du variant ;
une position prédite du variant ;
une longueur prédite du variant ; et
un génotype prédit du variant,
le procédé comprenant en outre :
la détection d'une ou de plusieurs bases insérées au sein de la pluralité de lectures de séquence, dans lequel l'augmentation des lectures de séquence et de la séquence de référence comprend :
pour chaque base insérée détectée dans n'importe laquelle des lectures de séquence, l'insertion d'un espace dans l'échantillon de référence au niveau de la position de la base insérée ; et
pour chaque base insérée détectée dans n'importe laquelle des lectures de séquence, l'insertion d'un espace au niveau de la position de la base insérée dans chaque lecture de séquence pour laquelle aucune insertion n'a été détectée au niveau de la position de la base insérée.

2. Procédé selon la revendication 1, dans lequel la matrice de référence a les mêmes dimensions que la matrice d'échantillon et dans lequel la matrice de référence fournit une représentation complète des emplacements des différents bases nucléotidiques et espaces au sein de la séquence de référence augmentée.

3. Procédé selon la revendication 1, dans lequel le réseau neuronal entraîné comprend un réseau neuronal convolutif entraîné.

4. Procédé selon la revendication 1, comprenant en outre la fourniture au réseau neuronal entraîné d'au moins une parmi :
une matrice de position de variant représentant la position du variant candidat au sein du segment des lectures de séquence augmentées ;
une matrice de couverture représentant une couverture ou une profondeur du segment des lectures de séquence augmentées ;
une matrice de caractéristique d'alignement représentant une caractéristique d'alignement des lectures de séquence augmentées ;
une matrice de base informée représentant des informations autour d'informations publiquement connues autour d'un ou de plusieurs variants.

5. Procédé selon la revendication 1, dans lequel les données de prédiction liées au variant comprennent un type prédit du variant, et dans lequel le réseau neuronal est configuré pour produire une parmi une pluralité de valeurs pour le type prédit du variant, la pluralité de valeurs comprenant :
une première valeur indiquant une probabilité que le variant soit un faux positif ;
une deuxième valeur indiquant une probabilité que le variant soit un variant de polymorphisme mononucléotidique ;
une troisième valeur indiquant une probabilité que le variant soit un variant de délétion ; et
une quatrième valeur indiquant une probabilité que le variant soit un variant d'insertion.
